# EUROPEAN PATENT APPLICATION

(11) **EP 2 719 696 A1**
(43) Date of publication of application: **16.04.2014**
(21) Application number: 12797262.8
(22) Date of filing: 31.05.2012
(51) Int. Cl.: C07D 417/12, C07D 417/14, A61K 31/427, A61K 31/4427, A61K 31/506, A61K 31/497, A61P 35/00

(54) **NOVEL KINASE INHIBITORS**

(30) Priority: 08.06.2011 RU 2011122942
(71) Applicant: Asinex Limited, Moscow 107078 (RU)
(72) Inventor: KAZULKIN, Denis Nikolaevich, Moskovskaya obl. 143430 (RU); KOCHUBEY, Valeriy Sergeevich, Moscow 125466 (RU)
(74) Representative: Glawe, Delfs, Moll
(86) International application number: PCT/RU2012/000428
(87) International publication number: WO 2012/169934

(57) **Abstract**

The present invention provides derivatives of thiazolylamino-substituted heterocycles. These compounds are inhibitors of kinases, including compounds that show antiproliferative activity against cells, including against tumor cells, and are useful in the treatment of diseases including cancer.

## Description

### Field of the Invention

The present invention provides derivatives of thiazolylamino-substituted heterocycles. These compounds are inhibitors of kinases, comprising compounds that show antiproliferative activity against cells, including against tumor cells, and are useful in the treatment of diseases including cancer.

### Background of the Invention

Kinases are important cellular enzymes that perform essential cellular functions such as regulating cell division and proliferation, and appear to play a decisive role in many disease states such as in disease states that are characterized by uncontrolled proliferation and differentiation of cells. These disease states encompass a variety of cell types and maladies such as cancer, atherosclerosis, and restenosis.

Drugs that target kinases, and especially drugs based on small-molecule chemical compounds that inhibit the activity of kinases, are important medicines that can significantly improve the health, lifespan or quality of life for people throughout the world, including patients suffering from highly debilitating diseases such as cancer. Indeed, the drug "imatinib" (GLEEVEC^{®}/GLIVEC^{®}; Novartis) is such a drug that inhibits a kinase and is used to successfully treat certain types of cancer including particular types of leukaemia (chronic myeloid leukemia, CML) and gastrointestinal cancer (stromal tumors of gastrointestinal (GI) tract). Its improved efficacy compared to previous standard treatments for CML, and its overall better tolerability, showing milder side-effects, has lead, among other reasons, for it to have made the cover of TIME magazine where it was described as a "magic bullet" to combat cancer.

Tyrosine kinases are essential for the propagation of growth factor signal transduction leading to cell cycle progression, cellular proliferation, differentiation, and migration. Tyrosine kinases include cell surface growth factor receptor tyrosine kinases (RTKs) such as FGFr and PDGFr as well as non-receptor tyrosine kinases including c-Src and a number of others. Inhibition of these enzymes has been demonstrated to cause antitumor and antiangiogenesis activity (Hamby et al., Pharmacol. Ther, 1999; 82(2-3):169-193).

The molecular mechanisms and signalling pathways that regulate cell proliferation and survival are receiving considerable attention as potential targets for anticancer drug development.

Malignant gliomas are among the most lethal tumors of the brain, with median survivals of not more than a year for patients with the most common type of glioma: glioblastoma. It has been shown that several receptor tyrosine kinases (RTK) are overexpressed or mutated in glioblastoma, which leads to constitutive activation of cell signalling, resulting in cell proliferation, survival, invasion and promotion of angiogenesis.

On the basis of their structure and unique motifs in the extracellular domain RTKs are characterized into several families. The ERBB family of proteins includes ERBB1 (HER1 or EGFR), ERBB2 (HER2/neu), ERBB3 (HER3) and ERBB4 (HER4). According to current data EGFR kinase mutation is found in 24-67% of glioblastoma samples, which is a poor prognostic factor. Other RTK-kinase that are involved in the pathogenesis of the development and maintenance of malignant glioma include receptors of so-called growth factors: PDGF-receptors (PDGFR kinases), VEGF receptors (VEGFR kinases) receptors of insulin-like growth factor (IGFR), and c-met receptor (kinase) of growth factor / scatter factor of hepatocytes (HGF / SF). Non-receptor Src-family kinases: Src, Lyn, Fyn also play an important role in the migration of aggressive glioblastoma cells.

In recent years new multitargeted therapeutic strategies have emerged to overcome a resistance, which is a common concern in monotherapy of oncologic diseases with single-targeted kinase inhibitors. Multitargeted kinase inhibitors (inhibiting several kinases) can be more effective in treating a variety oncologic diseases, since several simultaneous blocking of cellular signal transduction pathways in cancer cells are more likely to lead to the desired outcome of treatment. However, cross-reactivity of multitargeted kinase inhibitors also cause the safety problem, therefore, in order to optimize efficiency and minimize toxicity it is important to carefully analyze specificity and absorption properties, distribution, metabolism and excretion, and toxicity of kinase inhibitors. There are several US FDA approved multitargeted kinase inhibitors including imatinib, sunitinib, sorafenib, lapatinib, dasatinib and pazopanib for cancer treatment. These multitargeted kinase inhibitors along with other new ones are undergoing preclinical and clinical evaluation as agents for anti-glioblastoma therapy and other malignant tumors of the CNS. Unfortunately, many researched kinase inhibitor candidates failed to show efficacy due to problems with penetration of the blood brain barrier (BBB). The permeability of the BBB is one of the basic conditions for successful application of targeted therapy for malignant tumors of the CNS. Therefore, the identification of potent BBB-permeable kinase inhibitors represents a highly unmet medical need.

For the same reasons, the treatment of tumors metastasizing in CNS (secondary brain tumors) is hampered by a lack of efficacy due to problems with penetration of the blood brain barrier (BBB).

Thus, despite the progress that has been made, the search continues for low molecular weight kinase inhibitor compounds, especially compounds that inhibit serine/threonine kinases and/or receptor tyrosine kinases, that are useful for treating a wide variety of diseases, including cancer, tumors and other proliferative disorders or diseases including restenosis, angiogenesis, diabetic retinopathy, psoriasis, surgical adhesions, macular degeneration, and atherosclerosis, or other disorders or diseases mentioned above. Thus, a strong need exists to provide compositions, pharmaceuticals and/or medicaments with kinase inhibitory activity, particularly inhibitory activity against one or more serine/threonine kinases and/or receptor tyrosine kinases, or with anti-proliferative activity against cells such as tumor cells. Such compositions, pharmaceuticals and/or medicaments may possess not only such activity, but may also exert tolerable, acceptable or diminished side effects in comparison to other anti-proliferative agents. Furthermore, the spectrum of tumors or other diseases responsive to treatment with such compounds may be broad. The active ingredients of such compositions, pharmaceuticals and medicaments may be suitable in the mentioned indication as single agent, and/or in combination therapy, be it in connection with other therapeutic agents, with radiation, with operative/surgical procedures, heat treatment or any other treatment known in the mentioned indications.

It is known that specific classes of thiazolylamino-containing compounds, substituted in a specific manner, have pharmacologically useful properties, such as anti-proliferative activity. These compounds however are structurally dissimilar from the compounds of the present invention.

WO 2004/085388 (Bristol-Myers Squibb Company) discloses substituted thiazol-2-ylamino-containing heterocycles and substituted thiazol-2-ylamino-aryl structures that are shown to inhibit protein tyrosine kinases and to have certain activity in animal tumor models. WO 2004/085388 discloses *inter alia* a compound now known as Dasatinib, which was reported to cross the BBB with human CSF/plasma ratios ranging from 0.05 to 0.28 (Wei-Sheng Huang et al., J. Med. Chem. 2010, 53, 4701-4719). However, the data about Dasatinib's BBB penetration properties are controversial (see, for example, Chen Y, Agarwal S, Shaik NM, Chen C, Yang Z, Elmquist WF, J Pharmacol Exp Ther. 2009; 330, 956-63).

WO 2006/135604 discloses substituted 2-thiazol-2-ylamino-pyridines, which are shown to have checkpoint kinase (kinase of the cell cycle checkpoint) (serine/threonine kinases) inhibitor activity (Merck & Co).

WO 2007/141571 discloses substituted 2-thiazol-2-ylamino-pyridines and 4-thiazol-2-ylamino-pyrimidines, which are shown to have MARK kinase (microtubule-associated protein (MAP)-microtubule affinity regulating kinase) inhibitor activity.

### Summary of the Invention

We have invented a class of substituted thiazol-2-ylamino N-heteroaryl compounds that comprises compounds that exhibit surprising properties, including activity as inhibitors of a number of serine/threonine kinases and/or receptor tyrosine kinases, and anti-proliferative activity against cells such as tumour cells. Such substituted thiazol-2-ylamino N-heteroaryl compounds provide an opportunity to develop new and effective therapies for diseases associated with kinase de-regulation or cellular proliferation, such as cancer or inflammatory disorders.

Compounds of the present invention are specific derivatives of thiazol-2-ylamino N-heteroaryl compounds, as discussed in greater detail below, and are suitable for further preclinical or clinical research and development towards the treatment of a variety of disorders and diseases comprising cancer, proliferative, degenerative, inflammatory and other disorders and diseases. The present invention provides effective therapies and therapeutics for particularly severe and debilitating diseases such as cancer and other diseases and disorders comprising those listed above.

One aspect of the invention relates to thiazol-2-ylamino N-heteroaryl compounds having a structure represented by formula (I) presented below, or tautomeric or stereoisomeric form thereof, which are useful as serine/threonine kinase and/or receptor tyrosine kinase inhibitors and thus useful for treating disorders or diseases mentioned herein, comprising proliferative disorders or diseases such as cancer, and inflammatory disorders or diseases.

In another aspect, the invention relates to pharmaceutical compositions, comprising a pharmaceutically acceptable diluent, excipient or carrier and an amount, such as a therapeutically effective amount, of such serine/threonine kinase and/or receptor tyrosine kinase inhibitors, e.g., a dose which is expected to ameliorate the effects of disorders or diseases mentioned herein, comprising proliferative disorders or diseases such as cancer, and inflammatory disorders or diseases.

Another aspect of the invention relates to a pharmaceutical package, comprising such pharmaceutical composition, and instructions which indicate that said pharmaceutical composition may be used for the treatment of a patient suffering from a disorder or disease mentioned herein, comprising proliferative disorders or diseases such as cancer, and inflammatory disorders or diseases.

In another aspect, the invention relates to methods with the help of which a subject, a cell, a tissue, an organ or an organism is administered or contacted with a therapeutically effective amount of a compound or pharmaceutical composition disclosed herein. These methods include, but are not limited to, prophylaxis and/or treatment of a disorder or disease mentioned herein, comprising proliferative disorders or diseases such as cancer, and inflammatory disorders or diseases.

In another aspect, the present invention relates to uses of the compounds of the present invention for the preparation of medicaments for use in the treatment of a disorder or disease mentioned in the application, comprising proliferative disorders or diseases such as cancer.

In another aspect, the invention relates to uses of the compounds of the present invention for the preparation of medicaments for the treatment of an inflammatory disorder or disease.

Another aspect of the invention relates to methods of synthesizing the compounds of the present invention, and to intermediates for such compounds.

Accordingly, the present invention provides compounds having a structure represented by the general formula (I). 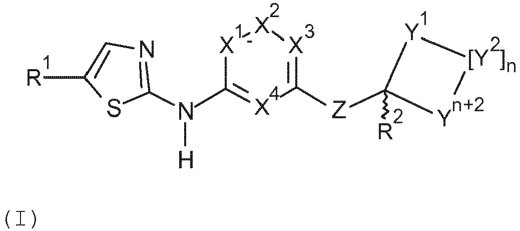 or any tautomeric form thereof, wherein
R¹ is selected from -hydrogen, -halogen, unsubstituted or substituted -alkyl, unsubstituted or substituted -alkynyl, unsubstituted or substituted -aryl, unsubstituted or substituted -heteroaryl, -C(=O)OR⁴, -C(=O)N(R⁵)₂, and -CN; wherein
R⁴ is selected from hydrogen, unsubstituted or substituted -alkyl;
   and
each R⁵ is independently selected from unsubstituted or substituted -alkyl, or alternatively, the two substituents R⁵, together with the N atom they are attached to, form a heterocycle, which may additionally contain a further heteroatom selected form N, O, or S;
X¹, X², X¹, and X⁴ are each independently selected from C-R⁶ and N, provided that either one, two or three of X¹, X², X³, and X⁴ is/are N; wherein
R⁶ is selected from -hydrogen, -halogen, unsubstituted or substituted -C₁₋₄-alkyl, and CN;
Z is selected from an ordinary bond, and unsubstituted or substituted -C₁₋₄-alkylene;
R² is selected from hydrogen, unsubstituted or substituted -C₁₋₄-alkyl, -C(=O)OR^{4'}, -C(=O)N(R⁵)₂, -CH₂OR^{4'}, and - CH₂N(R⁵)₂;
   wherein R^{4'} is selected from hydrogen, unsubstituted or substituted -alkyl;
Y¹, each of Y², and Yⁿ⁺² are each independently selected from C(R⁷)₂, N(R³) and O, wherein n is selected from 1, 2, 3, 4, and 5, provided that Y¹-[Y²]ₙ-Yⁿ⁺² consists of:
   (n+2) C(R⁷)₂ residues;
   (n+1) C(R⁷)2 residues and one N(R³) residue;
   (n+1) C(R⁷)₂ residues and one O residue;
   n C(R⁷)₂ residues, one N(R³) residue and one O residue;
   n C(R⁷)₂ residues and two N(R³) residues; or
   n C(R⁷)₂ residues and two O residues, provided that the two O residues are separated by at least one C(R⁷)₂ residue; wherein:
      each R⁷ is independently selected from -hydrogen, -F, and unsubstituted or substituted -C₁₋₄-alkyl; and
      each R³ is independently selected from -hydrogen and substituted or unsubstituted: -alkyl, -alkenyl, -alkynyl,-cycloalkyl, -cycloalkenyl, -(C-linked heterocycloalkyl), - (C-linked heterocycloalkenyl), -aryl, -heteroaryl, -C(=O)-R⁸, -C(=O)-aryl, -C(=O)-heteroaryl, -S(=O)₂R⁸, -S(=O)₂N(R⁵)₂, -C(=O)OR^{4'}, and -C(=O)N(R⁵)₂; wherein
      R⁸ is selected from substituted or unsubstituted: -alkyl, - alkenyl, -alkynyl, -cycloalkyl, -cycloalkenyl,-heterocycloalkyl), -heterocycloalkenyl), -aryl, and - heteroaryl; and/or
      two substituents R⁷ and/or R³, together with the ring atom(s) they are attached to, form a cycle, which may additionally contain a further heteroatom selected form N, O, or S;
      provided that the following compound is excluded:

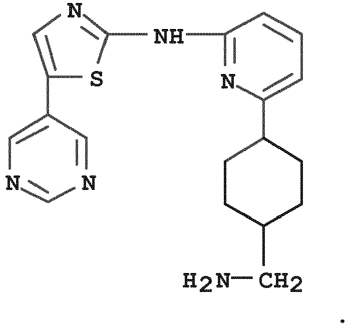

Other features and advantages of the invention will be apparent from the following detailed description and from the claims.

### Definitions

The term "alkyl" refers to straight- or branched-chain saturated hydrocarbon groups, comprising hydrocarbon groups having from 1 to 25 carbon atoms, from 1 to 20 carbon atoms, from 1 to 15 carbon atoms, and from 1 to 10 carbon atoms, comprising groups having from 1 to 8 carbon atoms. In certain embodiments, alkyl substituents may be lower alkyl substituents. The term "lower alkyl" refers to alkyl groups having from 1 to 6 carbon atoms. Examples of lower alkyl groups include, but are not limited to, methyl, ethyl, n-propyl, i-propyl, n-butyl, s-butyl, t-butyl, n-pentyl, s-pentyl, and n-hexyl.

The term "alkenyl" refers to hydrocarbon groups, wherein at least one of the carbon-carbon bonds is a double bond, while other bonds may be single bonds or further double bonds, comprising hydrocarbon groups having from 2 to 25 carbon atoms, from 2 to 20 carbon atoms, from 2 to 15 carbon atoms, and from 2 to 10 carbon atoms, comprising groups having from 2 to 8 carbon atoms.

The term "alkynyl" herein refers to hydrocarbon groups, wherein at least one of the carbon-carbon bonds is a triple bond, while other bonds may be single, double or further triple bonds, comprising hydrocarbon groups having from 2 to 25 carbon atoms, from 2 to 20 carbon atoms, from 2 to 15 carbon atoms, and from 2 to 10 carbon atoms, comprising groups having from 2 to 8 carbon atoms. Examples of alkenyl groups comprise ethenyl, 1-propenyl, 2-propenyl, 1-butenyl, 2-butenyl, 3-butenyl, 2-methyl-1-propenyl, 2-methyl-2-propenyl, and the like. Examples of alkynyl groups include ethynyl, 1-propynyl, 2-propynyl, and so forth. The terms "lower alkenyl" and "lower alkynyl" refer to alkenyl and alkynyl groups, respectively, having from 1 to 6 carbon atoms.

As used herein, "cycloalkyl" is intended to refer to a hydrocarbon ring being part of any stable monocyclic or polycyclic system, where such ring has between 3 and 12 carbon atoms, but no heteroatom, and where such ring is fully saturated, and the term "cycloalkenyl" is intended to refer to a hydrocarbon ring being part of any stable monocyclic or polycyclic system, where such ring has between 3 and 12 carbon atoms, but no heteroatom, and where such ring is at least partially unsaturated (but excluding any aryl ring). Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl. For the sake of clarity, if a substituent is a polycyclic ring system as described above wherein one ring is a least partially unsaturated, then such substituent will be referred to as "cycloalkenyl", if substitution occurs via the at least partially unsaturated ring, and as "cycloalkyl", if substitution occurs via a fully saturated ring.

The term "C_{x-y}-", when used in combination with a group as defined herein, is intended to indicate the range of carbon atoms being present in the respective group, excluding substituents. For example, the term "C₁₋₆-alkyl" refers to the alkyl groups having between one carbon atom (i.e. a methyl group) and six carbon atoms (e.g. n-hexyl); the term "C₃₋₆-cycloalkyl" " refers to cycloalkyl groups having between three carbon atom (i.e. a cyclopropyl group) and six carbon atoms (i.e.. cyclohexyl).

As used herein, the terms "heterocycloalkyl" and "heterocycloalkenyl", are intended to refer to a ring being part of any stable monocyclic or polycyclic ring system, where such ring has between 4 and 8 atoms, and where such ring consists of carbon atoms and at least one heteroatom, particularly at least one heteroatom independently selected from the group consisting of N, O and S, with heterocycloalkyl referring to such a ring that is fully saturated, and heterocycloalkenyl referring to a ring that is at least partially unsaturated (but excluding any aryl or heteroaryl ring). For the sake of clarity, if a substituent is a polycyclic ring system wherein one ring contains at least one heteroatom as described herein, then such substituent will be referred to as "heterocycloalkyl/- alkenyl", if substitution occurs via the ring containing the heteroatom(s). Heterocycloalkyl and heterocycloalkenyl groups may be linked to other groups via a carbon ring atom ("C-linked heterocycloalkyl" and "C-linked heterocycloalkenyl", respectively), or via a nitrogen ring atom ("N-linked heterocycloalkyl" and "N-linked heterocycloalkenyl", respectively). Heteroatoms such as nitrogen and sulfur may optionally be oxidized to form N-oxides or sulfoxides and sulfones, respectively. In certain embodiments, a nitrogen in the heterocycle may be quaternized. In certain embodiments, where the total number of S and O atoms in the heterocycle exceeds 1, these heteroatoms are not adjacent to one another. In particular embodiments, the total number of S atoms in the heterocycle is not more than 1.

Examples of heterocycloalkyls include, but are not limited to, pyrrolidinyl, tetrahydrofuranyl, morpholinyl, thiomorpholinyl, piperazinyl, piperidinyl, and decahydroquinolinyl (when linked via the piperidinyl moiety). Examples of heterocycloalkenyls include, but are not limited to, pyrrolinyl, and indolenyl (when linked via the 5-membered ring). Also included are fused ring systems sand spiro compounds containing, for example, any of the above heterocycles, in each case when linked via a heteratom-containing ring.

The term "aryl" is intended to mean a ring or ring system being part of any stable monocyclic or polycyclic system, where such ring or ring system has between 3 and about 20 carbon atoms, but has no heteroatom, and where the ring or ring system consists of an aromatic moiety as defined by the "(4n+2)" *π*-electron rule". For the sake of clarity, if a substituent is a polycyclic system wherein one ring or ring system comprised in said polycyclic system consists of an aromatic moiety as defined herein, then such substituent will be referred to as "aryl", if substitution occurs via said aromatic moiety. This includes, but is not limited to, phenyl and fused benzene ring systems, for example, naphthalene, anthracene, or phenanthrene ring systems, or, for example, a benzene ring fused to one or more cycloalkyl moieties to form, for example, indanyl, fluorenyl or tetrahydronaphthyl (tetralin), or fused to one or more heterocycloalklyl rings, e.g. as in indolenyl, *provided, however,* that in each such case, such fused system is linked as a substituent via the aromatic aromatic moiety.

As used herein, the term "heteroaryl" refers to a ring or ring system being part of any stable mono- or polycyclic system, where such ring or ring system has between 3 and about 20 atoms, which ring or ring system consists of an aromatic moiety as defined by the "(4n+2)" *π*-electron rule" and which contains carbon atoms and one or more nitrogen, sulfur, and/or oxygen heteroatoms. For the sake of clarity, if a substituent is a polycyclic system wherein one ring or ring system comprised in said polycyclic system consists of an aromatic moiety containing a heteroatom as defined herein, then such substituent will be referred to as "heteroaryl", if substitution occurs via the aromatic moiety containing the heteroatom. In certain embodiments, the total number of N, S and O atoms in the heteroaryl is between 1 and about 4. In certain embodiments, the total number of S and O atoms in the aromatic heteroaryl is not more than 1. In certain embodiments, a nitrogen in the heterocycle may be quaternized or oxidized to an N-oxide. Examples of heteroaryls include, but are not limited to, pyrrolyl, pyrazolyl, imidazolyl, indolyl, benzimidazolyl, furanyl, benzofuranyl, thiophenyl, benzothiophenyl, pyridinyl, pyrimidinyl, pyrazinyl, triazinyl, quinolinyl, quinazolinyl Also included in the term heteroaryl are fused heteroaryls containing, for example, the above heteroaryls fused to cycloalkyls or heterocycloalkyls (provided, in each case, that such fused system is linked as a substituent via the aromatic moiety containing at least one heteroatom). Wherever indicated herein, alkyl, alkenyl, alkynyl, cycloalkyl, cycloalkenyl, heterocycloalkyl, heterocycloalkenyl, aryl and heteroaryl groups as well as and any other substructure comprising at least one hydrogen in the substructure may be substituted by one or more substituents.

The term "substituted" is intended to indicate that one or more hydrogens on the atom or group indicated in the expression using "substituted" is replaced with a selection from the indicated group(s), provided that the indicated atom's normal valency, or that of the appropriate atom of the group that is substituted, is not exceeded, and that the substitution results in a stable compound. The terms "substituted or unsubstituted" or "optionally substituted" are intended to mean that a given compound, or substructure of a compound, is either unsubstituted, or substituted, as defined herein, with one or more substituents, as indicated or as defined below.

In a substituted moiety, unless a specific list of substituents is explicitly provided, at least one hydrogen atom of the moiety "M" to be substituted is replaced by a substituent -[(R^{Sm})ₙ], wherein m is an integer running from 1 to n, wherein n is any value from 1 to about 15, wherein each R^{Sm} is independently selected from the following lists (A) and (B):
(A): alkyl, alkenyl, alkynyl, cycloalkyl, cycloalkenyl, C-linked heterocycloalkyl, C-linked heterocycloalkenyl, aryl, C-linked heteroaryl, -O-R^{a}, -O-N(R^{a})₂, -S-R^{a}, -S-N(R^{a})₂, N-linked heterocycloalkyl, N-linked heteroaryl, -N(R^{a})-R^{a}, - N(R^{a})-OR^{a}, -N(R^{a})-SR^{a}, -N(R^{a})-N(R^{a})₂, -F, -Cl, -Br, -I;
(B): -C(=O)-R^{a}, -C(=S)-R^{a}, -C(=NR^{a})-R^{a}, -C(=N-OR^{a})-R^{a}, -C(=N-N(R^{a})₂)-R^{a}, -C(=O)-OR^{a}, -C(=S)-OR^{a}, -C(=O)-N(R^{a})₂, -C(=S)-N(R^{a})₂, -C(=NR^{a})-N(R^{a})₂, -C(=N-OR^{a})-N(R^{a})₂, -C(=N-N(R^{a})₂)-N(R^{a})₂, -C(=O)-N(R^{a})-N(R^{a})₂, -C(=S)-N(R^{a})-N(R^{a})₂, -C(=NR^{a})-N(R^{a})-N(R^{a})₂, -CN, -O-N=C(R^{a})₂, -O-C(=O)-R^{a}, -O-C(=S)-R^{a}, -0-C(=NR^{a})-R^{a}, -O-C(=N-OR^{a})-R^{a}, -O-C(=N-N(R^{a})₂)-R^{a}, -O-C(=NR^{a})-OR^{a}, -O-C(=NR^{a})-SR^{a}, -O-C(=NR^{a})-N(R^{a})₂, -S-N(R^{a})-N(R^{a})₂, -S-N=C(R^{a})₂, -S-N=NR^{a}, -SO-R^{a}, -SO-OR^{a}, -SO-SR^{a}, -SO-N(R^{a})₂, - SO-N(R^{a})-N(R^{a})₂, -SO₂-R^{a}, -SO₂-OR^{a}, -SO₂-SR^{a}, -SO₂-N(R^{a})₂, - SO₂-N(R^{a})-N(R^{a})₂, -N(R^{a})-N=C(R^{a})₂, -N(R^{a})-C(=O)-R^{a}, -N(R^{a}) - C(=S)-R^{a}, -N(R^{a})-C(=NR^{a})-R^{a}, -N(R^{a})-C(=N-OR^{a})-R^{a}, -N(R^{a})-C(=N-N(R^{a})₂)-R^{a}, -N(R^{a})-C(=O)-OR^{a}, -N(R^{a})-C(=S)-OR^{a}, -N(R^{a}) - C(=NR^{a})-OR^{a}, -N(R^{a})-C(=N-OR^{a})-OR^{a}, -N(R^{a})-C(=N-N(R^{a})₂)-OR^{a}, - N(R^{a})-C(=O)-N(R^{a})₂, -N(R^{a})-C(=S)-N(R^{a})₂, -N(R^{a})-C(=NR^{a})-N(R^{a})₂, -N(R^{a})-C(=N-OR^{a})-N(R^{a})₂, -N(R^{a})-C(=N-N(R^{a})₂)-N(R^{a})₂,-N(R^{a})-C(=O)-N(R^{a})-N(R^{a})₂, -N(R^{a})-C(=S)-N(R^{a})-N(R^{a})₂, -N(R^{a})-C(=NR^{a})-N(R^{a})-N(R^{a})₂, -N(R^{a})-C(=N-OR^{a})-N(R^{a})-N(R^{a})₂, -N(R^{a})- C(=N-N(R^{a})₂)-N(R^{a})-N(R^{a})₂, -N(R^{a})-N(R^{a})-C(=O)-R^{a}, -N(R^{a})-N(R^{a})-C(=S)-R^{a}, -N(R^{a})-N(R^{a})-C(=NR^{a})-R^{a}, -N(R^{a})-N(R^{a})-C(=NOR^{a})-R^{a}, -N(R^{a})-N(R^{a})-C(=N-N(R^{a})₂) -R^{a}, -N(R^{a})-N(R^{a})-C(=O)-OR^{a}, -N(R^{a})-N(R^{a})-C(=S)-OR^{a}, -N(R^{a})-N(R^{a})-C(=NR^{a})-OR^{a}, -N(R^{a})-N(R^{a})-C(=N-OR^{a})-OR^{a}, -N(R^{a})-N(R^{a})-C(=N-N(R^{a})₂)-OR^{a}, -N(R^{a})-N(R^{a})-C(=O)-N(R^{a})₂, -N(R^{a})-N(R^{a})-C(=S)-N(R^{a})₂, -N(R^{a})-N(R^{a})-C(=NR^{a})-N(R^{a})₂, -N(R^{a})-N(R^{a})-C(=N-OR^{a})-N(R^{a})₂, -N(R^{a})-N(R^{a})-C(=N-N(R^{a})₂)-N(R^{a})₂, -N(R^{a})-N(R^{a})-C(=O)-N(R^{a})-N(R^{a})₂, -N(R^{a})-N(R^{a})-C(=S)-N(R^{a})-N(R^{a})₂, -N(R^{a})-N(R^{a})-C(=NR^{a})-N(R^{a})-N(R^{a})₂, - N(R^{a})-N(R^{a})-C(=N-OR^{a})-N(R^{a})-N(R^{a})₂, -N(R^{a})-N(R^{a})-C(=N-N(R^{a})₂) - N(R^{a})-N(R^{a})₂*,* -N(R^{a})-N(R^{a})-C(=NR^{a})-N=C(R^{a})₂, -N(R^{a})-N(R^{a})-C(=N-OR^{a})-N=C(R^{a})₂, -N(R^{a})-N(R^{a})-C(=N-N(R^{a})₂)-N=C(R^{a})₂,-N(R^{a})-SO-R^{a}, -N(R^{a})-SO-OR^{a}, -N(R^{a})-SO-N(R^{a})₂, -N(R^{a})-SO-NR^{a}-N(R^{a})₂, -N(R^{a})-SO₂-R^{a}, -N(R^{a})-SO₂-OR^{a}, -N(R^{a})-SO₂-N(R^{a})₂,-N(R^{a})-SO₂-NR^{a}-N(R^{a})₂, -NO₂, -N₃, -F, -Cl, -Br, -I; wherein each R^{a} is independently selected from -H and R^{b}, with R^{b} being independently selected from alkyl, alkenyl, alkynyl, cycloalkyl, cycloalkenyl, C-linked heterocycloalkyl, C-linked heterocycloalkenyl, aryl, and C-linked heteroaryl, and wherein alternatively two substituents R^{b}, when attached to the same nitrogen atom, can form together with the nitrogen atom they are attached to a heterocycloalkyl, heterocycloalkenyl, or heteroaryl group, and wherein R^{S1} is attached to such moiety M, and wherein any additional R^{Sx}, if present, is substituting a hydrogen atom in a residue R^{b} of a group R^{Sy}, with x > y.

A methylene group present in a ring of a cycloalkyl, cycloalkenyl, heterocycloalkyl, and heterocycloalkenyl moiety may as well be substituted by a substituent R^{S} taken from the following list (C):
(C) : =O, =S, =NR^{a}, =N-OR^{a}, and =N-N(R^{a})₂, wherein for each such substituent both hydrogens on the methylene group of the unsubstituted moiety are being replaced. Nitrogen-containing substructures, such as amines or nitrogen-containing heterocycles, may be substituted as well by formation of quaternary amines or N-oxides, wherein R^{Sm} is alkyl or -O.

For example, a substituent -CH₂-O-CH₂-CH₂-F can be described as - [(R^{Sm})₃] with R^{S3} being -F that substitutes a hydrogen atom in R^{S2} being -O-C₂-alkyl, where such R^{S2} substitutes a hydrogen atom in R^{S1} being C₁-alkyl. In this example, the individual elements R^{Sm} in -[(R^{Sm})₃] are arranged in a "linear" fashion, since every R^{Sx} is linked to a R^{Sy} with x = y +1 (i.e. R^{S3} is linked to R^{S2}, and so on).

As a second example, the substituent -CH₂-O-CH₂-CF₃ can be described as - [(R^{Sm})₅] with each of R^{S3}, R^{S4}, and R^{S5} being a -F atom that substitutes a hydrogen atom in R^{S2} being -O-C₂-alkyl, where such R^{S2} substitutes a hydrogen atom in R^{S1} being C₁-alkyl. In this example, the individual elements R^{Sm} in -[(R^{Sm})₆] are arranged in a "branched" fashion, since at least one R^{Sx} (here, for example, R^{S4} or R^{S5}) is linked to a R^{Sy} (here R^{S2}) with x > y +1.

Table 10 in the Examples section demonstrates the substitution pattern at R¹ and R³ for exemplary compounds of the present invention.

As will be readily apparent to anyone of ordinary skill in the art, there are manifold combinations of individual elements R^{Sm} possible. However, as is apparent to one of ordinary skill in the art as well, there are certain combinations that result in are less favored arrangement and that will not be considered in the design and synthesis of compounds in accordance with the present invention, because of low stability, high reactivity, or unfavorable physicochemical properties, such as low solubility. Examples include, but are not limited to, semi-acetals or -ketals (-O-CR₂-OH), Michael systems (e.g. -C(=O)-CR=CR₂), □□halogen carbonyl compounds (e.g. -C(=O)-CR₂-Br), polycyclic aromatic systems, and the like.

In particular embodiments, the number of elements R^{Sm} in a substituent -[(R^{Sm})ₙ], that are selected from the lists (B) and (C) listed above, is not more than two. In particular embodiments, there is not more than one element R^{Sm} in a substituent -[(R^{Sm})ₙ], that is selected from the lists (B) or (C) listed above.

In particular embodiments, n in a substituent -[(R^{Sm})ₙ] is any value from 1 to about 12, from 1 to about 10, from 1 to about 8, or from 1 to about 6.

In particular embodiments, n in a substituent -[(R^{Sm})ₙ] for all substituents different from -F or -Cl is any value from 1 to about 6, from 1 to about 5, from 1 to about 4, or from 1 to about 3.

In particular embodiment, the number of atoms in a substituent -[(R^{Sm})ₙ] is any value from 1 to 80, from 1 to 60, from 1 to 50, from 1 to 40, from 1 to 30, from 1 to 20, from 1 to 15, or from 1 to 10.

In particular embodiment, the combined molecular weight of all atoms in a substituent -[(R^{Sm})ₙ] is any value from 1 to 360, from 1 to 300, from 1 to 240, from 1 to 180, from 1 to 120, from 1 to 90, or from 1 to 60.

The term "halogen" or "halo" refers to fluoro, chloro, bromo and iodo substituents.

The terms "stereoisomer" and "tautomer" as used herein include all possible stereoisomeric and tautomeric forms of the compounds of the present invention. Where the compounds of the present invention contain one or more chiral centers, all possible enantiomeric and diastereomeric forms are included, unless indicated otherwise.

The present invention is intended to include all isotopes of atoms occurring on the present compounds. Isotopes are atoms having the same atomic number but different mass numbers. By way of general example and without limitation, isotopes of hydrogen include tritium and deuterium. Isotopes of carbon include ¹²C, ¹³C and ¹⁴C.

The term "metabolite", as used herein, refers to any substance produced by the metabolism or by a metabolic process. Metabolism, as used herein, refers to the various physical/chemical/biochemical/pharmacological reactions involved in the transformation of molecules or chemical compounds occurring in the cell, tissue, system, body, animal, individual, patient or human therein.

The term "IC₅₀", as used herein, refers to concentrations at which a measurable activity, phenotype or response, for example growth or proliferation of cells such as tumor cells, is inhibited by 50%. IC₅₀ values can be estimated from an appropriate dose-response curve, for example by eye or by using appropriate curve fitting or statistical software. More accurately, IC₅₀ values may be determined using non-linear regression analysis.

As used herein, an "individual" means a multi-cellular organism, for example an animal such as a mammal, including a primate. In addition to primates, such as humans, a variety of other mammals can be treated according to a method that utilizes one or more compounds of the present invention. For example, mammals including, but not limited to, cows, sheep, goats, horses, dogs, cats, guinea pigs, rats or other bovine, ovine, equine, canine, feline, rodent, or murine species can be used.

As used herein, a "proliferative disorder" or a "proliferative disease" comprises a disease or disorder that affects a cellular growth, differentiation, or proliferation process.

As used herein, a "cellular growth, differentiation or proliferation process" is a process by which a cell increases in number, size or content, by which a cell develops a specialized set of characteristics which differ from that of other cells, or by which a cell moves closer to or further from a particular location or stimulus. A cellular growth, differentiation, or proliferation process comprises amino acid transport and degradation and other metabolic processes of a cell. A cellular proliferation disorder may be characterized by aberrantly regulated cellular growth, proliferation, differentiation, or migration. Cellular proliferation disorders comprise tumorigenic diseases or disorders.

As used herein, a "tumorigenic disease or disorder" comprises a disease or disorder characterized by aberrantly regulated cellular growth, proliferation, differentiation, adhesion, or migration, which may result in the production of or tendency to produce tumors. As used herein, a "tumor" comprises a benign or malignant mass of tissue. Examples of cellular growth or proliferation disorders include, but are not limited to tumors, cancer, autoimmune diseases, viral diseases, fungal diseases, neurodegenerative disorders and cardiovascular diseases.

As used herein, the terms "anti-cancer agent" or "antiproliferative agent" refer to compounds with anti-cancer and anti-proliferative properties, respectively. These compounds include, but are not limited to, altretamine, busulfan, chlorambucil, cyclophosphamide, ifosfamide, mechlorethamine, melphalan, thiotepa, cladribine, fluorouracil, floxuridine, gemcitabine, thioguanine, pentostatin, methotrexate, 6-mercaptopurine, cytarabine, carmustine, lomustine, streptozotocin, carboplatin, cisplatin, oxaliplatin, picoplatin, LA-12, iproplatin, tetraplatin, lobaplatin, JM216, JM335, satraplatin, fludarabine, aminoglutethimide, flutamide, goserelin, leuprolide, megestrol acetate, cyproterone acetate, tamoxifen, anastrozole, bicalutamide, dexamethasone, diethylstilbestrol, prednisone, bleomycin, dactinomycin, daunorubicin, doxirubicin, idarubicin, mitoxantrone, losoxantrone, mitomycin-c, plicamycin, paclitaxel, docetaxel, topotecan, irinotecan, 9-amino camptothecan, 9-nitro camptothecan, GS-211, JM 118, etoposide, teniposide, vinblastine, vincristine, vinorelbine, procarbazine, asparaginase, pegaspargase, octreotide, estramustine, and hydroxyurea. Said terms also include, but are not limited to, non-small molecule therapeutics, such as antibodies, e.g., 1D09C3 and other anti-HLA-DR antibodies as described in WO 01/87337 and WO 01/97338, Rituxan as described in US patents 5,736,137, 5,776,456, 5,843,437, 4D5, Mab225, C225, Daclizumab (Zenapax), Antegren, CDP 870, CMB-401, MDX-33, MDX-220, MDX-477, CEA-CIDE, AHM, Vitaxin, 3622W94, Therex, 5G1.1, IDEC-131, HU-901, Mylotarg, Zamyl (SMART M195), MDX-210, Humicade, LymphoCIDE, ABX-EGF, 17-1A, Trastuzumab (Herceptin^{®}, rhuMAb), Epratuzumab, Cetuxi-mab (Erbitux^{®}), Pertuzumab (Omnitarg^{®}, 2C4), R3, CDP860, Bevacizumab (Avastin^{®}), tositumomab (Bexxar^{®}), Ibritumomab tiuxetan (Zevalin^{®}), M195, 1D10, Hu1D10 (Remitogen^{®}, apolizumab), Danton/DN1924, an "HD" antibody such as HD4 or HD8, CAMPATH-1 and CAMPATH-1H or other variants, fragments, conjugates, derivatives and modifications thereof, or other equivalent compositions with improved or optimized properties, and proteins or peptides, e.g., those described in Trends in Biotechnology (2003), 21(12), p.556-562.

As used herein, an "inflammatory disorder" or an "inflammatory disease" comprises a disease or disorder that is caused or accompanied by inflammatory processes. This includes, but is not limited to, diseases or disorders such as arthritis, including but not limited to rheumatoid arthritis, spondyloarthropathies, gouty arthritis, osteoarthritis, systemic lupus erythematosus and juvenile arthritis, osteoarthritis, gouty arthritis and other arthritic conditions; pulmonary disorders or lung inflammation, including adult respiratory distress syndrome, pulmonary sarcoidosis, asthma, silicosis, and chronic pulmonary inflammatory disease; viral and bacterial infections, including sepsis, septic shock, gram negative sepsis, malaria, meningitis, cachexia secondary to infection or malignancy, cachexia secondary to acquired immune deficiency syndrome (AIDS), AIDS, ARC (AIDS related complex), pneumonia, and herpes virus; bone resorption diseases, such as osteoporosis, endotoxic shock, toxic shock syndrome, reperfusion injury, autoimmune disease including graft vs. host reaction and allograft rejections, cardiovascular diseases including atherosclerosis, thrombosis, congestive heart failure, and cardiac reperfusion injury, renal reperfusion injury, liver disease and nephritis, and myalgias due to infection; Alzheimer's disease, influenza, multiple sclerosis, cancer, diabetes, systemic lupus erythematosus (SLE), skin-related conditions such as psoriasis, eczema, burns, dermatitis, keloid formation, and scar tissue formation; gastrointestinal conditions such as inflammatory bowel disease, Crohn's disease, gastritis, irritable bowel syndrome and ulcerative colitis; ophthalmic diseases, such as retinitis, retinopathies, uveitis, ocular photophobia, and of acute injury to the eye tissue; angiogenesis, including neoplasia; metastasis; ophthalmological conditions such as corneal graft rejection, ocular neovascularization, retinal neovascularization including neovascularization following injury or infection, diabetic retinopathy, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; diabetic nephropathy and cardiomyopathy; and disorders of the female reproductive system such as endometriosis.

As used herein, "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from a parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, EtOAc, ethanol, isopropanol, or acetonitrile are preferred. Lists of suitable salts are found in Remington's Pharmaceutical Sciences, 18th ed., Mack Publishing Company, Easton, PA, 1990, p. 1445, the disclosure of which is hereby incorporated by reference.

Any salt that retains the desired biological activity of the compounds contained herein and that exhibits minimal or no undesired or toxicological effects is intended for inclusion here. Pharmaceutically acceptable salts comprise those derived from pharmaceutically acceptable organic or inorganic acids and bases. Non-pharmaceutically acceptable acids and bases also find use herein, as for example, in the synthesis and/or purification of the compounds of interest. Thus, all "salts" are also encompassed within the scope of the instant invention.

Non-limiting examples of suitable salts include those derived from inorganic acids, such as, for example, hydrochloric acid, hydrobromic acid, sulfuric acid, phosphoric acid, nitric acid, bicarbonic acid, carbonic acid; and salts formed with organic acids, such as, for example, formic acid, acetic acid, oxalic acid, tartaric acid, succinic acid, malic acid, malonic acid, ascorbic acid, citric acid, benzoic acid, tannic acid, palmoic acid, alginic acid, polyglutamic acid, tosic acid, methanesulfonic acid, naphthalenesulfonic acid, naphthalenedisulfonic acid, α-ketoglutaric acid, β-glycerophosphoric acid and polygalacturonic acid. Suitable salts include those derived from alkali metals such as lithium, potassium and sodium, from alkaline earth metals such as calcium and magnesium, as well as from other acids well known to those of skill in the pharmaceutical art. Other suitable salts include those derived from metal cations such as zinc, bismuth, barium, or aluminum, or with a cation formed from an amine, such as ammonia, N,N-dibenzylethylene-diamine, D-glucosamine, tetraethylammonium, or ethylenediamine. Moreover, suitable salts include those derived from a combination of acids and bases, such as, for example, a zinc tannate salt.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The term "prodrug", as used herein, refers to an agent that is converted into a pharmacologically active parent drug in vivo, such as a compound as defined herein. The term "prodrug" comprises any covalently bonded carriers that release an active parent drug of the present invention in vivo when such prodrug is administered to an animal. Since prodrugs are known to enhance numerous desirable qualities of pharmaceuticals (e.g., solubility, bioavailability, manufacturing, transport, pharmacodynamics, etc.), the compounds of the present invention may be delivered in prodrug form. Prodrugs, for instance, may be bioavailable by oral administration even when the parent drug is not. Thus, the present invention is intended to cover prodrugs of the presently claimed compounds, methods of delivering the same, and compositions containing the same. Prodrugs of the present invention are prepared by modifying functional groups present in the compound in such a way that the modifications are cleaved, either in routine manipulation or in vivo, to the parent compound. Prodrugs include compounds of the present invention wherein a hydroxy, amino, or sulfhydryl group is bonded to any group that, when the prodrug of the present invention is administered to a mammalian subject, it cleaves to form a free hydroxyl, free amino, or free sulfhydryl group, respectively. Examples of prodrugs include, but are not limited to, acetate, formate, and benzoate derivatives of alcohol and amine functional groups in the compounds of the present invention.

Generally speaking, prodrugs are derivatives of *per* se drugs that after administration undergo conversion or metabolism to the physiologically active species. The conversion may be spontaneous, such as hydrolysis in the physiological environment, or may be enzyme-catalyzed. Prodrugs include compounds that can be oxidized, reduced, aminated, deaminated, hydroxylated, dehydroxylated, hydrolyzed, esterified, alkylated, dealkylated, acylated, deacylated, phosphorylated, and/or dephosphorylated to produce the active compound.

From among the voluminous scientific literature devoted to prodrugs in general, the foregoing examples are cited: Gangwar et al., "Prodrug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic used", Drugs 45(6): 866-94 (1993); Sinhababu and Thakker, "Prodrugs of anticancer agent". Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999); Design of Prodrugs (Bundgaard H. ed.) 1985 Elsevier Science Publishers B. V. (Biomedical Division), Chapter 1; Design of Prodrugs: Bioreversible derivatives for various functional groups and chemical entities (Hans Bundgaard); Bundgaard et al. Int. J. of Pharmaceutics 22 (1984) 45 - 56 (Elsevier); Bundgaard et al. Int. J. of Pharmaceutics 29 (1986) 19 - 28 (Elsevier); Bundgaard et al. J. Med. Chem. 32 (1989) 2503 - 2507 Chem. Abstracts 93, 137935y (Bundgaard et al.); Chem. Abstracts 95, 138493f (Bundgaard *et al.);* Chem. Abstracts 95, 138592n (Bundgaard *et al.);* Chem. Abstracts 110, 57664p (Alminger *et al.);* Chem. Abstracts 115, 64029s (Buur et al.); Chem. Abstracts 115, 189582y (Hans*en et al.);* Chem. Abstracts 117, 14347q (Bundgaard *et al.);* Chem. Abstracts 117, 55790x (Jens*en et al.*); and Chem. Abstracts 123, 17593b (Thomsen et all.).

The terms "administered", "administration", or "administering" a compound will be understood to mean providing any compound of the invention to an individual, including an animal, in need of treatment by bringing such individual in contact with, or otherwise exposing such individual to, such compound.

The term "in vitro" refers to a biological entity, a biological process, or a biological reaction outside the body in artificial conditions. For example a cell grown in vitro is to be understood as a cell grown in an environment outside the body, e.g., in a test tube, a culture tray or a microtiter plate.

The term "therapeutically effective amount" means the amount of the subject compound that will elicit the biological, physiological, pharmacological, therapeutic or medical response of a cell, tissue, system, body, animal, individual, patient or human that is being sought by the researcher, scientist, pharmacologist, pharmacist, veterinarian, medical doctor, or other clinician, e.g., lessening of the effects/symptoms of a disorder or disease, such as a proliferative disorder or disease, for example, a cancer or tumor, or killing or inhibiting growth of a proliferating cell, such as a tumor cell. The therapeutically effective amount can be determined by standard procedures, including those described below in the section "Dosages".

The term "further treated", "further administer", or "further administered" means that different therapeutic agents or compounds may be administered together, alternatively or intermittently. Such further administration may be temporally or spatially separated, for example, at different times, on different days or via different modes or routes of administration.

### Certain compounds of the present invention

Those skilled in the art will recognize that all specific combinations of the individual possible residues of the variable regions of the compounds as disclosed herein, i.e. X¹, X², X³, X⁴, Y¹, Y², Yⁿ⁺², n, Z, R¹, R², R³, R⁴, R^{4'}, R⁵, R⁶, R⁷, and R⁸ are within the scope of the invention.

One embodiment of the invention relates to a compound having a structure represented by formula (I) or any tautomeric or stereoisomeric form thereof, wherein one or more hydrogen atoms in moieties indicated as being optionally substituted ("unsubstituted or substituted") in one or more of R¹ to R⁸ may be independently substituted by one or more substituents -[(R^{Sm})ₙ], as described above.

Table 10 shows a selection of exemplary compounds of the present invention having a substituted aryl as R¹, i.e. a group -R¹-[(R^{Sm})ₙ], or a substituted alkyl as R³, i.e. a group -R³-[(R^{Sm})ₙ], and how the substituent -[(R^{Sm})ₙ] can be dissected in each case.

In one embodiment of the present invention, compounds of the invention have a molecular weight of between 350 and 1000, particularly between 400 and 800, more particularly between 400 and 600, and even more particularly between 400 and 550. In certain embodiments, compounds of the invention have one or more of the following characteristics: (i) not more than 5 hydrogen bond donors, (ii) not more than 10 hydrogen bond acceptors, and (iii) not more than 10 rotatable bonds (excluding bonds to terminal atoms). In certain embodiments, the compounds of formula (I) are in accordance with Lipinski's "Rule of Five" (Lipinski, Adv. Drug Del. Rev. 1997; 23: 3), by having a molecular weight below 500, not more than 5 hydrogen bond donors, not more than 10 hydrogen bond acceptors, and a cLogP value between -2 and 5.

### (iv) Specific embodiments of the invention

In one embodiment of the present invention, compounds of the invention are inhibitors of the activity of Abl, Abl T315I, Aurora A, Aurora B, Axl, BTK, BRAF, BRAF V600E, CDK1, CDK2, CDK4, CDK5, CDK6, CDK9, cKIT, CSF-1R, Csk, DYRK1A, EGFR, EphA2, EphA4, EphB4, FLT3, FLT3 ITD, FLT3 D835Y, FGFR family kinases, Lck, IRAK1, IRAK4, Met, PDGFR-alfa, PDGFR-beta, RET, Ron, Src family kinases (Src, Lyn, Fyn, Hck etc.), TGFR1, TGFR-2, TGFR-3, TrkA, Tyro-3, VEGFR-1, VEGFR-2 and VEFGR-3, particularly cKIT, FLT3, FLT3 ITD, FLT3 D835Y, FGFR family kinases, CSF-1R, PDGFR-alfa, PDGFR-beta, Ron, Src family kinases (Src, Lyn, Fyn, Hck etc.), TGFR1, TGFR-2, TGFR-3, Tyro-3, VEGFR-1, VEGFR-2 and VEFGR-3. In certain embodiments, the compounds of the present invention inhibit BTK, CSK, cKIT, EGFR, FLT3, FLT3 ITD, IRAK-4, Lyn, Lck, PDGFR-alfa, PDGFR-beta, RON, VEGFR-1, and/orVEGFR-2 activity with IC₅₀ values below 1 µM, such IC₅₀ value being determined in accordance with the ELISA or HTRF inhibition assays described in the examples shown below. In certain embodiments, the IC₅₀ value is below 0.5 µM, below 0.2 µM, below 0.1 µM, or below 0.01 µM.

A compound of the present invention may exist in one or more crystalline forms, including two or more polymorphic forms, and may exist as dry solids or as solvates including defined amounts of solvents, including hydrates including defined amounts of water.

In another embodiment, compounds of the invention are the planned and deliberate products of a synthetic chemistry scheme, i.e., produced by specific and planned chemical processes conducted in reaction vessels, and not by degradation, metabolism or fermentation, or produced as impurities or by-products in the synthesis of other compounds.

In certain embodiments of the present invention, a compound is purified or isolated, e.g., to have a purity of at least 80%, particularly at least 90%, more particularly at least 95%, such as at least 97%, at least 98% or even at least 99%. Purity, as used herein, can refer to either absolute or relative purity. Absolute purity refers to the amount of a compound of the invention obtained as the product of a synthetic chemistry scheme, either before or after one or more purification steps. Relative purity refers to the amount of a compound of the invention relative to one or more impurities such as by-products, degradation products (e.g., metabolites, products of oxidation or hydrolysis, etc.) and/or compounds that degrade to form a compound of the invention (e.g., precursors or prodrugs), e.g., that may be present in the product of a synthetic chemistry scheme. Thus, absolute purity refers to the amount of a compound relative to all others, while relative purity is generally unaffected by the addition of unrelated compounds, such as excipients, stabilizers, or other medicaments for conjoint administration. Purity can be assessed based upon weight, volume or molar ratios of one compound relative to others. Purity can be measured by a variety of analytical techniques, including elemental abundance, UV-visible spectrometry, HPLC, GC-MS, NMR, mass spectrometry, and thin layer chromatography, preferably by HPLC, GC-MS, or NMR.

Yet another aspect of the invention relates to prodrugs of a compound described above.

### Particular embodiments

In particular embodiments, the present invention relates to a compound having a structure represented by formula (I) or any tautomeric or stereoisomeric form thereof as defined above, wherein R⁴ is selected from unsubstituted or substituted -alkyl.

In particular embodiments, the invention relates to a compound having a structure represented by formula (I) or any tautomeric or stereoisomeric form thereof as defined above, wherein R¹ is selected from -hydrogen, -halogen, unsubstituted or substituted -C₁₋₄-alkyl, unsubstituted or substituted -C₂₋₄-alkynyl, unsubstituted or substituted -aryl, unsubstituted or substituted -heteroaryl, -C(=O)O-C₁₋₄-alkyl, -C(=O)N(C₁₋₄-alkyl)₂, and -CN.

In particular embodiments, the invention relates to compounds, wherein R¹ is selected from alkyl, particularly propyl or 2,2-dimethylpropyl; phenyl; fluoro-substituted phenyl, particularly 2-fluorophenyl, 3-fluorophenyl, 4-fluorophenyl, 3,4-difluorophenyl, or 3,5-difluorophenyl; fluoroalkyl, particularly 2,2,2-trifluoroethyl; substituted ethenyl, particularly 2-phenylethenyl; substituted ethinyl, particularly cyclopropylethinyl; N-aryl aminocarbonyl, particularly N-(2-chlorophenyl)-aminocarbonyl, and optionally substituted pyridyl, particularly 2-chloro-pyridin-4-yl.

In particular embodiments, the invention relates to compounds represented by formula (I), wherein X¹ is N, and X² to X⁴ are independently CR⁶, particularly wherein R⁶ is hydrogen.

In particular embodiments, the invention relates to compounds represented by formula (I), wherein X³ is N, and X¹, X² and X⁴ are independently CR⁶, particularly wherein R⁶ is hydrogen.

In particular embodiments, the invention relates to compounds represented by formula (I), wherein X⁴ is N, and X¹ to X³ are independently CR⁶, particularly wherein R⁶ is hydrogen.

In particular embodiments, the invention relates to compounds represented by formula (I), wherein X¹ and X⁴ are N, and X² and X³ are independently CR⁶, particularly wherein both R⁶ are hydrogen, wherein X² is methyl and X³ is hydrogen, or wherein X² is hydrogen and X³ is fluoro.

In particular embodiments, the invention relates to compounds represented by formula (I), wherein X¹ and X³ are N, and X² and X⁴ are independently CR⁶, particularly wherein X² is methyl and X³ is hydrogen.

In particular embodiments, the invention relates to compounds represented by formula (I), wherein X² and X⁴ are N, and X¹ and X³ are independently CR⁶, particularly wherein R⁶ is hydrogen.

In particular embodiments, Z is selected from a direct bond, methylene or ethylene.

In particular embodiments, Z is selected from a direct bond, and -CH₂-.

In particular embodiments, the invention relates to a compound having a structure represented by formula (I) or any tautomeric or stereoisomeric form thereof as defined above, wherein n is selected from 2, 3 and 4.

In particular embodiments, a maximum of two of Y¹ to Yⁿ⁺² in 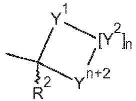 carry a substituent R³ and/or R⁷ different from hydrogen.

In a particular embodiment, the invention relates to a compound represented by a structure of formula (Ia) 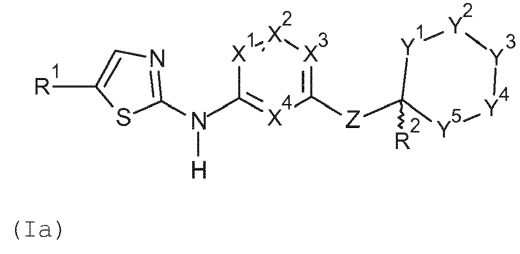 or any tautomeric or stereoisomeric form thereof, wherein Y³ and Y⁴ correspond to Y² as defined above, wherein Y⁵ corresponds to Yⁿ⁺² as defined above, and wherein X¹, X², X³, X⁴, Y¹, Y², Z, R¹, R², R³, R⁴, R^{4'}, R⁵, R⁶, R⁷, and R⁸ are as defined above.

In certain embodiments, the invention relates to a compound represented by a structure of formula (Ib) 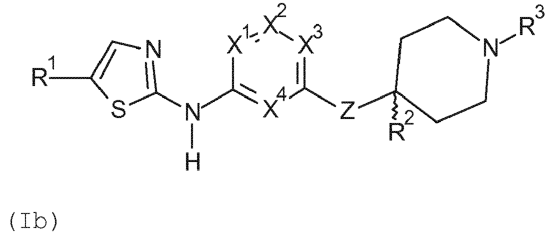 or any tautomeric or stereoisomeric form thereof, wherein X¹, X², X³, X¹, Z, R¹, R², R³, R⁴, R^{4'}, R⁵, R⁶, and R⁸ are as defined above.

In certain embodiments, the invention relates to a compound represented by a structure of formula (Ic) 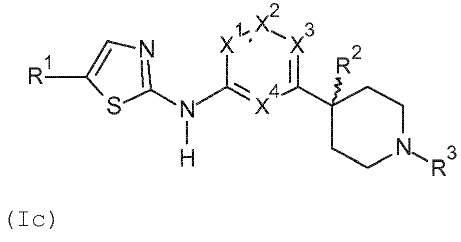 or any tautomeric or stereoisomeric form thereof, wherein X¹, X², X³, X⁴, R¹, R², R³, R⁴, R^{4'}, R⁵, R⁶, and R⁸ are as defined above.

In certain embodiments, the invention relates to a compound represented by a structure of formula (I), wherein the cyclic substructure 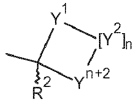 is represented by one of the following cyclic substructures: 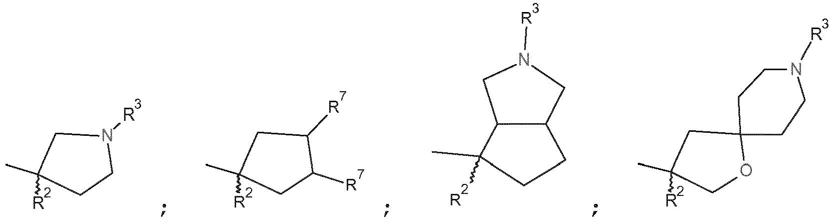 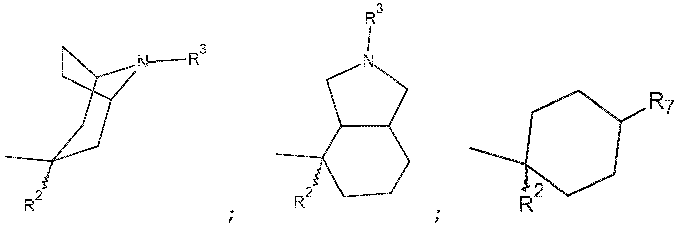 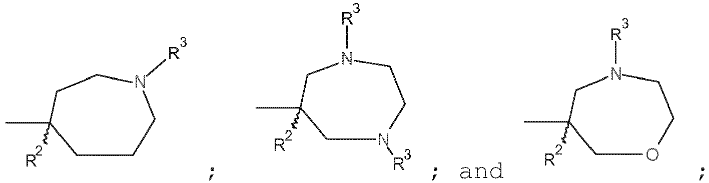 wherein R³ and R⁷ are as defined above.

In particular embodiments of the compounds represented by a structure of formula (I), (Ia), (Ib) or (Ic), the substituent R² is hydrogen.

In particular embodiments of the compounds represented by a structure of formula (I), (Ia), (Ib) or (Ic), each substituent R³ and R⁷ is independently selected from hydrogen; alkyl, particularly methyl; -C(=O)alkyl, particularly - C(=O)-Me and -C(=O)-CMe₂NH₂, hydroxy-substituted alkyl, particularly -CH₂-CH₂-OH and -CH(OH)-CH₂-OH; -SO₂-alkyl, particularly -SO₂-Me; -NR⁷₂, particularly -NH₂ and -NH-Me; and -C(=O)O-alkyl, particularly -C(=O)OMe.

In particular embodiments of the compounds represented by a structure of formula (I), (Ia), (Ib) or (Ic), each substituent R⁷ is independently selected from hydrogen; alkyl, particularly methyl; -C(=O)OR^{4'}, particularly -C(=O)-OMe; and -NR⁷₂.

In certain embodiments of formula (I), the compound is selected from:
[5-((4-Fluoro-phenyl)-thiazol-2-yl]-(4-piperidin-4-ylmethyl-pyridin-2-yl)-amine;
1-((4-{2-[5-(4-Fluoro-phenyl)-thiazol-2-ylamino]-pyridin-4-ylmethyl}-piperidin-1-yl)-ethanone;
2-((4-{2-[5-(4-Fluoro-phenyl)-thiazol-2-ylamino]-pyridin-4-ylmethyl}-piperidin-1-yl)-ethanol;
[5-((4-Fluoro-phenyl)-thiazol-2-yl]-[4-(1-methyl-piperidin-4-ylmethyl)-pyridin-2-yl]-amine;
[5-((4-Fluoro-phenyl)-thiazol-2-yl]-[4-(1-methanesulfonyl-piperidin-4-ylmethyl)-pyridin-2-yl]-amine;
(6-Azepan-4-ylmethyl-pyridin-2-yl)-[5-(4-fluoro-phenyl)-thiazol-2-yl]-amine;
1-((4-{6-[5-(4-Fluoro-phenyl)-thiazol-2-ylamino]-pyridin-2-ylmethyl}-azepan-1-yl)-ethanone;
[5-((4-Fluoro-phenyl)-thiazol-2-yl]-[6-(1-methyl-azepan-4-ylmethyl)-pyridin-2-yl]-amine;
[5-((4-Fluoro-phenyl)-thiazol-2-yl]-(4-[1,4]oxazepan-6-ylmethyl-pyridin-2-yl)-amine;
[5-((4-Fluoro-phenyl)-thiazol-2-yl]-[4-(4-methyl-[1,4]oxazepan-6-ylmethyl)-pyridin-2-yl]-amine;
2-((6-{2-[5-(4-Fluoro-phenyl)-thiazol-2-ylamino]-pyridin-4-ylmethyl}-[1,4]oxazepan-4-yl)-ethanol;
1-((6-{2-[5-(4-Fluoro-phenyl)-thiazol-2-ylamino]-pyridin-4-ylmethyl}-[1,4]oxazepan-4-yl)-ethanone;
[5-((4-Fluoro-phenyl)-thiazol-2-yl]-(6-[1,4]oxazepan-6-ylmethyl-pyridin-2-yl)-amine;
(5-Phenyl-thiazol-2-yl)-(4-pyrrolidin-3-ylmethyl-pyridin-2-yl)-amine;
(5-Phenyl-thiazol-2-yl)-[4-(2-pyrrolidin-2-yl-ethyl)-pyridin-2-yl]-amine;
[5-((4-Fluoro-phenyl)-thiazol-2-yl]-[4-(4-methylamino-cyclohexylmethyl)-pyridin-2-yl]-amine;
[5-((4-Fluoro-phenyl)-thiazol-2-yl]-[4-(octahydro-isoindol-4-ylmethyl)-pyridin-2-yl]-amine;
[5-((4-Fluoro-phenyl)-thiazol-2-yl]-[4-(octahydro-cyclopenta[c]pyrrol-4-ylmethyl)-pyridin-2-yl]-amine;
(4-[1,4]Diazepan-6-ylmethyl-pyridin-2-yl)-[5-(4-fluorophenyl)-thiazol-2-yl]-amine;
[5-((4-Fluoro-phenyl)-thiazol-2-yl]-[6-(octahydro-cyclopenta[c]pyrrol-4-ylmethyl)-pyridin-2-yl]-amine;
[5-((4-Fluoro-phenyl)-thiazol-2-yl]-[6-(octahydro-isoindol-4-ylmethyl)-pyridin-2-yl]-amine;
[6-((8-Aza-bicyclo[3.2.1]oct-3-ylmethyl)-pyridin-2-yl]-(5-phenyl-thiazol-2-yl)-amine;
2-Amino-4-[2-(5-phenyl-thiazol-2-ylamino)-pyridin-4-ylmethyl]-cyclopentanecarboxylic acid methyl ester;
[5-((4-Fluoro-phenyl)-thiazol-2-yl]-[6-(1-oxa-8-aza-spiro[4.5]dec-3-ylmethyl)-pyridin-2-yl]-amine;
2-((4-{5-Fluoro-2-[5-(4-fluoro-phenyl)-thiazol-2-ylamino]-pyrimidin-4-ylmethyl}-piperidin-1-yl)-ethanol;
(4-Azepan-4-ylmethyl-pyridin-2-yl)-(5-phenyl-thiazol-2-yl)-amine;
(5-Phenyl-thiazol-2-yl)-(4-pyrrolidin-2-yl-pyridin-2-yl)-amine;
(1,2,3,4,5,6-Hexahydro-[3,4']bipyridinyl-2'-yl)-(5-phenyl-thiazol-2-yl)-amine;
2-{4-[4-Methyl-6-(5-phenyl-thiazol-2-ylamino)-pyrimidin-2-yl]-piperidin-1-yl}-ethanol;
2-{4-[6-(5-Phenyl-thiazol-2-ylamino)-pyrazin-2-yl]-piperidin-1-yl}-ethanol;
2-Amino-2-methyl-1-{4-[2-methyl-6-(5-phenyl-thiazol-2-ylamino)-pyrimidin-4-yl]-piperidin-1-yl}-propan-1-one;
2-{4-[2-Methyl-6-(5-phenyl-thiazol-2-ylamino)-pyrimidin-4-yl]-piperidin-1-yl}-ethanol;
2-[4-(5-Phenyl-thiazol-2-ylamino)-3',4',5',6'-tetrahydro-2'H-[2,4']bipyridinyl-1'-yl]-ethanol;
3-{2'-[5-(4-Fluoro-phenyl)-thiazol-2-ylamino]-3,4,5,6-tetrahydro-2H-[4,4']bipyridinyl-1-yl}-propane-1,2-diol;
2-{2'-[5-(4-Fluoro-phenyl)-thiazol-2-ylamino]-3,4,5,6-tetrahydro-2H-[4,4']bipyridinyl-1-yl}-ethanol;
2-{2'-[5-(2-Fluoro-phenyl)-thiazol-2-ylamino]-3,4,5,6-tetrahydro-2H-[4,4']bipyridinyl-1-yl}-ethanol;
2-{2'-[5-(3-Fluoro-phenyl)-thiazol-2-ylamino]-3,4,5,6-tetrahydro-2H-[4,4']bipyridinyl-1-yl}-ethanol;
2-{2'-[5-(3,4-Difluoro-phenyl)-thiazol-2-ylamino]-3,4,5,6-tetrahydro-2H-[4,4']bipyridinyl-1-yl}-ethanol;
2-{2'-[5-(3,5-Difluoro-phenyl)-thiazol-2-ylamino]-3,4,5,6-tetrahydro-2H-[4,4']bipyridinyl-1-yl}-ethanol;
2-{2'-[5-(2,2,2-Trifluoro-ethyl)-thiazol-2-ylamino]-3,4,5,6-tetrahydro-2H-[4,4']bipyridinyl-1-yl}-ethanol;
2-Amino-2-methyl-1-[6-(5-propyl-thiazol-2-ylamino)-3',4',5',6'-tetrahydro-2'H-[2,4']bipyridinyl-1'-yl]-propan-1-one;
2-[4'-Hydroxymethyl-6-(5-phenyl-thiazol-2-ylamino)-3',4',5',6'-tetrahydro-2'H-[2,4']bipyridinyl-1'-yl]-ethanol;
2-[4'-Dimethylaminomethyl-6-(5-phenyl-thiazol-2-ylamino)-3',4',5',6'-tetrahydro-2'H-[2,4']bipyridinyl-1'-yl]-ethanol;
2-[1'-(2-Hydroxy-ethyl)-1',2',3',4',5',6'-hexahydro-[4,4']bipyridinyl-2-ylamino]-thiazole-5-carboxylic acid (2-chloro-phenyl)-amide;
6-((5-Cyclopropylethynyl-thiazol-2-ylamino)-3',4',5',6'-tetrahydro-2'H-[2,4']bipyridinyl-1'-carboxylic acid tert-butyl ester;
2-{6-[5-(2-Chloro-pyridin-4-yl)-thiazol-2-ylamino]-3',4',5',6'-tetrahydro-2'H-[2,4']bipyridinyl-1'-yl}-ethanol;
2-Amino-1-{6-[5-(2,2-dimethyl-propyl)-thiazol-2-ylamino]-3',4',5',6'-tetrahydro-2'H-[2,4']bipyridinyl-1'-yl}-2-methyl-propan-1-one; and
2-{2'-[5-((E)-Styryl)-thiazol-2-ylamino]-3,4,5,6-tetrahydro-2H-[4,4']bipyridinyl-1-yl}-ethanol;
   where such compounds have the structures as disclosed in Table 5 below. In case of a discrepancy between the chemical name given above and the corresponding structure shown in Table 5, the structure should be regarded as correct, and the name amended accordingly.

In another aspect, the present invention relates to a method for synthesizing a compound according to formula (I) by reacting an aminothiazole compound (II) with a compound (III) 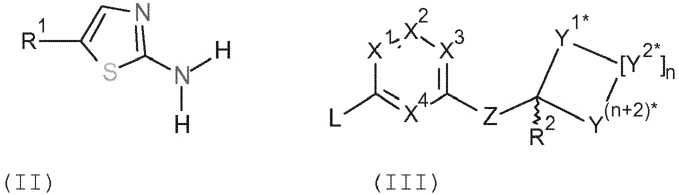 wherein
L is a leaving group, particularly a leaving group selected from -Cl, -Br and -I;
Y^{1*}, each of Y^{2*}, and Y^{(n+2)*} are each independently selected from C(R⁷)₂, N(R⁹) and O, wherein n is selected from 1, 2, 3, 4, and 5, provided that Y^{1*}-[Y^{2*}]ₙ-Y^{(n+2)*} consists of: (n+2) C(R⁷)₂ residues;
(n+1) C(R⁷)₂ residues and one N(R⁹) residue;
(n+1) C(R⁷)₂ residues and one O residue;
n C(R⁷)₂ residues, one N(R⁹) residue and one O residue;
n C(R⁷)₂ residues and two N(R⁹) residues; or
n C(R⁷)₂ residues and two O residues, provided that the two
O residues are separated by at least one C(R⁷)₂ residue;
   wherein:
   each R⁹ is selected from R^{3*} and an N-protecting group, particularly an N-protecting group, particularly a protecting group selected from: t-butyloxycarbonyl, benzyloxycarbonyl,
   each R⁷ is independently selected from -hydrogen, -F, and unsubstituted or substituted -C₁₋₄-alkyl; wherein
   each R^{3*} is independently selected from substituted or unsubstituted: -alkyl, -alkenyl, -alkynyl, -cycloalkyl,-cycloalkenyl, -(C-linked heterocycloalkyl), -(C-linked het-erocycloalkenyl), -aryl, -heteroaryl, -C(=O)-R⁸, -C(=O)-aryl, -C(=O)-heteroaryl, -S(=O)₂R⁸, -S(=O)₂N(R⁵)₂,-C(=O)OR⁴', and -C(=O)N(R⁵)₂; and/or
   two substituents R⁷ and/or R^{3*}, together with the ring atom(s) they are attached to, form a cycle, which may additionally contain a further heteroatom selected form N, O, or S;
   and wherein X¹, X², X³, X⁴, Z, R¹, R², R⁴, R^{4'}, R⁵, R⁶, and R⁸ are as defined above.

In another aspect, the present invention relates to aminothiazole compound (II) 5h (see Table 1).

In another aspect, the present invention relates to a methylene precursor molecule selected from the list of: 2e, 2f, 2g, 2h, 2i, and 2k (see Table 2).

In another aspect, the present invention relates to a compound (III) as defined above, provided that compound 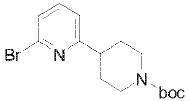 is excluded.

In particular embodiments, the present invention relates to to a compound (III) selected from the list of: 4a, 4b, 4c, 4d, 4e, 4f, 4g, 4h, 4i, 4j, 4k, 41, 4m, 4n, 4o, 4q, 4r, 10a, 10b, 10c, 10d, 10e, 10f, 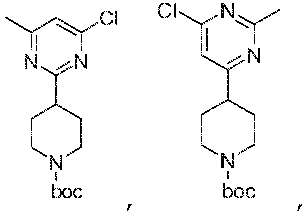 11a, 11b, 11c, 11d, 11e, 11f, 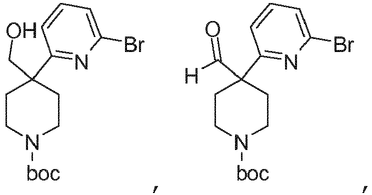 12a, 12b, 12c, 12d, 12e, and 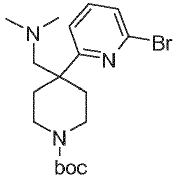 (see Tables 3 and 4).

### Formulations, Dosages and Applications

The present invention further provides a pharmaceutical composition comprising a compound as described above, or prodrug thereof, and a pharmaceutically acceptable diluent, excipient or carriercomprising a therapeutically effective amount of such compound or prodrug.

### Formulations

Compositions of the present invention may be incorporated into the dosage form and used to treat an individual in need thereof by any means that produces contact of the active ingredient with the site of action of a substance, such as a cell in the body of an individual. They can be administered by any conventional means available for use in conjunction with pharmaceuticals, either as individual therapeutic active ingredients or in a combination with other therapeutic active ingredients. They can be administered alone, but are generally administered with a pharmaceutically acceptable diluent, excipient or carrier selected on the basis of the chosen route of administration and standard pharmaceutical practice.

A pharmaceutical composition comprising less than a therapeutically effective amount of any of the compounds described above, or a prodrug thereof, may also be used, for example, when used in combination with another pharmaceutical composition, such as an anti-cancer agent, so that such combination is therapeutically effective, or may be useful for prophylactic treatment.

Pharmaceutical compositions for use in accordance with the present invention may be formulated in conventional manner using one or more pharmaceutically acceptable diluents, excipients or carriers. The pharmaceutical compositions of the invention can be formulated for a variety of routes of administration, comprising systemic and topical or localized administration. Techniques and formulations generally may be found in Remington's Pharmaceutical Sciences, Meade Publishing Co., Easton, PA. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, capsules, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intrarectally, for example, as a pessary, cream or foam. In certain embodiments, the pharmaceutical preparations may be non-pyrogenic, i.e., do not substantially elevate the body temperature of a patient.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of inhibitor, which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

For systemic administration, injection is preferred, including intramuscular, intravenous, intraperitoneal, and subcutaneous (i.m., i.v., i.p., and s.c. respectively). The phrases "systemic administration", "administered systemically", "peripheral administration", and "administered peripherally" as used herein mean the administration of a compound, drug or other material other than directly into the central nervous system, such that it enters the patient's system and, thus, is subject to metabolism and other like processes, for example, subcutaneous administration.

For injection, the pharmaceutical compositions of the invention can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

Pharmaceutical compositions of the invention may be formulated to be suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In formulating the pharmaceutical compositions of the invention in solid dosage forms for oral (p.o.) administration (capsules, tablets, pills, dragees, powders, granules and the like), a compound of the invention as active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, high molecular weight polyethylene glycols, and the like.

Gelatin capsules contain a compound of the present invention as active ingredient and powdered carriers, such as lactose, starch, cellulose derivatives, magnesium stearate, stearic acid, and the like. Similar carriers can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract. Solid compositions of a similar type are also employed as fillers in soft and hard-filled gelatin capsules; preferred materials in this connection also include lactose or milk sugar as well as high molecular weight polyethylene glycols. A preferred formulation is a solution or suspension in an oil, for example olive oil, Miglyol, or Capmul, in a soft gelatin capsule. Antioxidants may be added to prevent long-term degradation as appropriate.

A tablet may be made by compression or molding, optionally with one or more accessory ingredients. Compressed tablets may be prepared using a binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered inhibitor moistened with an inert liquid diluent.

The tablets and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulations so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally in a delayed manner. Examples of embedding compositions, which can be used, include polymeric substances and waxes. The active ingredient can also be in micro-encapsulated form, if appropriate, with one or more of the above-described excipients.

Liquid dosage forms for oral administration of the pharmaceutical compositions of the invention include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the pharmaceutical compositions for oral administration can also include adjuvants such as wetting agents, emulsifying and suspending agents, sweetening, flavoring, coloring, perfuming, and preservative agents.

Suspensions, in addition to the pharmaceutical composition of the present invention, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

For buccal administration the pharmaceutical compositions may take the form of tablets or lozenges formulated in a conventional manner.

For administration by inhalation, the pharmaceutical compositions of the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The pharmaceutical compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions of this invention suitable for parenteral administration comprise one or more inhibitors of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers, which may be employed in the pharmaceutical compositions of the invention, include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Specified pharmaceutical compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the pharmaceutical compositions. In addition, prolonged absorption of the injectable pharmaceutical form may be brought about by the inclusion of agents that delay absorption such as aluminum monostearate and/or gelatin.

In addition to the formulations described previously, the pharmaceutical compositions may also be formulated as a depot preparation. Such long acting formulations may be administered by implantation (for example subcutaneously or intramuscularly) or by intramuscular injection. Thus, for example, the pharmaceutical compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the pharmaceutical compositions of the invention are formulated into ointments, salves, gels, or creams as generally known in the art. A wash solution can be used locally to treat an injury or inflammation to accelerate healing.

In some cases, in order to prolong the therapeutic effect of an inhibitor, it is desirable to slow the absorption of the inhibitor from subcutaneous or intramuscular injection. This may be accomplished by the use of a liquid suspension of crystalline or amorphous material having poor water solubility. The rate of absorption of the inhibitor then depends upon its rate of dissolution, which, in turn, may depend upon crystal size and crystalline form. Alternatively, delayed absorption of a parenterally administered inhibitor form is accomplished by dissolving or suspending the inhibitor in an oil vehicle.

Pharmaceutical compositions of the invention may be formulated for rectal or vaginal administration as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active inhibitor. Formulations of the pharmaceutical compositions of the present invention, which are suitable for vaginal administration, also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. Such compound may be mixed under sterile conditions with a pharmaceutically acceptable carrier, and with any preservatives, buffers, or propellants, which may be required.

The ointments, pastes, creams and gels may contain, in addition to a compound of the invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing an inhibitor of the present invention in the proper medium. Absorption enhancers can also be used to increase the flux of the drug across the skin. The rate of such flux can be controlled by either providing a rate controlling membrane or dispersing the compound of the present invention in a polymer matrix or gel.

Ophthalmic formulations, eye ointments, powders, solutions and the like, are also contemplated as being within the scope of this invention.

The pharmaceutical compositions may, if desired, be presented in a pack or dispenser device, which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton.

A pharmaceutical composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions of the present invention can be used to provide slow or sustained release of one or more of the active ingredients using, for example, hydroxypropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

Injectable depot forms are made by forming microencapsuled matrices of the subject inhibitors in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

When the compounds of the present invention are administered as pharmaceuticals, to individuals, such as humans and animals, they can be given per se or as a pharmaceutical composition containing, for example, 0.1 to 99.5% (in certain embodiments, 0.5 to 90%) of active ingredient in combination with a pharmaceutically acceptable carrier.

The present invention provides new methods of treating proliferative, degenerative and other disorders or diseases, comprising cancer, by administering an amount such as a therapeutically effective amount of at least one of the compounds disclosed herein or a prodrug, tautomeric, pharmaceutically acceptable salt, N-oxide or stereoisomeric form thereof. The present invention further provides methods of treating proliferative, degenerative or other disorders or diseases, comprising cancer, by administering a therapeutically effective combination of at least one of these compounds and another anti-cancer or antiproliferative agent.

A compound of the present invention may be administered as a salt or prodrug that, upon administration to the individual, is capable of providing directly or indirectly the parent compound, such as a compound as defined herein, or that exhibits activity itself. Nonlimiting examples include a pharmaceutically acceptable salt, alternatively referred to as a "physiologically acceptable salt". In addition, modifications made to a compound can affect its biological activity, in some cases increasing the activity over the parent compound. This activity can be assessed by preparing a salt or prodrug form of the compound, and testing its activity by using methods described herein or other methods known to those of skill in the art.

As will be apparent to a person skilled in the art, through the use of a prodrug of a given subject compound, an individual such as an animal administered or treated with such prodrug will be exposed to, and hence indirectly administered with, the subject compound. Such a procedure may expose those cells associated with a disease, such as a proliferative disease or disorder including cancer, to the subject compound.

The compounds of the present invention may contain an asymmetrically substituted carbon atom, and may be isolated in optically active or racemic forms. It is well known in the art how to prepare optically active forms, such as by resolution of racemic forms or by synthesis from optically active starting materials. All chiral, diastereomeric, racemic forms and all geometric isomeric forms of a structure are intended, unless the specific stereochemistry or isomer form is specifically indicated. All processes used to prepare compounds of the present invention and intermediates made therein are considered to be part of the present invention.

### Dosages

A dosage administered that will be a therapeutically effective amount of the compound sufficient, or reasonably expected by a health-care professional such as a physician, pharmacist or nurse, to result in amelioration of symptoms of, for example, the cancer or tumor will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

The subject compounds may also be administered in prophylactic treatment. If the compound is administered prior to clinical manifestation of the unwanted condition (e.g., disease or other unwanted state of the host animal) then the treatment is prophylactic (i.e., it protects the individual against initiating, developing or further developing the unwanted condition). The subject compounds may also be administered to prevent a condition, disorder or diseases, such as cancer, or a syndrome complex, such as heart failure or any other medical condition. This includes administration of a compound the intent of which is to reduce the frequency of, or delay the onset of, symptoms of a medical condition in an individual relative to an individual, which does not receive the compound. Thus, prevention of cancer includes, for example, reducing the number of detectable cancerous growths, tumors, or malignancies in a population of patients receiving a prophylactic treatment relative to an untreated control population, delaying the appearance of detectable cancerous growths in a treated population versus an untreated control population, and/or delaying disease progression and/or improving the quality of patient life, e.g., by a statistically and/or clinically significant amount.

Toxicity and therapeutic efficacy of pharmaceutical compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit large therapeutic indices are useful for many circumstances. In certain circumstances, even therapeutic compositions that appear to exhibit debilitating or toxic side effects may be used, including circumstances where care is taken to design a delivery system that targets such therapeutic agents to the site of affected tissue in order to minimize potential damage to unaffected cells and, thereby, reduce or localize side effects.

The data obtained from cell culture assays and animal studies can be used in formulating a range of dosage for use in humans. The dosage lies preferably within a range of circulating concentrations that include the ED50 with little or no toxicity. The dosage may vary within this range depending upon the dosage form employed and the route of administration utilized. For any agents used in the method of the invention, the therapeutically effective dose can be estimated initially from cell culture assays. A dose may be formulated in animal models to achieve a circulating plasma concentration range that includes the IC₅₀ (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depends upon a number of factors known to those or ordinary skill in the art, e.g., a physician. The dose(s) of the subject compounds will vary, for example, depending upon the identity, size, and condition of the subject or sample being treated, further depending upon the route by which the composition is to be administered, if applicable, and the effect which the practitioner desires the therapeutic to have upon the therapeutic target of targets, such as cells, nucleic acid or polypeptides, through with the disease causes, symptoms or effects are mediated.

Exemplary doses include milligram or microgram amounts of the compounds of the present invention per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram.

A person skilled in the art will appreciate that doses can also be calculated on a body surface basis. A person of 70 kg has an approximate body surface area of 1.8 square meter, and doses can be expressed as milligram or microgram amounts of the compound per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

### Applications

The present invention further provides the compounds as described above for therapy. In other aspects, the invention provides the compounds of the present invention for prophylactic uses.

In certain embodiments, said therapy or prophylactic use is the treatment or prevention of a proliferative disorder or disease, such as a tumor or cancer. In certain embodiments, said treatment is the treatment of a cancer that can be treated by the inhibition of the activity of a protein kinase or mutant thereof.

Thus, the present invention additionally provides a method for treating an individual, such as a mammal, having a disease-state selected from the group of proliferative disorders or diseases, or inflammatory disorders or diseases, comprising administering to said individual a therapeutically effective amount of a compound, a prodrug, or a pharmaceutical composition of the invention as described above. In certain embodiments, said individual is a human. In certain embodiments, said proliferative disorder or disease is cancer. In certain embodiments, said treatment is the treatment of a cancer that can be treated by the inhibition of the activity of a protein kinase or mutant thereof.

The present invention also provides a method for prophylactic treatment of an individual such as an animal, including a mammal, particularly a human, the intent of which is to reduce the frequency of, delay the onset of, or the symptoms of a medical condition, such as cancer, in a subject relative to a subject which does not receive the composition.

In a further aspect, the invention provides methods of treating or preventing an individual suffering from a disease, such as a mammal, including a domestic mammal, cat, dog, horse, sheep, cow, rodent, and human, comprising the step of exposing said individual to an amount, including a therapeutically effective amount, of a subject compound. In certain embodiments, the disease is a proliferative disorder or disease, such as a cancer or tumour. In yet another embodiment, cells associated with said proliferative disorder or disease, including tumour cells included in a cancer, are exposed to the subject compound. In certain embodiments, said compound, or a prodrug thereof, is administered to said individual. In certain embodiments, said treatment is the treatment of a cancer that can be treated by the inhibition of the activity of a protein kinase or mutant thereof. In certain embodiments, said compound, or a prodrug thereof, is administered to said individual.

In a further aspect, the invention provides a method of killing or inhibiting proliferation or growth of a cell, comprising contacting the cell with a compound of the invention. In one embodiment, the cell is cultured *in vitro,* while in an alternative embodiment the cell is present in an individual. In a particular embodiment the cell is a cancer cell, for example a cell from a tumour cell line or a cell included in a tumour, including cancer cells from a tumour that can be treated by the inhibition of the activity of a protein kinase or mutant thereof.

Yet another aspect of the invention relates to the use of a compound as described above, or a prodrug thereof, for the preparation of a medicament for the treatment or prevention of a proliferative disorder or disease, including cancer, including cancers that can be treated by the inhibition of the activity of a protein kinase or mutant thereof. Additionally, the invention relates to a pharmaceutical composition comprising a compound as described above, or a prodrug thereof, and a pharmaceutically acceptable diluent, excipient or carrier, for the treatment of a proliferative disorder or disease, including cancer, including cancers that can be treated by the inhibition of the activity of a protein kinase or mutant thereof.

The subject compounds are useful to treat various disorders or diseases, including proliferative disorders or diseases, and inflammatory disorders or diseases. The term "proliferative disorder or disease" is also art recognized and includes a disorder or disease affecting an individual, such as an animal, in a manner which is marked by aberrant, or otherwise unwanted, proliferation of a subset of cells of an individual. Cancer and tumors are proliferative disorders or diseases. Cells comprising or derived from a tumor will generally be understood to be a proliferating cell, typically a hyper-proliferating cell, and in other circumstances, a tumor cell may be dysplastic, or may have proliferated. In certain embodiments, said treatment is the treatment of a cancer that can be treated by the inhibition of the activity of a protein kinase or mutant thereof.

It will be apparent to a person skilled in the art, on reading the disclosure of the instant invention, that the methods, pharmaceutical compositions and packaged pharmaceuticals comprising the subject compounds will be useful for the treatment of other proliferative disorders or diseases, or for killing or inhibiting proliferating cells including tumor cells.

Compounds of the present invention may be useful in the treatment of disease processes which feature abnormal cellular proliferation, such as hyperproliferative diseases, including cancer, benign prostate hyperplasia, familial adenomatosis polyposis, neurofibromatosis, psoriasis, fungal infections, endotoxic shock, hypertrophic scar formation, inflammatory bowel disease, transplant rejection, vascular smooth muscle cell proliferation associated with atherosclerosis, psoriasis, pulmonary fibrosis, arthritis, glomerulonephritis, restenosis following angioplasty or vascular surgery, and other post-surgical stenosis and restenosis. See, for example, U.S. Patent Nos. 6,114,365 and 6,107,305.

The compounds disclosed herein are expected to be useful in the therapy of proliferative or hyperproliferative disorders or diseases such as cancer, autoimmune diseases, viral diseases, fungal diseases, neurodegenerative disorders and cardiovascular disease.

In certain embodiments, tumors may be solid tumors, which are cancer of body tissues other than blood, bone marrow, or the lymphatic system. In other embodiments, tumors may be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, thyroid cancer, skin cancer, including squamous cell carcinoma, esophagus cancer, kidney cancer, bladder cancer, gall cancer, cervical cancer, ovarian cancer, lung cancer, bronchial, small and non-small-cell lung cancer, gastric, and head and neck cancer.

Hematological tumors may be leukemia, such as Acute Myelogenous Leukemia (AML), Acute Lymphoblastic Leukemia (ALL), Acute Lymphocytic Leukemia, Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin's Lymphoma, Non-Hodgkin's Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Hairy Cell Lymphoma, Extramedullary myeloma, Granulocytic Sarcomae.

Hematological tumors may also be tumors of myeloid lineage, including acute and chronic myelogenous leukemias, myelodysplastic syndrome, and promyelocytic leukaemia.

Tumors may also be of mesenchymal origin, such as fibrosarcoma and rhabdomyosarcoma. Furthermore, tumors may be tumors of the central and peripheral nervous system, such as astrocytoma, neuroblastoma, glioma, and schwannomas; and tumors may be other tumors, such as melanoma, seminoma, teratocarcinoma, osteosarcoma, xenoderoma pigmentosum, keratoctanthoma, thyroid follicular cancer, and Kaposi's sarcoma.

Tumors that are resistant or refractory to treatment with other anti-cancer or anti-proliferative agents may also benefit from treatment with the methods and pharmaceutical compositions of the present invention.

Compounds disclosed herein may also be useful in the chemoprevention of cancer. Chemoprevention is defined as inhibiting the development of invasive cancer by either blocking the initiating mutagenic event or by blocking the progression of pre-malignant cells, such as by blocking growth of the tumor, that have already suffered an insult or inhibiting tumor relapse.

Compounds disclosed herein may also be useful in inhibiting tumor angiogenesis and metastasis.

The compounds of this invention may also be useful in combination (administered together or sequentially) with known anti-cancer treatments such as radiation therapy or with anti-cancer, anti-proliferative, cytostatic or cytotoxic agents. Other anti-cancer and anti-proliferative agents, which may be used in combination with the compounds of the present invention, include those described herein. In combination treatment, the compounds of the present invention may be further administered with any other anti-cancer and anti-proliferative agent disclosed herein.

If formulated as a fixed dose, such combination products employ the compounds of this invention within the dosage range described herein and the other pharmaceutically active agent or treatment within its approved dosage range. For example, the cdc2 inhibitor olomucine has been found to act synergistically with known cytotoxic agents in inducing apoptosis (J. Cell Sci., 108, 2897 (1995)). Compounds described herein may also be administered sequentially with known anti-cancer or anti-proliferative agents when a combination formulation is inappropriate. The invention is not limited in the sequence of administration; compounds described herein may be administered either prior to or after administration of the known anti-cancer or antiproliferative agent. For example, the cytotoxic activity of the cyclin-dependent kinase inhibitor flavopiridol is affected by the sequence of administration with anticancer agents (Cancer Research, 57, 3375 (1997)).

### Further Aspects of the Invention

Another aspect the invention provides a pharmaceutical package, wherein said package includes a compound of any of the formulae of the present invention. In certain embodiments, the package comprises instructions, which indicate that said composition may be used for the treatment of an individual in need thereof, including a human. In certain other embodiments, the pharmaceutical package includes one or more compounds of the present invention formulated together with another pharmaceutical ingredient such as an anti-cancer or anti-proliferative agent. In this case, the compound(s) of the present invention and the other pharmaceutical ingredient may be formulated separately and in individual dosage amounts.

Other pharmaceutical ingredients that may be formulated together or separately with the compounds of the present invention include but are not limited to other anti-cancer and anti-proliferative agents such as described above. In certain still further embodiments, the pharmaceutical package comprises instructions to treat a patient in need of such treatment. In yet another aspect the invention provides a pharmaceutical package for treating an individual suffering from a proliferative disorder or disease, such as a tumor or a cancer, wherein said package includes at least one compound of the present invention. In certain still further embodiments, the pharmaceutical package comprises instructions to treat the disorder.

As used herein the term "pharmaceutical package" or "pharmaceutical pack" refer to any packaging system for storing and dispensing individual doses of medication. Preferably the pharmaceutical package contains sufficient daily dosage units appropriate to the treatment period or in amounts, which facilitate the patient's compliance with the regimen. In certain embodiments, the pharmaceutical pack comprises one or more vessels that include the active ingredient, e.g., a compound of the present invention. Such vessel can be a container such as a bottle, vial, syringe, or capsule, or may be a unit dosage form such as a pill. The active ingredient may be provided in the vessel in a pharmaceutically acceptable form or may be provided, for example, as a lyophilized powder. In further embodiments, the pharmaceutical pack may further include a solvent to prepare the active ingredient for administration. In certain embodiments, the active ingredient may be already provided in a delivery device, such as a syringe, or a suitable delivery device may be included in the pack. The pharmaceutical package may comprise pills, liquids, gels, tablets, dragees or the pharmaceutical preparation in any other suitable form. The package may contain any number of daily pharmaceutical dosage units. The package may be of any shape, and the unit dosage forms may be arranged in any pattern, such as circular, triangular, trapezoid, hexagonal or other patterns. One or more of the doses or subunits may be indicated, for example to aid the doctor, pharmacist or patient, by identifying such dose or subunits, such as by employing colorcoding, labels, printing, embossing, scorings or patterns. The pharmaceutical package may also comprise instructions for the patient, the doctor, the pharmacist or any other related person.

Some embodiments comprise the administration of more than one active ingredient, including compounds as disclosed herein. Such administration may occur concurrently or sequentially. The active ingredients may be formulated together such that one administration delivers both components. Alternatively the active ingredients may be formulated separately. The pharmaceutical package may comprise the compound of the present invention and the other pharmaceutical ingredient in a single formulation, i.e., they are formulated together, or the compound of the present invention and the other pharmaceutical ingredient in individual formulations, i.e., they are formulated separately. Each formulation may comprise the compound of the present invention and the other pharmaceutical ingredient in individual dosage amounts (in approximately equal or unequal amounts). Administration of the compound of the present invention and the other pharmaceutical ingredient results in a concentration that results in a therapeutically effective amount of the combination.

As used herein, the term "instructions" means a product label and/or documents or other information describing relevant materials or methodologies pertaining to assembly, preparation or use of a kit or packaged pharmaceutical. These materials may include any combination of the following: background information, steps or procedures to follow, list of components, proposed dosages, warnings regarding possible side effects, instructions for administering the drug, technical support, and any other related documents. Instructions can be supplied in printed form, such as a package label or a package insert. Instructions for a packaged pharmaceutical or a pharmaceutical composition can be inserted in a delivery carton or finished package, e.g., as a package insert, and the text of such has been approved by a competent regulatory authority such as the Food and Drug Administration (FDA) of the United States. Alternatively or complementarily, instruction may also be stored in electronic form, e.g., on a computer-readable storage medium such as a computer-readable memory device, a centralized database, magnetic media such as hard disks, floppy disks, and magnetic tape; optical media such as compact discs, CD-ROMs and holographic devices; magneto-optical media such as floptical disks; and hardware devices that are specially configured to store and execute program code, such as application-specific integrated circuits (ASICs), programmable logic devices (PLDs) and ROM (read only memory) and RAM (random access memory) devices. Instructions may comprise a web address of an internet website from which more detailed instructions may be downloaded, or a recorded presentation. Instructions can contain one or multiple documents or future updates.

Thus, in one aspect the invention relates to a pharmaceutical package, including a pharmaceutical composition of the present invention, and instructions, which indicate, that said pharmaceutical composition may be used for the treatment of an individual in need thereof.

In certain embodiments of such pharmaceutical package, said instructions indicate that said pharmaceutical composition may be used for the treatment of a human.

In certain embodiments of such pharmaceutical package, said instructions indicate that said pharmaceutical composition may be used for the treatment of an individual suffering from a disorder or disease.

In certain embodiments of such pharmaceutical package, said instructions indicate that said pharmaceutical composition may be used for the treatment of a human suffering from a disorder or disease.

In another aspect the invention relates to a method for treating a disorder or disease in an individual, comprising exposing cells included in said a disorder or disease to a compound of the present invention.

In certain embodiments of such method, said compound, or a prodrug thereof, is administered to said individual.

In certain embodiments of such method, said individual is a mammal is selected from: domestic mammal, cat, dog, horse, sheep, cow, rodents, and human.

In certain embodiments of such method, said mammal is a human.

In another aspect the invention relates to a method for inhibiting cell proliferation, comprising contacting a cell with a compound of the present invention.

In another aspect the invention relates to a use of a compound of the present invention, for the preparation of a medicament for the treatment of a disorder or disease.

In another aspect the invention relates to a pharmaceutical composition comprising a compound of the present invention, and a pharmaceutically acceptable carrier, diluent or excipient, for the treatment of a disorder or disease.

In certain embodiments of such a pharmaceutical composition, the pharmaceutical package, method, or use of the present invention, the disorder or disease is a proliferative disorder or disease.

In certain such embodiments, said proliferative disorder or disease is a cancer.

In certain such embodiments, said disorder or disease is an inflammatory disorder or disease.

### Examples

A selection of compounds within the scope of the present invention is listed in Table 5. The compounds in Table 5 were synthesized according to examples 1 to 48 below, and the surprising inhibitory activities in biochemical assays, anti-proliferative activities in cellular assays, and BBB penetration properties *in vivo* of these compounds are shown in Tables 7 to 9, respectively.

### A. Material and Methods

All starting materials, reagents, and solvents for reactions were reagent grade and used as purchased. Chromatography solvents were HPLC grade and were used without further purification. Reactions were monitored by thin layer chromatography (TLC) analysis using Merck Silica Gel 60 F-254 thin layer plates. Flash column chromatography was carried out on Merck silica gel 60 (0.015-0.040 mm).

The LC/MS analyses for the compounds were done at Surveyor MSQ (Thermo Fisher Scientific) with APCI ionization.
1. Type of HPLC column: ZORBAX Eclipse XDB-C18 2.1 x15 mm,1.8 µm; Part No: 921700-902
2. Solvent: 50% DMSO, 50% acetonitrile
3. Flow rate: 750 µl/min; column temperature 25°C
4. Mobile phase: A = 0.1% solution of formic acid in water, B = acetonitrile
5. Gradient:

| time, min. | A% | B% |
|---|---|---|
| 0.0 | 100 | 0 |
| 0.2 | 100 | 0 |
| 1.0 | 1 | 99 |
| 2.7 | 1 | 99 |
| 2.8 | 100 | 0 |
| 3.0 | 100 | 0 |

6. Detection: diode array (PDA), 200-800 nm; photodiode array detector. Detection was carried out in the full ultraviolet-visible range from 200 to 800 nm.
APCI (+ or/and - ions) - atmospheric pressure chemical ionization
ELSD (PL-ELS 2100)
7. Total run time: 3 min
8. Injection volume: 2.5 µL.

The ¹H NMR spectra were recorded on «MERCURY plus 400 MHz» spectrometer (Varian). Chemical shift values are given in ppm relative to tetramethylsilane (TMS), with the residual solvent proton resonance as internal standard.

Preparative HPLC purifications were performed on an Agilent 1100 Series, using column Luna C18(2) (Phenomenex, USA), 250x21.2 mm, 10µm

Microwave reactions were run on a CEM Discovery Unit operating at 200 W with simultaneous stirring.

### B. Synopsis of Synthesis Schemes

### Abbreviations

RING = saturated heterocycle
PG = protective groups selected from Boc or Cbz;
X¹, X², X³, X⁴ = C or N
Z linker selected from the unsubstituted or substituted - C₁₋₄-alkylene
LG¹, LG² are leaving groups like chlorine, bromine, iodine, triflate;
Subst = residues introduced on the last step.

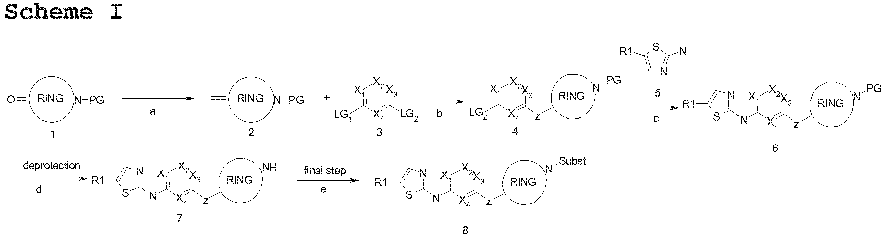

The compound 1 is commercially available or can be prepared by known methods.

The compound 1 contains one carbonyl functional group: ketone (cyclic, non-lactam) or aldehyde (exocyclic).

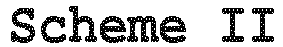

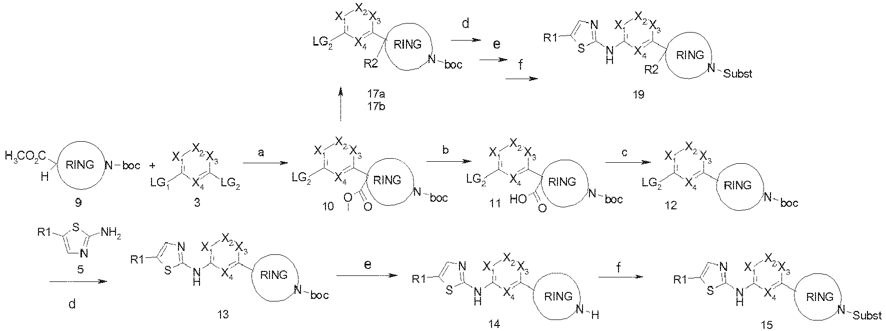

Compound **9** is commercially available or can be prepared by known methods. Compound **9** contains one exocyclic carboxylate methyl or ethyl ester
R² = CH₂OR^{4'} or CH₂N(R⁵)₂;

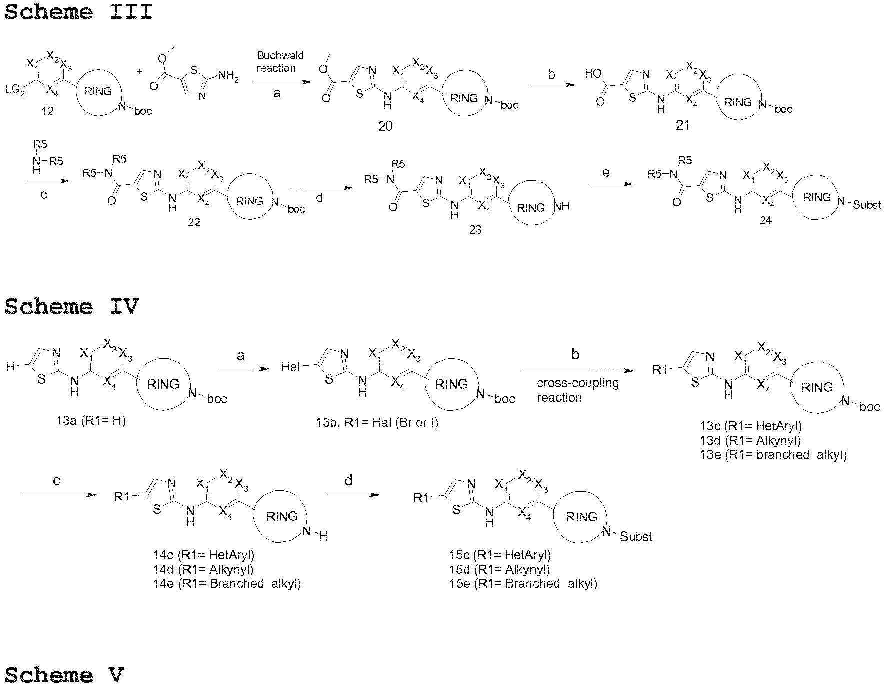

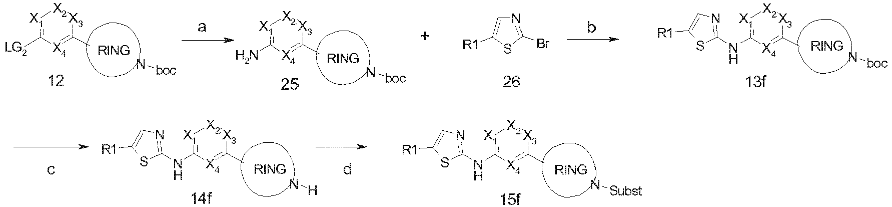

### C. Synthesis of key intermediates

### Synthesis of 2-aminothiazoles (5). Method A

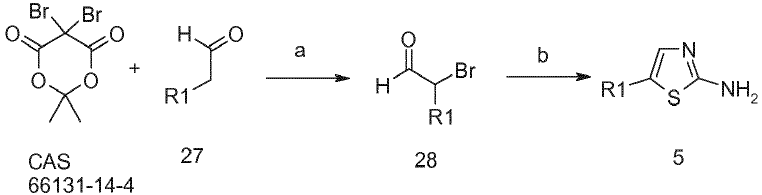

To a mixture of 5-dibromo-2,2-dimethyl-[1,3] dioxane-4,6-dione (0.012 mol) and corresponding aldehyde **27** (0.024 mol) in anhydrous ether (50 ml) bromine (0.2 ml) was added. The reaction mixture was stirred at room temperature overnight. The resulting solution was washed consequently with the solution of sodium bicarbonate and brine. The organic layer was concentrated *in vacuo* to give the crude bromoaldehyde **28,** which was used on next step without purification.

A solution of **28** and thiourea (0.012 mol) in anhydrous ethanol (20 ml) was refluxed for 6 h (TLC control). After cooling to room temperature water (60 ml) and aqueous ammonia (2 ml) were added, followed by extraction with dichloromethane (2X50 ml). The combined organic phases were dried over anhydrous sodium sulfate; the solvent was removed *in vacuo.* The title thiazole **5** was purified by column chromatography on silica gel.

### Synthesis of 2-aminothiazoles (5). Method B

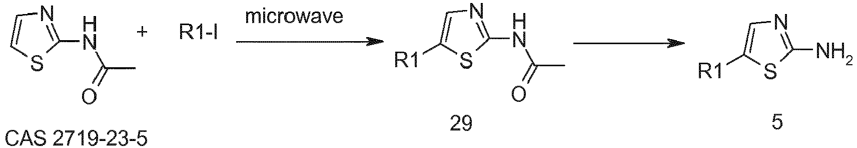

To a solution of N-thiazol-2-yl-acetamide (5 mmol), tripotassium phosphate (6 mmol), palladium(II)acetate (0.056 g, 0.25 mmol) and the corresponding iodide R1I (5 mmol) in anhydrous DMF (10 ml) tricyclohexyl phosphine (0.5 mmol) was added under argon. The reaction mixture was irradiated in microwave for 1 h at 140°C. After the reaction was cooled to room temperature, the reaction mixture was diluted with water (60 ml). The formed precipitate of compound **29** was filtered off and dried.

A solution of **29** (10 mmol) and aqueous HCl (8 M, 100 ml) in dioxane (25 ml) was refluxed for 6 h, then cooled down to room temperature, diluted with water (100 ml) and neutralized with aqueous sodium hydroxide. The formed precipitate of thiazole **5** was filtered off, washed with water and dried.

The yields and appropriate methods are specified in **Table 1**

**Table 1. Synthesis of 2-aminothiazoles (5)**

| **2-aminothiazole** | **Structure** | **Method** | **Yield, %** | **Compound** |
|---|---|---|---|---|
| 5-Propyl-thiazol-2ylamine | 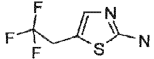 | A | 25 | 5a |
| 5-(2,2,2-trifluoroethyl)-thiazol-2-ylamine | 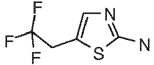 | A | 33 | 5b |
| 5-phenyl-thiazol-2-ylamine | 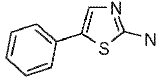 | A | 43 | 5c |
| 5- (4-fluorophenyl)-thiazol-2-ylamine | 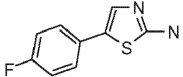 | B | 30 | 5d |
| 5- (2-fluorophenyl)-thiazol-2-ylamine | 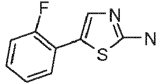 | B | 20 | 5e |
| 5- (3-fluorophenyl)-thiazol-2-ylamine | 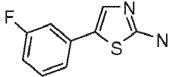 | B | 29 | 5f |
| 5-(3,4-difluorophenyl)-thiazol-2-ylamine | 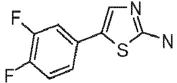 | B | 28 | 5g |
| 5-(2,4-difluorophenyl)-thiazol-2-ylamine | 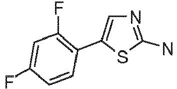 | B | 30 | 5h |
| 5-(3,5-difluorophenyl)-thiazol-2-ylamine | 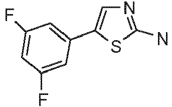 | B | 25 | 5i |
| 5-Pyridin-3-yl-thiazol-2-ylamine | 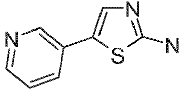 | B | 15 | 5j |

### Synthesis of substituted alkenes (2) (Scheme I, Step a)

To a suspension of methyl triphenyl phosphonium iodide (50 mmol) in anhydrous THF (100 ml) sodium hydride (75 mmol) was added gradually in the inert atmosphere at 5°C. The reaction mixture was stirred for 1 h at 10°C. After that a solution of corresponding carbonyl compound **1** (45 mmol) in anhydrous THF (20 ml) was added to the reaction mixture at 5°C. The resulting reaction mixture was stirred at room temperature for 12 h. After the reaction was completed, resulting mixture was poured into the water (150 ml) and extracted with dichloromethane (2x70 ml). Organic layer was separated, dried over sodium sulfate and evaporated in *vacuo.* The residue was purified by column chromatography on silica gel. The yields are specified in **Table 2**

**Table 2. Synthesis of alkenes 2a-i**

| **carbonyl compound 1** | **Product 2** | **Yield, %** |
|---|---|---|
| 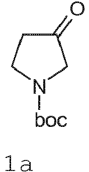 | 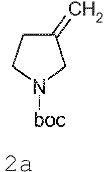 | 61 |
| 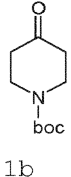 | 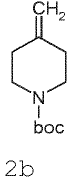 | 78 |
| 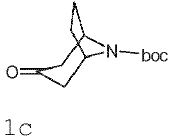 | 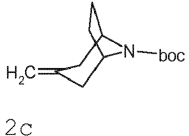 | 56 |
| 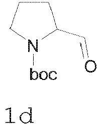 | 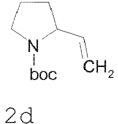 | 34 |
| 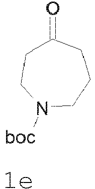 | 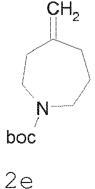 | 67 |
| 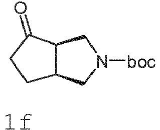 | 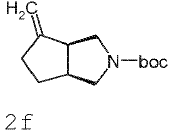 | 53 |
| 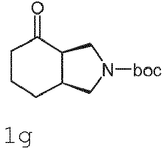 | 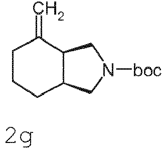 | 45 |
| 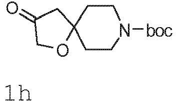 | 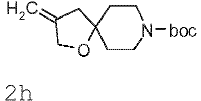 | 70 |
| 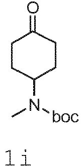 | 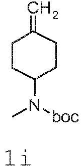 | 67 |

### Synthesis of intermediates (4) (Scheme I, Step b)

Alkenes **2a-i** were synthesized as described above. Alkene **2j** (Table 3) was synthesized in accordance with the literature method (WO2004/74291 A1, p. 67-68). Alkene **21** (Table 3) was synthesized in accordance with the literature method (US2006/106016 A1, p. 32)

To a solution of alkene **2** (0.1 mol) in 80 ml of anhydrous THF 9-borabicyclo[3.3.1]nonane (0.1 mol, 200 ml, 0.5M solution in THF) under argon atmosphere. The resulting solution was stirred at ambient temperature for 24 h. Thereafter the corresponding halide **3** (0.12 mol), potassium carbonate (110.4g, 0.8 mol), water (80 ml), and Pd(PPh₃)₄ (5.5 g, 0.0067 mol) were added. The resulting reaction mixture was heated under reflux for 3 h, then cooled down to the room temperature and poured into the ice-cold water. The resulting mixture was extracted with ethyl acetate several times. The combined organic extracts were dried over anhydrous sodium sulfate and evaporated *in vacuo.* The residue was purified by a column chromatography on silica gel to give the corresponding key intermediates **4.** The yields are specified in **Table 3.**

**Table 3. Synthesis of intermediates (4)**

| **Alkene 2** | **Halide 3** | **Product 4** | **Yield , %** |
|---|---|---|---|
| 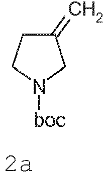 | 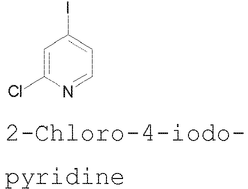 | 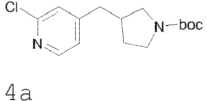 | 85 |
| 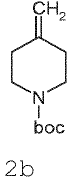 | 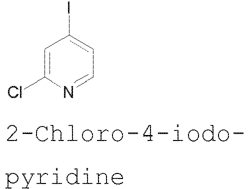 | 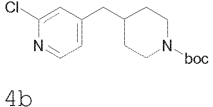 | 77 |
| 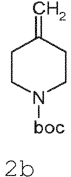 | 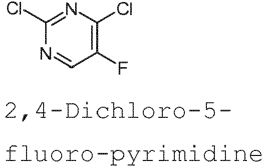 | 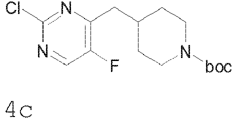 | 56 |
| 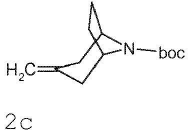 | 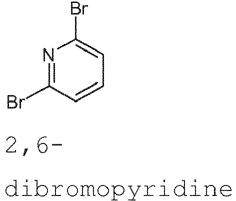 | 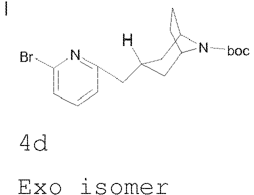 | 71 |
| 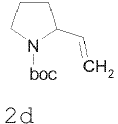 | 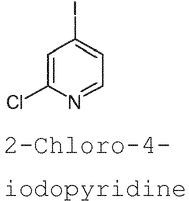 | 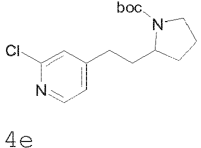 | 34 |
| 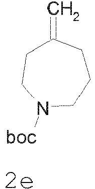 | 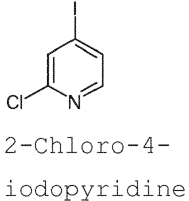 | 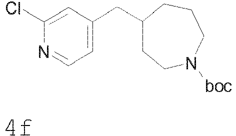 | 59 |
| 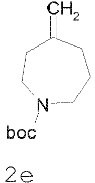 | 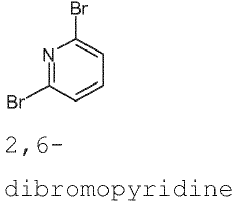 | 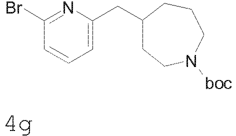 | 56 |
| 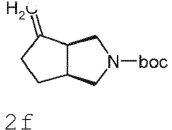 | 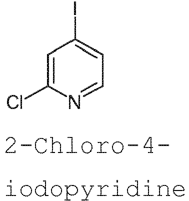 | 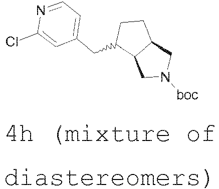 | 43 |
| 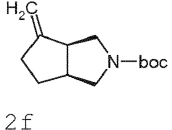 | 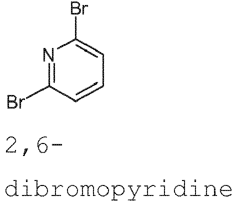 | 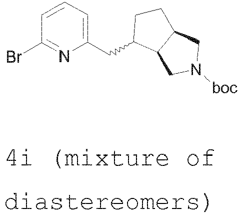 | 47 |
| 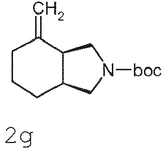 | 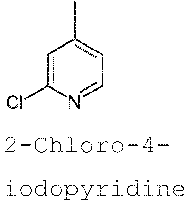 | 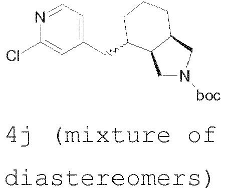 | 52 |
| 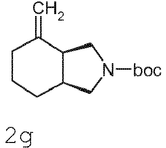 | 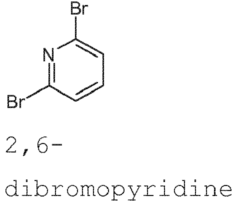 | 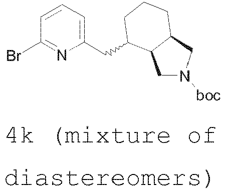 | 35 |
| 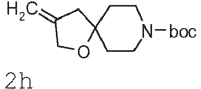 | 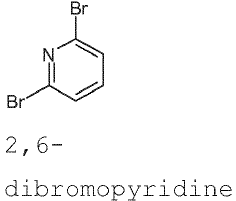 | 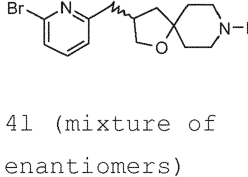 | 52 |
| 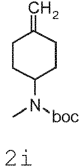 | 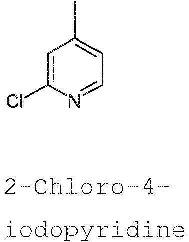 | 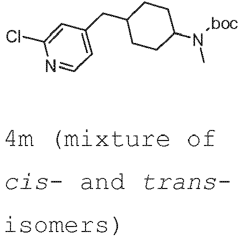 | 37 |
| 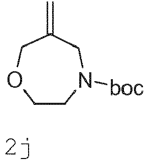 | 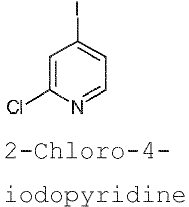 | 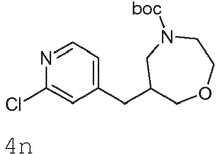 | 39 |
| 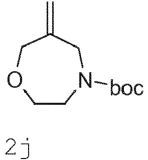 | 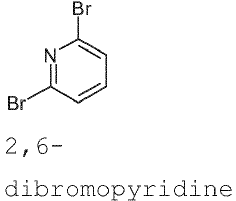 | 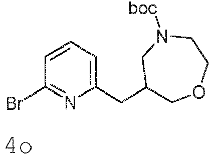 | 45 |
| 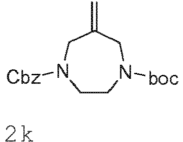 | 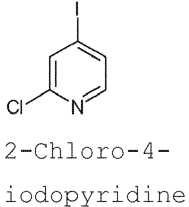 | 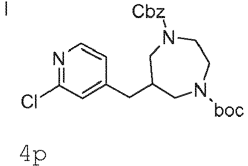 | 52 |
| 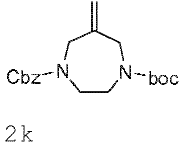 | 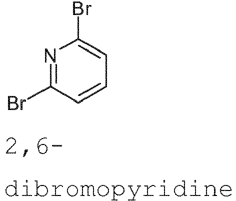 | 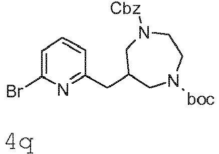 | 40 |
| 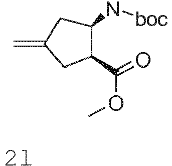 | 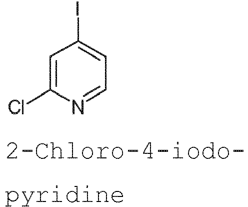 | 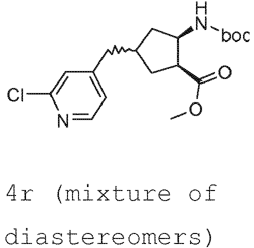 | 32 |

### Synthesis of Intermediates (10a-f, 11a-f and 12a-f) (Scheme II).

### Intermediates 10a-f, Step a

To a solution of ester **9** (1.5 mol) in anhydrous THF (800 ml) solution of sodium bis(trimethylsilyl)amide (1M in THF, 1950 ml) was added dropwise at 0°C and vigorous stirring. The mixture was stirred for 1.5 h at 0°C, and thereafter a solution of corresponding halide **3** (1.6 mol) in anhydrous THF (700 ml) was added dropwise. The reaction mixture was stirred for additional 3 h at 0°C. Then solution of ammonium chloride (67 g, 1.1 mol) in water was added cautiously. The organic layer was separated and concentrated *in vacuo.* The product **10** was used in the next step without additional purification.

### Intermediates 11a-f, Step b

To a solution of ester **10** in ethanol (300 ml) was added 10% solution of sodium hydroxide in water (2000 ml). The mixture was heated at reflux for 5 h (TLC control). The reaction mixture was cooled down, concentrated to a half of the primary volume *in vacuo* and extracted with MTBE (2x150 ml). The aqueous layer was acidified with 3M hydrochloric acid to pH=4 and extracted with dichloromethane (3x150ml). The combined organic layers were dried over anhydrous sodium sulfate. The solvent was evaporated *in vacuo* to give a product **11.** The product **11** was used in the next step without additional purification.

### Intermediates 12a-f, Step c

A solution of acid **11** in DMSO (400 ml) was stirred at 120°C for 5 h (TLC control). The reaction mixture was cooled down to room temperature, diluted with water (700 ml) and extracted with ethyl acetate (3 x 400 ml). The combined organic layers were dried over sodium sulfate. The solvent was evaporated *in vacuo* to provide the crude product **12, which** was purified by column chromatography on silica gel.

**Table 4. Synthesis of intermediates 10, 11, 12 (Scheme II)**

| **Ester 9** | **Halide 3** | **Int 10** | **Yield** | **Int 11** | **Yield** | **Int 12** | **Yield** |
|---|---|---|---|---|---|---|---|
| 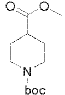 | 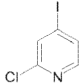 | 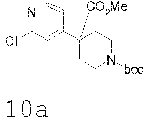 | 53 | 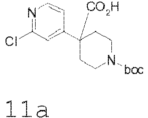 | 87 | 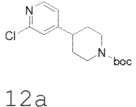 | 40 |
| 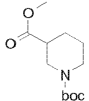 | 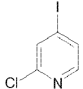 | 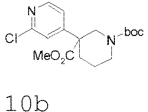 | 45 | 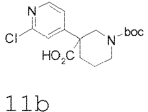 | 89 | 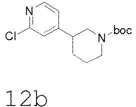 | 59 |
| 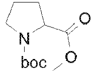 | 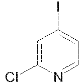 | 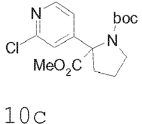 | 34 | 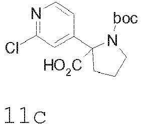 | 68 | 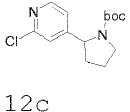 | 67 |
| 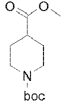 | 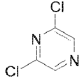 | 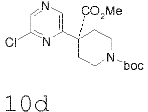 | 79 | 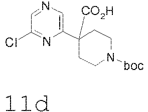 | 91 | 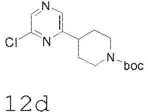 | 78 |
| 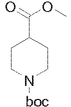 | 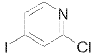 | 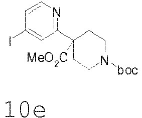 | 51 | 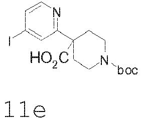 | 56 | 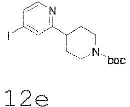 | 90 |
| 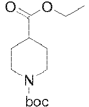 | 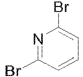 | 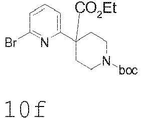 | 78 | 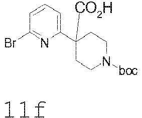 | 90 | 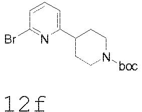 | 95 |

The intermediate **12g** was obtained as described below:

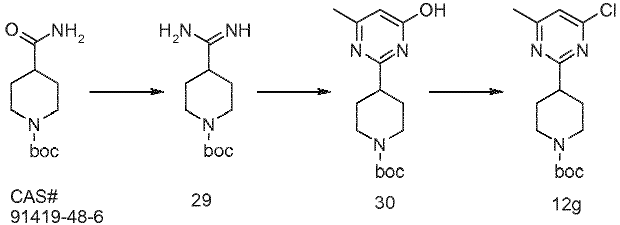

### 4-Carbamimidoyl-piperidine-1-carboxylic acid tert-butyl ester (29)

To a solution of 4-carbamoyl-piperidine-1-carboxylic acid tert-butyl ester (296 g, 1.3 mol) in dichloromethane, triethyloxonium tetrafluoroborate (270 g, 1.365 mol) was added at constant stirring; the resulted mixture was stirred for 4 h at room temperature. After that the reaction was concentrated at ∼45°C. The residue was treated with solution of NH₃/CH₃OH (pH=10), and left to stay over-night. The reaction mixture was concentrated *in vacuo.* The crude residue was used in the next step without additional purification.

### 4-(4-Hydroxy-6-methyl-pyrimidin-2-yl)-piperidine-1-carboxylic acid tert-butyl ester (30)

The compound **29** (94 g, 0.3 mol) was added to solution of sodium ethylate (0.3 mol) in anhydrous ethanol (200 ml). The mixture was stirred at ambient temperature for 10 min, and methyl 3-oxobutanoate (23.2g, 0.2 mol) was added in small portions. The resulting mixture was heated at reflux for 2 h (TLC control). The reaction mixture was concentrated under reduced pressure, the residue was dissolved in water, acidified with 1N HCl to pH 5.0 and the product was extracted with ethyl acetate (2 x 200 ml). The combined extracts were dried over sodium sulfate and concentrated *in vacuo.* The residue was purified by flash-chromatography on silica gel (eluent: n-hexane/ethyl acetate - 1/1). As a result, the compound **30** (39 g, 74%) was obtained.

### 4-(4-Chloro-6-methyl-pyrimidin-2-yl)-piperidine-1-carboxylic acid tert-butyl ester (12g)

The compound **9** (39 g, 0.147 mol) and N,N-dimethylaniline (144 g, 1.17 mol) was dissolved in anhydrous toluene (1000 m1). POCl₃ (45g, 0.294 mol) was added dropwise. The reaction mixture was heated at reflux for 3 h. After that a resulting solution was cooled and allowed to stay overnight. To this end, the mixture was poured into water; organic layer was separated, washed subsequently with 1N hydrochloric acid and water. The organic layer was concentrated under reduced pressure and the residue was purified by flash chromatography on silica gel (eluent: n-hexane/ethylacetate 4/1), to provide the title product (29.2 g, 70%).

The intermediate **12h** was obtained as describe below:

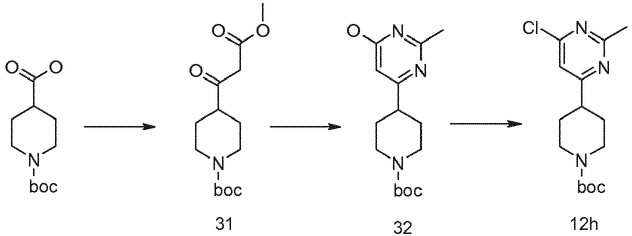

### 4-(2-ethoxycarbonyl-acetyl)-piperidine-1-carboxylic acid tert-butyl ester (31)

To a suspension of the piperidine-1,4-dicarboxylic acid mono-tert-butyl ester (150 g, 0.655 mol) in anhydrous acetonitrile (800 ml), 1,1'-carbonyldiimidazole was added in small portions (132 g, 0.851 mol) under vigorous stirring. The resulting mixture was stirred for 30 min at ambient temperature. Then powder of mixture of anhydrous MgCl₂ (62g, 0.655 mol) and methyl potassium malonate (102g, 0.655 mol) was added in portions. The resulted slurry was heated at reflux for 2 h. The reaction mixture was cooled down, diluted with mixture of ice-cold water and dichloromethane and neutralized by citric acid. The separated organic layer was washed with 5% aqueous solution of potassium carbonate, dried over sodium sulfate and concentrated under reduced pressure. Further flash-chromatography using ethyl acetate/hexane (1/1) gave 151 g (81%) of intermediate **31.**

### 4-(6-Hydroxy-2-methyl-pyrimidin-4-yl)-piperidine-1-carboxylic acid tert-butyl ester (32)

Acetamidine hydrochloride (29 g, 0.3 mol) was added to the solution of sodium ethylate (0.3 mol) in anhydrous ethanol (400 ml). The mixture was stirred at ambient temperature for 10 min, and compound **31** (0.3 mol) in ethanol was added. The resulting mixture was heated at reflux for 5 h (TLC control). The reaction mixture was concentrated under reduced pressure, the remains was dissolved in water and acidified with 1N HCl to pH 5.0 and extracted with ethyl acetate (2x200 ml). The combined extracts were dried over sodium sulfate and concentrated *in vacuo.* The residue was purified by flash-chromatography on silica gel (eluent: n-hexane/ethyl acetate - 1/1). As a result, compound **32** (50 g, 33%) was obtained.

### 4-(6-Chloro-2-methyl-pyrimidin-4-yl)-piperidine-1-carboxylic acid tert-butyl ester (12h)

Intermediate **32** (50 g, 0.170 mol) and N,N-dimethylaniline (166 g, 1.365 mol) was dissolved in anhydrous toluene (1000 ml) and POCl₃ (52 g, 0.34 mol) was added dropwise. The reaction mixture was heated at reflux for 3 h; then cooled down to ambient temperature and allowed to stay overnight. To this end, the mixture was poured into water; organic layer was separated, washed with 1N HCl and water. The crude product was concentrated under reduced pressure and purified with flash chromatography on silica gel (eluent: n-hexane/ethyl acetate 4/1) to provide **12h** (34.3 g , 65%).

### Synthesis of intermediates 17a and 17b

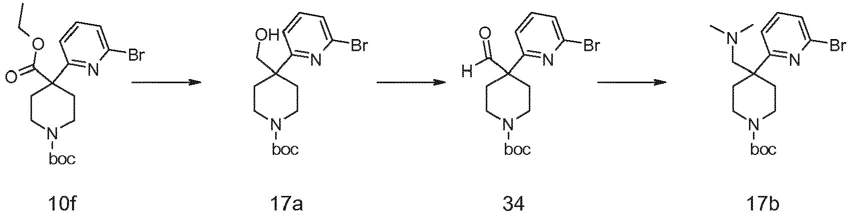

### 6-Bromo-4'-hydroxymethyl-3',4',5',6'-tetrahydro-2'H-[2,4']bipyridinyl-1'-carboxylic acid tert-butyl ester (17a)

To a suspension of lithium aluminum hydride (8g, 0.235 mol) in anhydrous THF (400 ml) compound **10f** (20.6 g, 0.05 mol) was added in small portions under stirring at room temperature. The resulting mixture was heated at reflux for 6 h (TLC control). The reaction mixture was gingerly quenched at 0°C and vigorous stirring with ice-cold water (6 ml) and then 15% aqueous potassium hydroxide (4 ml). Formed precipitate was filtered off and thoroughly washed with THF (5x10 ml). The filtrate was concentrated *in vacuo* t give a title product **17a** (17.8 g, 95%).

### 6-Bromo-4'-formyl-3',4',5',6'-tetrahydro-2'H-[2,4']bipyridinyl-1'-carboxylic acid tert-butyl ester (34)

To a solution of oxalyl chloride (1.7 ml, 19.4 mmol) in dichloromethane (25 ml) anhydrous DMSO (2.8 ml, 38.8 mmol) in dichloromethane (15 ml) was added at -60°C. The reaction mixture was stirred for 10 min at -60°C, then compound **17a** (6 g, 16.1 mmol) in anhydrous dichloromethane (30 ml) was added dropwise. The reaction mixture was stirred for 40 min at -60°C. A solution of triethylamine (11.5 ml, 83 mmol) in dichloromethane was added dropwise and resulting mixture was stirred for additional 30 min at -60°C. After warming to -10°C hexane (90 ml) was added. The formed precipitate was filtered off. The collected filtrate was washed with water (200 ml), dried over sodium sulfate and concentrated *in vacuo* to dryness to provide the title product (5.3 g, 89%).

### 6-Bromo-4'-dimethylaminomethyl-3',4',5',6'-tetrahydro-2'H-[2,4']bipyridinyl-1'-carboxylic acid tert-butyl ester (17b)

To a solution of compound **34** (1.9 g, 5 mmol) and N,N-dimethylamine hydrochloride (0.4 g, 5.5 mmol) in anhydrous acetonitrile (40 ml) sodium triacetoxyborohydride (2.1g, 10 mmol) was added in small portions under vigorous stirring. The reaction mixture was stirred overnight at ambient temperature. After the reaction was completed (TLC control) the solution was diluted with saturated potassium carbonate solution (100 ml), extracted with dichloromethane (2 x 50 ml) and dried over sodium sulfate. The solvent was removed *in vacuo* and the residue was purified by column chromatography on silica gel to provide the title product (1.4 g, 70%).

### Synthesis of intermediate 23a (Scheme III)

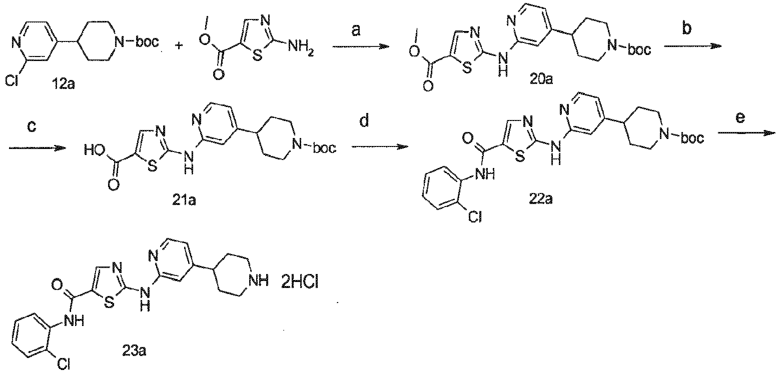

### 2'-(5-Methoxycarbonyl-thiazol-2-ylamino)-3,4,5,6-tetrahydro-2H-[4,4']bipyridinyl-1-carboxylic acid tert-butyl ester (20a)

A mixture of 2-amino-thiazole-5-carboxylic acid methyl ester (0.474g, 3 mmol), compound **12a**, obtained in accordance with Scheme II, (0.98 g, 3.3 mmol), Xantphos (4% mol), tris(dibenzylideneacetone)dipalladium(0)-chloroform complex (2% mol), cesium carbonate (5 mmol) in anhydrous 1,4-dioxane (10 ml) was heated at reflux for 16 h in argon atmosphere. The mixture was cooled down to the ambient temperature and diluted with ice-cold water. The resulting mixture was extracted with dichloromethane. The combined organic phases were dried over sodium sulfate and evaporated *in vacuo.* The residue was purified by column chromatography on silica gel to give **20a** (0.23 g, 18%)

### 2'-(5-Carboxy-thiazol-2-ylamino)-3,4,5,6-tetrahydro-2H-[4,4']bipyridinyl-1-carboxylic acid tert-butyl ester (21a)

A mixture of compound **20a** (0.23 g, 0.55 mmol), 1,4-dioxane (3 ml), potassium hydroxide (0.123 g, 2.2 mmol) and water (7 ml) was heated at reflux for 8 h (TLC control). Then reaction mixture was cooled down to the ambient temperature and diluted with water (10 ml). The resulting solution was filtered through porous glass filter. The filtrate was acidified with 5% aqueous hydrochloric acid (pH=4-5). The formed precipitate was filtered off, washed thoroughly with water and dried *in vacuo* to provide **21a** (0.22 g, 99%).

### 2'-[5-(2-Chloro-phenylcarbamoyl)-thiazol-2-ylamino]-3,4,5,6-tetrahydro-2H-[4,4']bipyridinyl-1-carboxylic acid tert-butyl ester (22a)

A mixture of acid **21a** (0.22 g), 2-chloroaniline (0.344 g, 2.7 mmol), TBTU (0.208 g, 0.65 mmol) DMAP (0.012 g, 0.1 mmol), triethyl amine (0.091 g, 0.65 mmol) in anhydrous acetonitrile (10 ml) was stirred for 48 h at ambient temperature. The reaction was diluted with saturated solution of potassium carbonate, extracted with dichloromethane. Organic extracts were dried over sodium sulfate and evaporated *in vacuo.* The residue was purified by flash chromatography on silica gel, followed by re-crystallization from chloroform to give a title compound (57 mg, 17%)

### 2-(1',2',3',4',5',6'-Hexahydro-[4,4']bipyridinyl-2-ylamino)-thiazole-5-carboxylic acid (2-chloro-phenyl)-amide dihydrochloride, (23a)

To a solution of compound **22a** (0.047 g, 0.091 mmol) in methanol (5 ml) 18% HCl in 1, 4-dioxane (1 ml) was added. The mixture was stirred for 6 h at room temperature and evaporated to dryness to provide **23a** (0.044 g, 99%).

### Synthesis of intermediate 36 (Scheme IV)

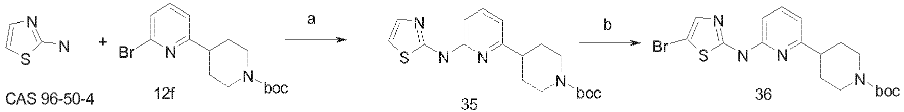

### 6-(Thiazol-2-ylamino)-3',4',5',6'-tetrahydro-2'H-[2,4']bipyridinyl-1'-carboxylic acid tert-butyl ester (35)

A mixture of 2-amino-thiazole (0.57 g, 6.4 mmol), compound ***12f*** (2.2 g, 6.5 mmol), obtained in accordance with Scheme II, Xantphos (4% mol), tris(dibenzylideneacetone)dipalladium(0)-chloroform complex (2% mol), cesium carbonate (10 mmol) in anhydrous 1,4-dioxane (20 ml) was heated at reflux for 10 h in argon atmosphere. The mixture was cooled down to the ambient temperature and diluted with ice-cold water. The resulting mixture was extracted with dichloromethane. The combined organic phases were dried over sodium sulfate and evaporated *in vacuo.* The residue was purified by column chromatography on silica gel to provide ***35*** (0.9 g, 45%)

### 6-(5-Bromo-thiazol-2-ylamino)-3',4',5',6'-tetrahydro-2'H-[2,4']bipyridinyl-1'-carboxylic acid tert-butyl ester (36)

To a solution of 6-(thiazol-2-ylamino)-3',4',5',6'-tetrahydro-2'H-[2,4']bipyridinyl-1'-carboxylic acid tert-butyl ester (3.2 g, 8.8 mmol) in dichloromethane (40 ml) 1-bromo-pyrrolidine-2,5-dione (1.74 g, 9.8 mmol) was added. The reaction mixture was stirred at room temperature for 1 h (TLC control). The reaction mixture was diluted with water (100 ml). Formed precipitated was filtered off and dried. The crude product was purified by column chromatography on silica gel to provide **36** (2.4 g, 62%).

### D. Synthesis of final compounds

### Example 1

### [5-(4-Fluoro-phenyl)-thiazol-2-yl]-(4-piperidin-4-ylmethyl-pyridin-2-yl)-amine dihydrochloride

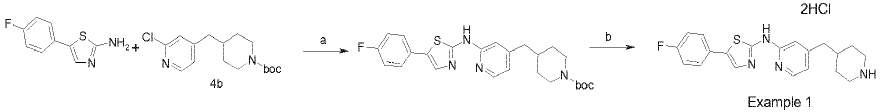

### Step a

### 4-{2-[5-(4-Fluoro-phenyl)-thiazol-2-ylamino]-pyridin-4-ylmethyl}-piperidine-1-carboxylic acid tert-butyl ester

A mixture of 5-(4-Fluoro-phenyl)-thiazol-2-ylamine (1 mmol), key intermediate **4b** (1 mmol), 9,9 dimethyl-4,5-bis(diphenylphosphino)xanthene (4% mol), tris(dibenzylideneacetone)dipalladium(0)-chloroform complex (2% mol), sodium carbonate (1.5 mmol) in the mixture of toluene/water (8/1) was refluxed for 16 h (TLC control) under inert atmosphere. After cooling to room temperature water was added, followed by extraction with ethyl acetate. The combined organic phases were dried over sodium sulfate and filtered thought silica gel, the solvent was removed in *vacuo.* The residue was purified by column chromatography on silica gel to give the title product (yield: 35%)

### Step b

### [5-(4-Fluoro-phenyl)-thiazol-2-yl]-(4-piperidin-4-ylmethyl-pyridin-2-yl)-amine dihydrochloride

To a solution of 4-{2-[5-(4-Fluoro-phenyl)-thiazol-2-ylamino]-pyridin-4-ylmethyl}-piperidine-1-carboxylic acid tert-butyl ester (0.3 mmol) in methanol (5 ml) 18% HCl in 1,4-dioxane (5 ml) was added. The mixture was stirred for 6 h at room temperature. The solvent was removed *in vacuo.* The dark oily residue was treated with diethyl ether. The formed precipitate was collected by filtration, washed with diethyl ether and dried to provide the title compound (yield: 90%).

### Example 2

### 1-(4-{2-[5-(4-Fluoro-phenyl)-thiazol-2-ylamino]-pyridin-4-ylmethyl}-piperidin-1-yl)-ethanone

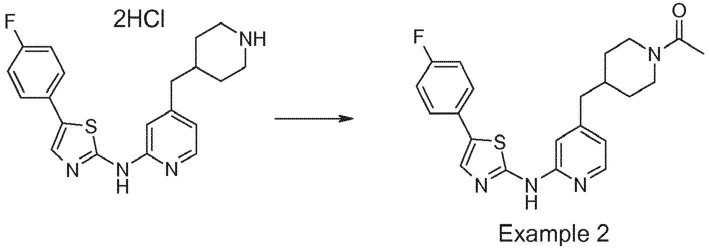

A mixture of [5-(4-Fluoro-phenyl)-thiazol-2-yl]-(4-piperidin-4-ylmethyl-pyridin-2-yl)-amine dihydrochloride (0.5 mmol), acetic acid (0.6 mmol), TBTU (0.6 mmol) and triethylamine (0.14 ml, 1 mmol) in anhydrous acetonitrile (3 ml) was stirred for 12 h at room temperature. The mixture was diluted with aqueous potassium carbonate solution (10 ml), and the product was extracted with dichloromethane (2x10 ml). The organic layers were washed with brine, water and dried over sodium sulfate. The solvent was evaporated *in vacuo.* The title product was purified by column chromatography on silica gel. Yield is 77%.

### Example 3

### 2-(4-{2-[5-(4-Fluoro-phenyl)-thiazol-2-ylamino]-pyridin-4-ylmethyl}-piperidin-1-yl)-ethanol

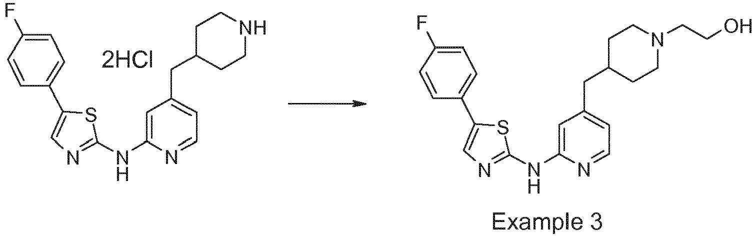

A mixture of [5-(4-Fluoro-phenyl)-thiazol-2-yl]-(4-piperidin-4-ylmethyl-pyridin-2-yl)-amine dihydrochloride (0.33 mmol), sodium bicarbonate (0.13 g, 1.5 mmol), 2-bromoethanol (0.25 g, 2 mmol) and anhydrous DMF (5 ml) was stirred at 70°C for 24 h. The reaction mixture was diluted with water (10 ml), formed precipitated was filtered off and dried on air. The crude product was purified by column chromatography on silica gel. Yield is 60%

### Example 4

### [5-(4-Fluoro-phenyl)-thiazol-2-yl]-[4-(1-methyl-piperidin-4-ylmethyl)-pyridin-2-yl]-amine

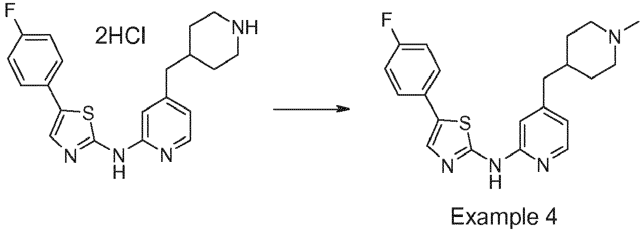

A mixture of [5-(4-Fluoro-phenyl)-thiazol-2-yl]-(4-piperidin-4-ylmethyl-pyridin-2-yl)-amine dihydrochloride (0.33 mmol), sodium bicarbonate (0.13 g, 1.5 mmol), methyl iodide (2 mmol) and anhydrous DMF (5 ml) was stirred at 70°C for 24 h. The reaction mixture was diluted with water (10 ml), formed precipitated was filtered off and dried. The crude product was purified by column chromatography on silica gel. Yield is 30%

### Example 5

### [5-(4-Fluoro-phenyl)-thiazol-2-yl]-[4-(1-methanesulfonyl-piperidin-4-ylmethyl)-pyridin-2-yl]-amine

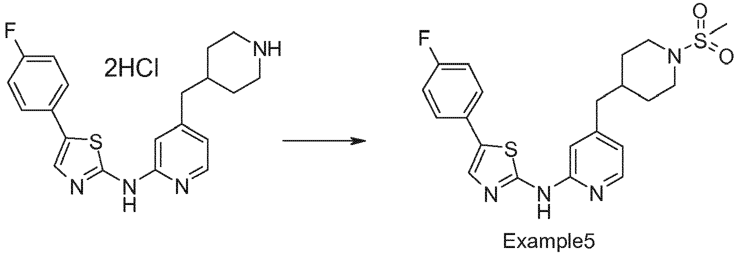

The mixture of [5-(4-fluoro-phenyl)-thiazol-2-yl]-(4-piperidin-4-ylmethyl-pyridin-2-yl)-amine dihydrochloride (0.100 g, 0.22 mmol), methanesulfonyl chloride (0.075 g, 0.66 mmol), triethyl amine (0.101 g, 1 mmol) in anhydrous acetonitrile (5 ml) was stirred at room temperature for 12 h. The reaction mixture was diluted with saturated potassium carbonate solution and extracted with dichloromethane. An organic extract was dried over sodium sulfate and evaporated. The crude product was purified by column chromatography on silica gel. Yield is 21%

### Example 6

### (6-Azepan-4-ylmethyl-pyridin-2-yl)-[5-(4-fluoro-phenyl)-thiazol-2-yl]-amine dihydrochloride

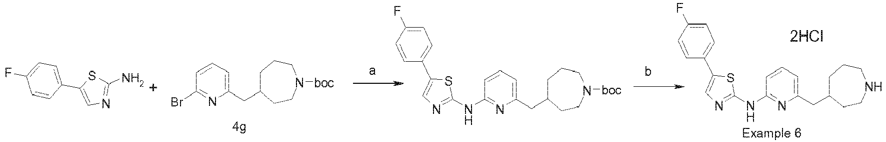

A title compound was obtained in two steps in the same manner as **Example 1** starting from 5-(4-Fluoro-phenyl)-thiazol-2-ylamine (1mmol) and key intermediate 4g (Table 3). Total yield is 30%.

### Example 7

### 1-(4-{6-[5-(4-Fluoro-phenyl)-thiazol-2-ylamino]-pyridin-2-ylmethyl}-azepan-1-yl)-ethanone

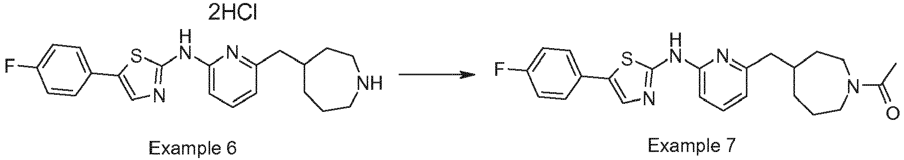

A title compound was obtained in the same manner as **Example** 2 starting from Example 6. Yield is 88%.

### Example 8

### [5-(4-Fluoro-phenyl)-thiazol-2-yl]-[6-(1-methyl-azepan-4-ylmethyl)-pyridin-2-yl]-amine

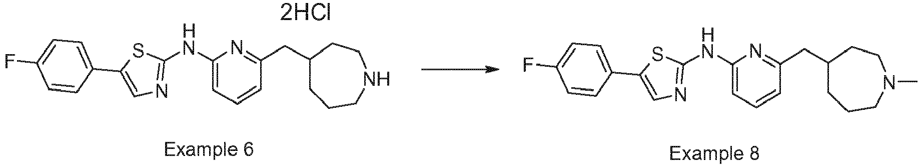

A title compound was obtained in the same manner as **Example** 4 starting from **Example** 6. Yield is 52%.

### Example 9

### [5-(4-Fluoro-phenyl)-thiazol-2-yl]-(4-[1,4]oxazepan-6-ylmethyl-pyridin-2-yl)-amine dihydrochloride

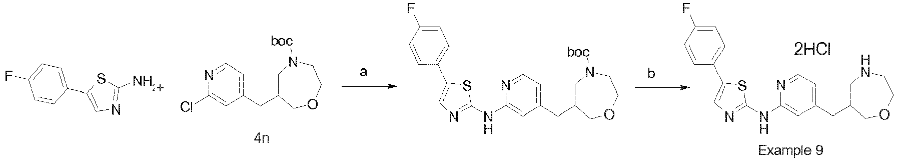

A title compound was obtained in the same manner as **Example 1** starting from 5-(4-Fluoro-phenyl)-thiazol-2-ylamine (1 mmol) and key intermediate 4n (Table 3). Total yield is 56%.

### Example 10

### [5-(4-Fluoro-phenyl)-thiazol-2-yl]-[4-(4-methyl-[1,4]oxazepan-6-ylmethyl)-pyridin-2-yl]-amine

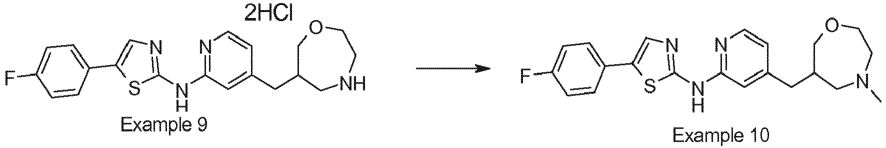

A title compound was obtained in the same manner as **Example** 4 starting from [5-(4-Fluoro-phenyl)-thiazol-2-yl]-(4-[1,4]oxazepan-6-ylmethyl-pyridin-2-yl)-amine dihydrochloride Yield is 48%.

### Example 11

### 2-(6-{2-[5-(4-Fluoro-phenyl)-thiazol-2-ylamino]-pyridin-4-ylmethyl}-[1,4]oxazepan-4-yl)-ethanol

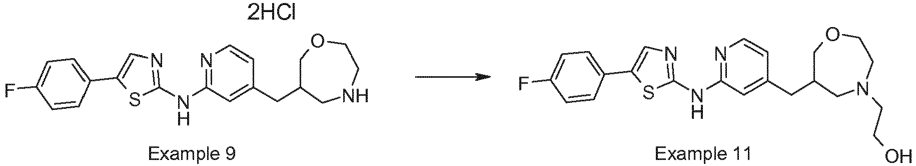

A title compound was obtained in the same manner as **Example** 3 starting from [5-(4-Fluoro-phenyl)-thiazol-2-yl]-(4-[1,4]oxazepan-6-ylmethyl-pyridin-2-yl)-amine dihydrochloride. Yield is 60%.

### Example 12

### 1-(6-{2-[5-(4-Fluoro-phenyl)-thiazol-2-ylamino]-pyridin-4-ylmethyl}-[1,4]oxazepan-4-yl)-ethanone

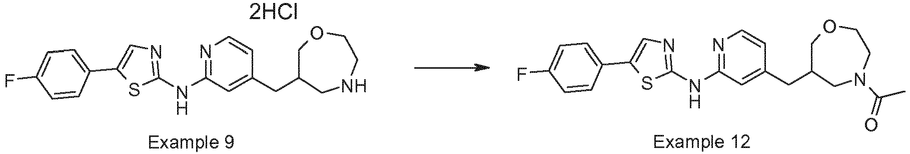

A title compound was obtained in the sane manner as **Example** 2 starting from [5-(4-Fluoro-phenyl)-thiazol-2-yl]-(4-[1,4]oxazepan-6-ylmethyl-pyridin-2-yl)-amine dihydrochloride, Yield is 90%.

### Example 13

### [5-(4-Fluoro-phenyl)-thiazol-2-yl]-(6-[1,4]oxazepan-6-ylmethyl-pyridin-2-yl)-amine dihydrochloride

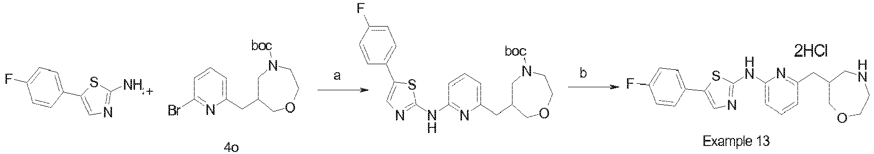

A title compound was obtained in same manner as **Example 1** starting from 5-(4-Fluoro-phenyl)-thiazol-2-ylamine (1 mmol) and key intermediate 4o (Table 3). Total yield is 67%.

### Example 14

### (5-Phenyl-thiazol-2-yl)-(4-pyrrolidin-3-ylmethyl-pyridin-2-yl)-amine dihydrochloride

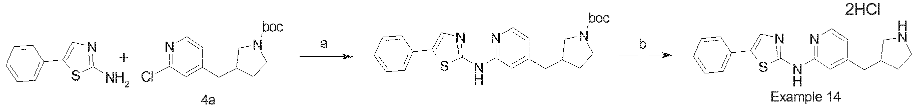

A title compound was obtained in two steps in the same manner as **Example 1** starting from 5-Phenyl-thiazol-2-ylamine (1 mmol) and key intermediate **4a** (Table 3). Total yield is 31%.

### Example 15

### (5-Phenyl-thiazol-2-yl)-[4-(2-pyrrolidin-2-yl-ethyl)-pyridin-2-yl]-amine dihydrochloride

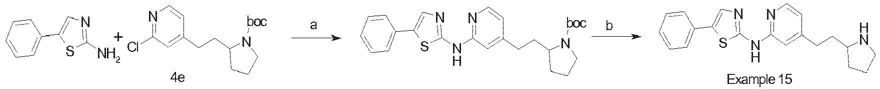

A title compound was obtained in two steps in the same manner as **Example 1** starting from 5-Phenyl-thiazol-2-ylamine (1 mmol) and key intermediate **4e** (Table 3). Summary yield is 21%.

### Example 16

### [5-(4-Fluoro-phenyl)-thiazol-2-yl]-[4-(4-methylamino-cyclohexylmethyl)-pyridin-2-yl]-amine dihydrochloride

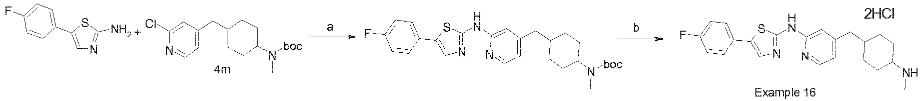

A title compound was obtained in the same manner as **Example 1** starting from 5-(4-Fluoro-phenyl)-thiazol-2-ylamine (1 mmol) and key intermediate **4m** (Table 3). Total yield is 31%.

### Example 17

### [5-(4-Fluoro-phenyl)-thiazol-2-yl][4-(octahydro-isoindol-4-ylmethyl)-pyridin-2-yl]-amine dihydrochloride (mixture of diastereomers)

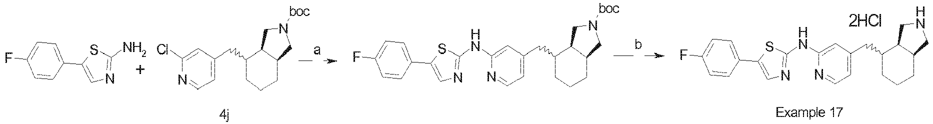

A title compound was obtained in the same manner as **Example 1** starting from 5-(4-Fluoro-phenyl)-thiazol-2-ylamine (1 mmol) and key intermediate **4j** (Table 3).Total yield is 56%.

### Example 18

### [5-(4-Fluoro-phenyl)-thiazol-2-yl]-[4-(octahydro-cyclopenta[c]pyrrol-4 -ylmethyl)-pyridin-2-yl]-amine dihydrochloride (mixture of diastereomers)

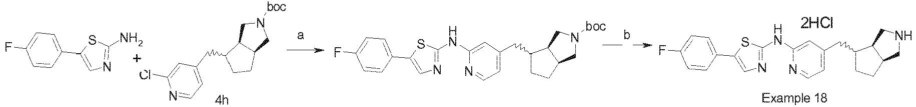

A title compound was obtained in the same manner as **Example 1** starting from 5-(4-Fluoro-phenyl)-thiazol-2-ylamine (1 mmol) and key intermediate **4h** (Table 3). Total yield is 67%.

### Example 19

### (4-[1,4]Diazepan-6-ylmethyl-pyridin-2-yl)-[5-(4-fluoro-phenyl)-thiazol-2-yl]-amine dihydrobromide

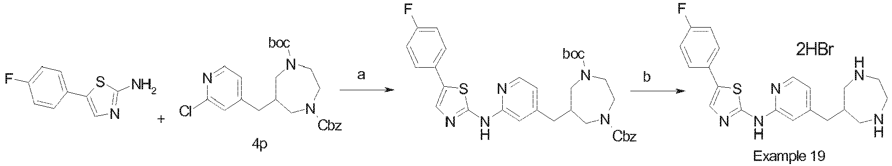

### Step a

### {2-[5-(4-Fluoro-phenyl)-thiazol-2-ylamino]-pyridin-4-ylmethyl}-[1,4]diazepane-1,4-icarboxylic acid 1-benzyl ester 4-tert-butyl ester

A mixture of 5-(4-Fluoro-phenyl)-thiazol-2-ylamine (1 mmol), key intermediate **4p** (Table 3) (1 mmol), 9,9 dimethyl-4,5-bis(diphenylphosphino)xanthene (4% mol), tris(dibenzylideneacetone)dipalladium(0)-chloroform complex (2% mol), sodium carbonate (1.5 mmol) in the mixture of toluene/water (8/1) was refluxed for 16 h (TLC control) under inert atmosphere. After cooling to room temperature water was added, followed by extraction with ethyl acetate. The combined organic phases were dried over sodium sulfate and filtered thought silica gel, the solvent was removed in *vacuo.* The residue was purified by column chromatography on silica gel to provide the title product (yield: 48%).

### Step b

### (4-[1,4]Diazepan-6-ylmethyl-pyridin-2-yl)-[5-(4-fluoro-phenyl)-thiazol-2-yl]-amine dihydrobromide

A mixture of 6-{2-[5-(4-Fluoro-phenyl)-thiazol-2-ylamino]-pyridin-4-ylmethyl}-[1,4]diazepane-1,4-icarboxylic acid 1-benzyl ester 4-tert-butyl ester (0.295 g, 0.48 mmol) and 33% HBr in acetic acid (2 ml) was stirred at 50°C for 40 min (TLC control). The mixture was diluted with diethyl ether. The formed precipitate was collected by filtration, washed with diethyl ether and dried on air to provide the title compound (98%)

### Example 20

### [5-(4-Fluoro-phenyl)-thiazol-2-yl]-[6-(octahydro-cyclopenta[c]pyrrol-4-ylmethyl)-pyridin-2-yl]-amine dihydrochloride (mixture of diastereomers)

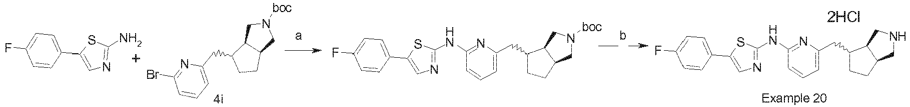

A title compound was obtained in the same manner as **Example 1** starting from 5-(4-Fluoro-phenyl)-thiazol-2-ylamine (1 mmol) and key intermediate **4i** (Table 3). Total yield is 66%.

### Example 21

### [5-(4-Fluoro-phenyl)-thiazol-2-yl]-[6-(octahydro-isoindol-4-ylmethyl)-pyridin-2-yl]-amine dihydrochloride (mixture of diastereomers)

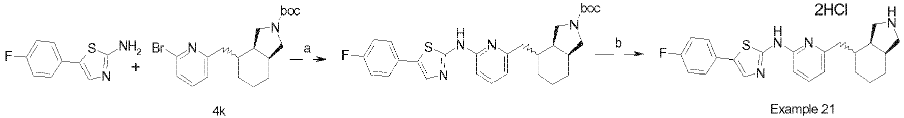

A title compound was obtained in the same manner as **Example 1** starting from 5-Phenyl-thiazol-2-ylamine (1 mmol) and key intermediate 4k (Table 3). Total yield is 56%.

### Example 22

### [6-(8-Aza-bicyclo[3.2.1]oct-3-ylmethyl)-pyridin-2-yl]-(5-phenyl-thiazol-2-yl)-amine dihydrochloride

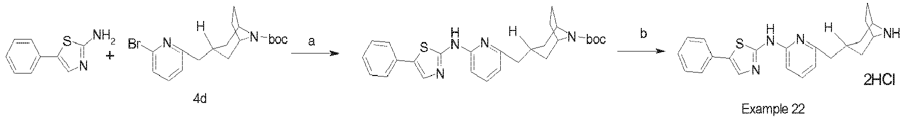

A title compound was obtained in the same manner as **Example 1** starting from 5-(4-Fluoro-phenyl)-thiazol-2-ylamine (1 mmol) and key intermediate **4d** (Table 3). Total yield is 43%.

### Example 23

### 2-Amino-4-[2-(5-phenyl-thiazol-2-ylamino)-pyridin-4-ylmethyl]-cyclopentanecarboxylic acid methyl ester dihydrochloride (mixture of diastereomers)

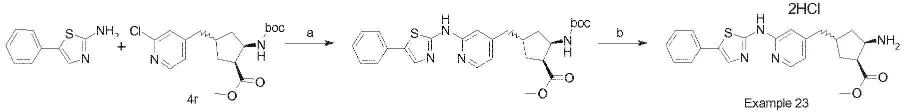

A title compound was obtained in the same manner as **Example 1** starting from 5-Phenyl-thiazol-2-ylamine (1 mmol) and key intermediate **4r** (Table 3). Total yield is 35%.

### Example 24

### [5-(4-Fluoro-phenyl)-thiazol-2-yl]-[6-(1-oxa-8-aza-spiro[4.5]dec-3-ylmethyl)-pyridin-2-yl]-amine dihydrochloride

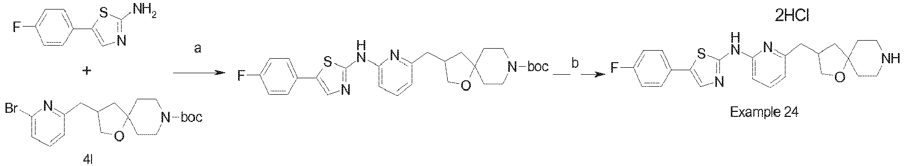

A title compound was obtained in the same manner as **Example 1** starting from 5-(4-Fluoro-phenyl)-thiazol-2-ylamine (1 mmol) and key intermediate **41** (Table 3). Total yield is 41%.

### Example 25

### 2-(4-{5-Fluoro-2-[5-(4-fluoro-phenyl)-thiazol-2-ylamino]-pyrimidin-4-ylmethyl}-piperidin-1-yl)-ethanol

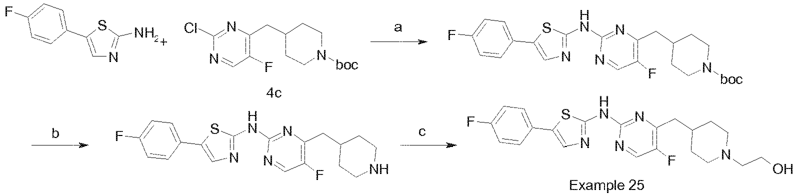

A title compound was obtained in the same manner as in **Example 1** (synthetic steps a and b) and **Example 3** (synthetic step c) starting from 5-(4-Fluoro-phenyl)-thiazol-2-ylamine (1 mmol) and key intermediate **4c** (Table 3). Total yield is 17%.

### Example 26

### (4-Azepan-4-ylmethyl-pyridin-2-yl)-(5-phenyl-thiazol-2-yl)-amine dihydrochloride

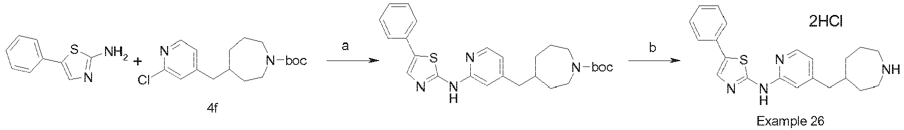

A title compound was obtained in the same manner as **Example 1** starting from 5-Phenyl-thiazol-2-ylamine (1 mmol) and key intermediate **4f** (Table 3). Total yield is 63%.

### Example 27

### (5-Phenyl-thiazol-2-yl)-(4-pyrrolidin-2-yl-pyridin-2-yl)-amine dihydrochloride

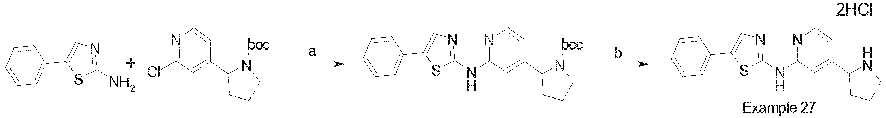

A title compound was obtained in the same manner as **Example 1** starting from 5-Phenyl-thiazol-2-ylamine (1 mmol) and key intermediate **12c** (Table 4). Total yield is 29%.

### Example 28

### (1,2,3,4,5,6-Hexahydro-[3,4']bipyridinyl-2'-yl)-(5-phenyl-thiazol-2-yl)-amine dihydrochloride

A title compound was obtained in the same manner as **Example 1** starting from 5-Phenyl-thiazol-2-ylamine (1 mmol) and key intermediate **12b.** Total yield is 45%.

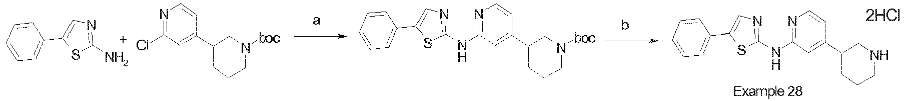

### Example 29

### 2-{4-[4-Methyl-6-(5-phenyl-thiazol-2-ylamino)-pyrimidin-2-yl]-piperidin-1-yl}-ethanol

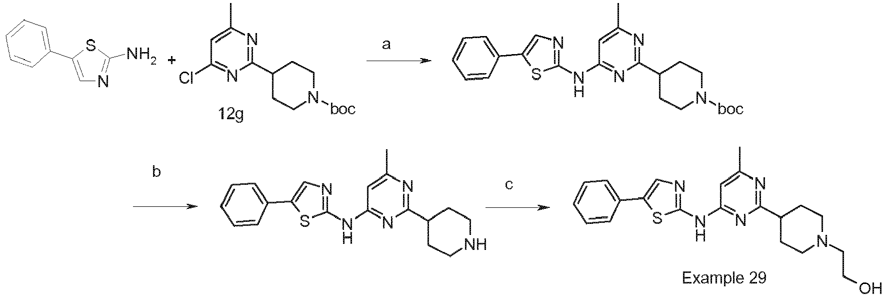

A title compound was obtained in three synthetic steps a-c the same manner as **Example 25** starting from 5-(4-Fluoro-phenyl)-thiazol-2-ylamine (1 mmol) and key intermediate **12g.** Total yield is 17%.

### Example 30

### 2-{4-[6-(5-Phenyl-thiazol-2-ylamino)-pyrazin-2-yl]-piperidin-1-yl}-ethanol

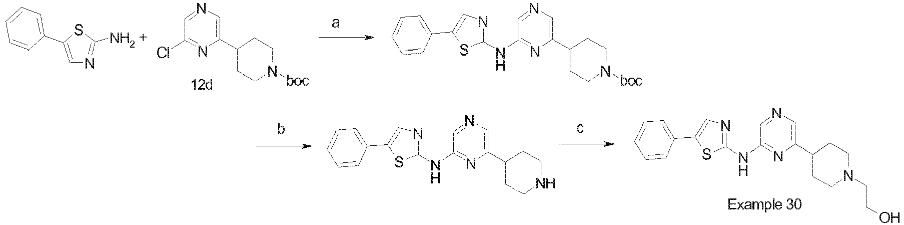

A title compound was obtained in three synthetic steps a-c the same manner as **Example 25** starting from 5-(4-Fluoro-phenyl)-thiazol-2-ylamine (1 mmol) and key intermediate **12d** (Table 4). Total yield is 25%.

### Example 31

### 2-Amino-2-methyl-1-{4-[2-methyl-6-(5-phenyl-thiazol-2-ylamino)-pyrimidin-4-yl]-piperidin-1-yl}-propan-1-one

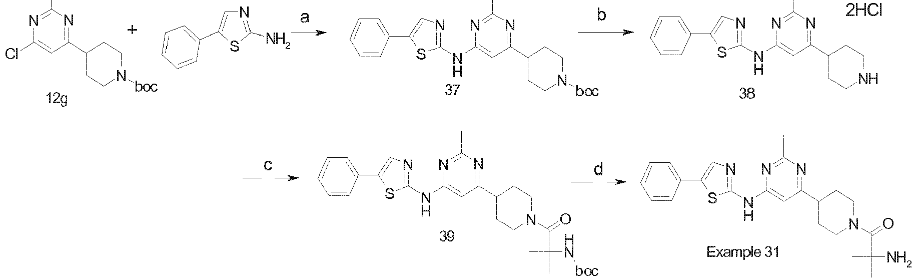

### Steps a, b

### (2-Methyl-6-piperidin-4-yl-pyrimidin-4-yl)-(5-phenyl-thiazol-2-yl)-amine dihydrochloride (38)

A title compound was obtained in the same manner as **Example 1** starting from 5-Phenyl-thiazol-2-ylamine (10 mmol) and key intermediate **12h** (Table 4). Yield is 80%.

### Step c

### (1,1-Dimethyl-2-{4-[2-methyl-6-(5-phenyl-thiazol-2-ylamino)-pyrimidin-4-yl]-piperidin-1-yl}-2-oxo-ethyl)-carbamic acid tert-butyl ester (39)

A mixture of compound **38** (0.3 mmol), 2-tert-butoxycarbonylamino-2-methyl-propionic acid (0.32 mmol), TBTU (0.105 g, 0.33 mmol) and triethylamine (0.14 ml, 1 mmol) in anhydrous acetonitrile (3 ml) was stirred for 12 h at the room temperature. The mixture was diluted with aqueous potassium carbonate solution (10 ml), and the product was extracted with dichloromethane (2 x 10 ml). The organic layers were washed with brine, water and dried over sodium sulfate. The solvent was evaporated in *vacuo.* The residue was purified by column chromatography on silica gel to provide **39** (77%)

### Step d

### 2-Amino-2-methyl-1-{4-[2-methyl-6-(5-phenyl-thiazol-2-ylamino)-pyrimidin-4-yl]-piperidin-1-yl}-propan-1-one

A mixture of compound **39** and 18% HCl in 1,4-dioxane (5 ml) was stirred for 6 h at room temperature. The solvent was removed *in vacuo* and residue was diluted with saturated solution of potassium carbonate, extracted with dichloromethane. The solvent was evaporated at reduced pressure, and the residue was treated with diethyl ether. The separated solid precipitate was collected by filtration, washed with diethyl ether and dried to deliver the title product (90%).

### Example 32

### 2-{4-[2-Methyl-6-(5-phenyl-thiazol-2-ylamino)-pyrimidin-4-yl]-piperidin-1-yl}-ethanol

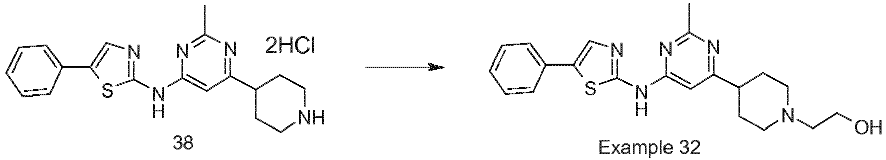

A mixture of compound **38** (0.33 mmol), sodium bicarbonate (0.13 g, 1.5 mmol), 2-bromoethanol (0.25 g, 2 mmol) and anhydrous DMF (5 ml) was stirred at 70°C for 24 h. The reaction mixture was diluted with water (10 ml), formed precipitated was filtered off and dried. The crude product was purified by column chromatography on silica gel. Yield is 70%

### Example 33

### 2-[4-(5-Phenyl-thiazol-2-ylamino)-3',4',5',6'-tetrahydro-2'H-[2,4']bipyridinyl-1'-yl]-ethanol

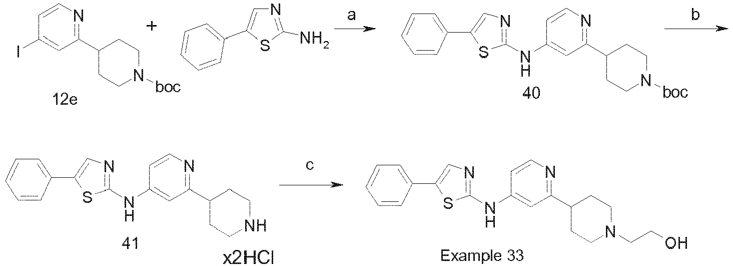

### Step a and b

**1',2',3',4',5',6'-Hexahydro-[2,4']bipyridinyl-4-yl)-(5-phenyl-thiazol-2-yl)-amine dihydrochloride (41)** was obtained in the same manner as **Example 1** starting from 5-Phenyl-thiazol-2-ylamine (2 mmol) and key intermediate **12e** (Table 4). Yield is 58%.

### Step c

### 2-[4-(5-Phenyl-thiazol-2-ylamino)-3',4',5',6'-tetrahydro-2'H-[2,4']bipyridinyl-1'-yl]-ethanol (Example 33) was prepared in the same manner as Example 3. Yield is 90%.

### Example 34

### (S)-3-{2'-[5-(4-Fluoro-phenyl)-thiazol-2-ylamino]-3,4,5,6-tetrahydro-2H-[4,4']bipyridinyl-1-yl}-propane-1,2-diol

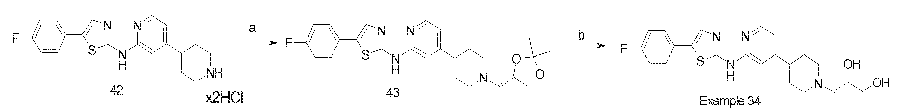

**[5-(4-Fluoro-phenyl)-thiazol-2-yl]-(1',2',3',4',5',6'-hexahydro-[4,4']bipyridinyl-2-yl)-amine dihydrochloride (42)** was obtained in the same manner as **Example 1** starting from 5-(4-Fluoro-phenyl)-thiazol-2-ylamine (1 mmol) and key intermediate **12a.** yield is 32%.

### Step a

### [1'-((S)-2,2-Dimethyl-[1,3]dioxolan-4-ylmethyl)-1',2',3',4',5',6'-hexahydro-[4,4']bipyridinyl-2-yl]-[5-(4-fluoro-phenyl)-thiazol-2-yl]-amine (43)

To a solution of [5-(4-fluoro-phenyl)-thiazol-2-yl]-(1',2',3',4',5',6'-hexahydro-[4,4'] bipyridinyl-2-yl)-amine dihydrochloride (0.2 g, 0.47 mmol) and (R)-2,2-dimethyl-[1,3]dioxolane-4-carbaldehyde (0.07 g, 0.5 mmol) in acetonitrile (10 ml) sodium triacetoxyborohydride (0.22 g, 1 mmol) was added in small portions. The reaction mixture was stirred overnight at room temperature. After the reaction was completed (TLC control) the solution was diluted with saturated potassium carbonate solution (10 ml), extracted with dichloromethane (2 x 20 ml) and dried over sodium sulfate. The solvent was removed *in vacuo* and the residue was purified by column chromatography on silica gel. Yield is 0.18 g (82%).

### Step b

### Synthesis of (S)-3-{2'-[5-(4-fluoro-phenyl)-thiazol-2-ylamino]-3,4,5,6-tetrahydro-2H-[4,4']bipyridinyl-1-yl}-propane-1,2-diol (Example 34)

To a solution of [1'-((S)-2,2-dimethyl-[1,3]dioxolan-4-ylmethyl)-1',2',3',4',5',6'-hexahydro-[4,4']bipyridinyl-2-yl]-[5-(4-fluoro-phenyl)-thiazol-2-yl]-amine (0.18 g, 0.38 mmol) in anhydrous dichloromethane (2 ml) trifluoro-acetic acid (1 ml) was added. The reaction mixture was stirred for 2 h at room temperature (TLC control), diluted with saturated potassium carbonate solution (5 ml). The formed precipitated was filtered off and dried. The title compound was purified by column chromatography on silica gel. Yield 0.13 g (80%).

### Example 35

### 2-{2'-[5-(4-Fluoro-phenyl)-thiazol-2-ylamino]-3,4,5,6-tetrahydro-2H-[4,4']bipyridinyl-1-yl}-ethanol

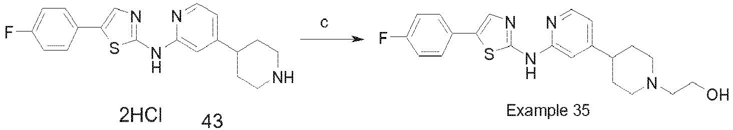

A title compound was obtained in the same manner as **Example 33** starting from the key intermediate **43** (yield is 84%).

### Example 36

### 2-{2'-[5-(2-Fluoro-phenyl)-thiazol-2-ylamino]-3,4,5,6-tetrahydro-2H-[4,4']bipyridinyl-1-yl}-ethanol

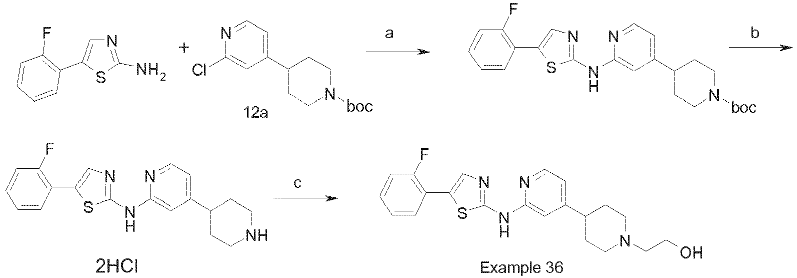

A title compound was obtained in the same manner as **Example 33** starting from 5-(2-Fluoro-phenyl)-thiazol-2-ylamine (2 mmol) and key intermediate **12a** (Table 4). Total yield is 6%.

### Example 37

### 2-{2'-[5-(3-Fluoro-phenyl)-thiazol-2-ylamino]-3,4,5,6-tetrahydro-2H-[4,4']bipyridinyl-1-yl}-ethanol

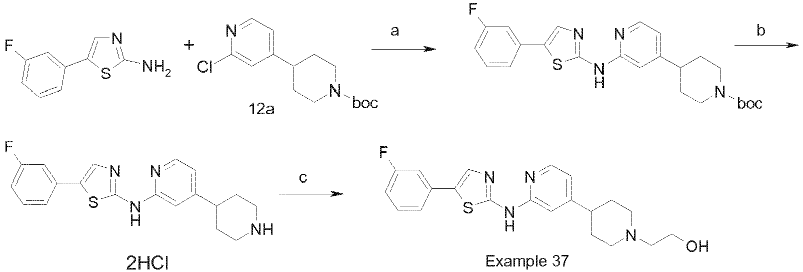

A title compound was obtained in the same manner as **Example 33** starting from 5-(3-Fluoro-phenyl)-thiazol-2-ylamine (2 mmol) and key intermediate **12a** (Table 4). Total yield is 15%.

### Example 38

### 2-{2'-[5-(3,4-Difluoro-phenyl)-thiazol-2-ylamino]-3,4,5,6-tetrahydro-2H-[4,4']bipyridinyl-1-yl}-ethanol

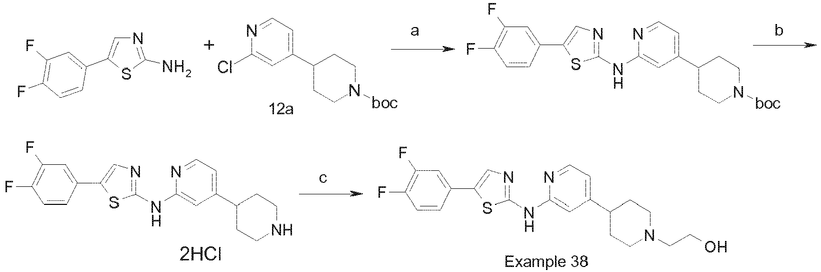

A title compound was obtained in the same manner as **Example** 33 starting from 5-(3,4-Difluoro-phenyl)-thiazol-2-ylamine (2 mmol) and key intermediate **12a** (Table 4). Total yield is 19%.

### Example 39

### 2-{2'-[5-(3,5-Difluoro-phenyl)-thiazol-2-ylamino]-3,4,5,6-tetrahydro-2H-[4,4']bipyridinyl-1-yl}-ethanol

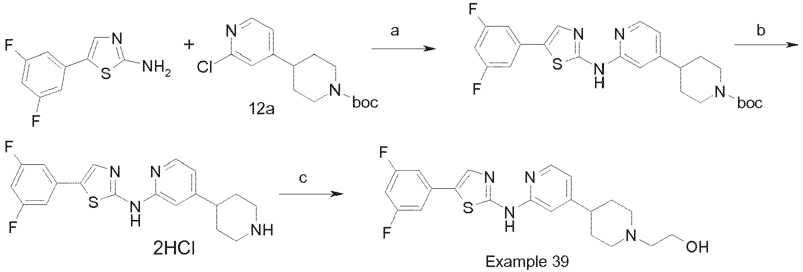

A title compound was obtained in the same manner as **Example 33** starting from 5-(3,5-Difluoro-phenyl)-thiazol-2-ylamine (2 mmol) and key intermediate **12a** (Table 4). Total yield is 11%.

### Example 40

### 2-{2'-[5-(2,2,2-Trifluoro-ethyl)-thiazol-2-ylamino]-3,4,5,6-tetrahydro-2H-[4,4']bipyridinyl-1-yl}-ethanol

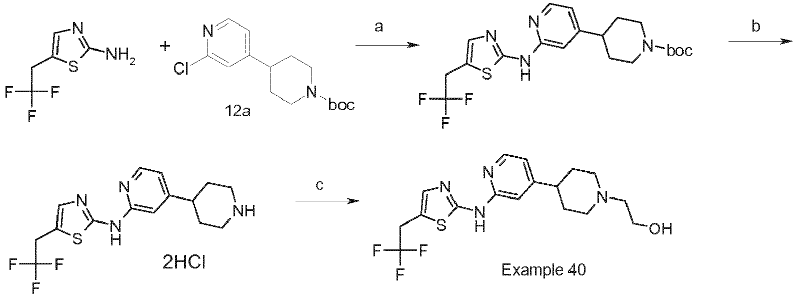

A title compound was obtained in the same manner as **Example** 33 starting from 5-(2,2,2-Trifluoro-ethyl)-thiazol-2-ylamine (2 mmol) and key intermediate **12a** (Table 4). Total yield is 22%.

### Example 41

### 2-Amino-2-methyl-1-[6-(5-propyl-thiazol-2-ylamino)-3',4',5',6'-tetrahydro-2'H-[2,4']bipyridinyl-1'-yl]-propan-1-one

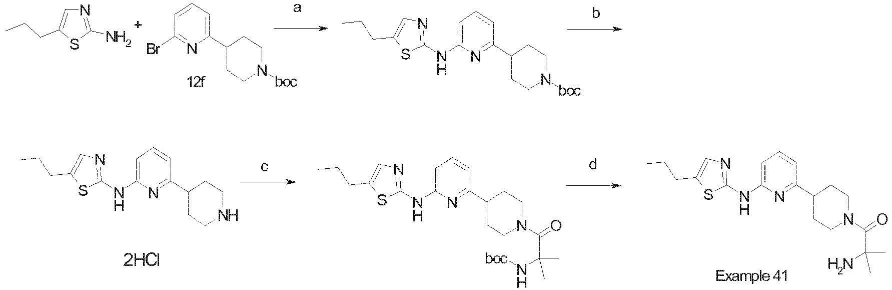

A title compound was obtained in the same manner as **Example 31** starting from 5-Propyl-thiazol-2-ylamine (5 mmol) and key intermediate **12f** (Table 4). Total yield is 22%.

### Example 42

### 2-[4'-Hydroxymethyl-6-(5-phenyl-thiazol-2-ylamino)-3',4',5',6'-tetrahydro-2'H-[2,4']bipyridinyl-1'-yl]-ethanol

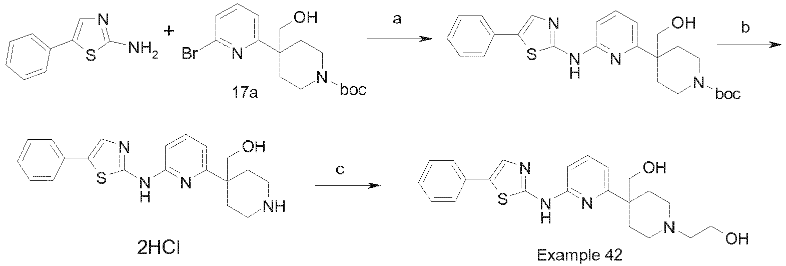

A title compound was obtained in the same manner as **Example** 33 starting from 5-Phenyl-thiazol-2-ylamine (2 mmol) and key intermediate **17a.** Total yield is 31%.

### Example 43

### 2-[4'-Dimethylaminomethyl-6-(5-phenyl-thiazol-2-ylamino)-3',4',5',6'-tetrahydro-2'H-[2,4']bipyridinyl-1'-yl]-ethanol

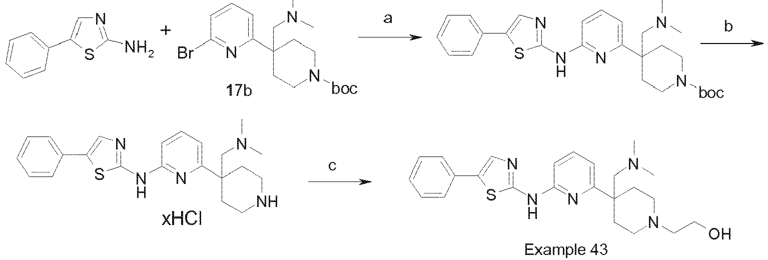

A title compound was obtained in the same manner as **Example 31** starting from 5-Phenyl-thiazol-2-ylamine (2 mmol) and key intermediate **17b**. Total yield is 44%.

### Example 44

### 2-[1'-(2-Hydroxy-ethyl)-1',2',3',4',5',6'-hexahydro-[4,4']bipyridinyl-2-ylamino]-thiazole-5-carboxylic acid (2-chloro-phenyl)-amide

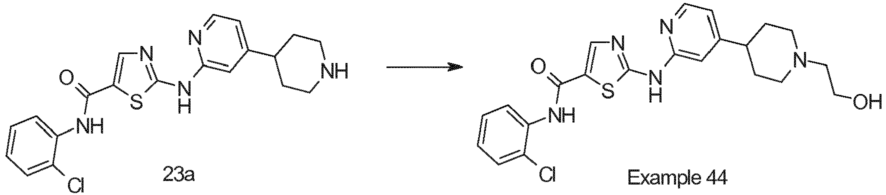

A title compound was obtained in the same manner as **Example 3** starting from key intermediate **23a.** The yield is 86%.

### Example 45

### 6-(5-Cyclopropylethynyl-thiazol-2-ylamino)-3',4',5',6'-tetrahydro-2'H-[2,4']bipyridinyl-1'-carboxylic acid tert-butyl ester

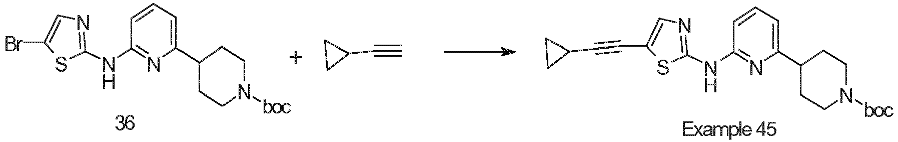

To the mixture of 6-(5-bromo-thiazol-2-ylamino)-3', 4',5',6'-tetrahydro-2'H-[2,4']bipyridinyl-1'-carboxylic acid tert-butyl ester **36** (0. 5 g, 1 mmol), 5% mol copper iodide (0.01 g), triethylamine (0.15 ml, 2 mmol), 7% mol PdCl₂(PPh₃)₂ (0.05 g) in anhydrous THF (5 ml) ethynyl-cyclopropane (0.2 g, 3 mmol) was added under argon. The reaction mixture was stirred at room temperature for 0.5 h (TLC control). The reaction mixture was diluted with water (20 ml), followed by extraction with ethyl acetate (2x15 ml). The combined organic layers were dried with sodium sulfate, filtered through silica gel and the filtrate was concentrated in *vacuo.* The residue was purified by prepara-tive HPLC to provide the title product (0.065 g, 15%).

### Example 46

### Synthesis of 2-{6-[5-(2-chloro-pyridin-4-yl)-thiazol-2-ylamino]-3',4',5',6'-tetrahydro-2'H-[2,4']bipyridinyl-1'-yl}-ethanol

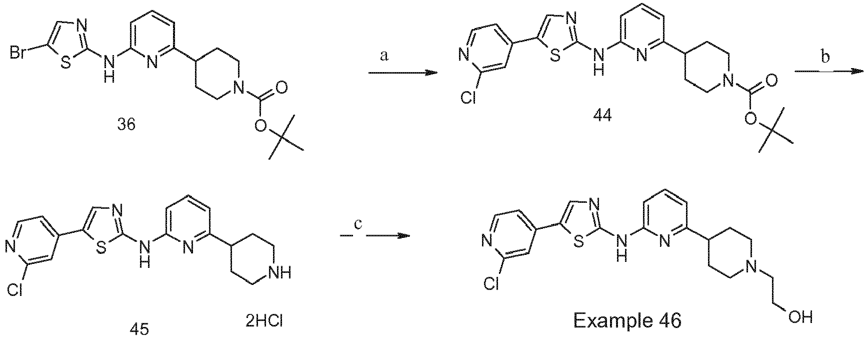

### 6-[5-(2-Chloro-pyridin-4-yl)-thiazol-2-ylamino]-3', 4',5',6'-tetrahydro-2'H-[2,4']bipyridinyl-1'-carboxylic acid tert-butyl ester (44)

To a solution of 6-(5-bromo-thiazol-2-ylamino)-3', 4',5',6'-tetrahydro-2'H-[2,4']bipyridinyl-1'-carboxylic acid tert-butyl ester **36** (0.43 g, 1 mmol), 2-chloropyridine - 4-boronic acid (0.23 g, 1.5 mmol) and sodium carbonate (0.3 g, 2 mmol) in mixture of dioxane/water (6/2ml) tetrakis(triphenylphosphine)palladium (0) (0.006 g, 5%mol) was added under argon atmosphere. The mixture was refluxed for 12h (TLC control), then cooled to room temperature and dichloromethane (30 ml) and water (20 ml) were added. The product was extracted with dichloromethane (2x20 ml). The organic layers were dried over sodium sulfate. And concentrated in *vacuo.* The residue was purified by column chromatography on silica gel to provide the title product (0.35 g, 74%).

### [5-(2-Chloro-pyridin-4-yl)-thiazol-2-yl]-(1', 2',3',4',5',6'-hexahydro-[2,4']bipyridinyl-6-yl)-amine dihydrochloride (45)

Compound **45** was prepared according to the procedure as described for intermediate **23a** (step d) from 6-[5-(2-chloro-pyridin-4-yl)-thiazol-2-ylamino]-3', 4',5',6'-tetrahydro-2'H-[2,4']bipyridinyl-1'-carboxylic acid tert-butyl ester (0.35 g, 0.7 mmol). Yield 0.3 g (97%).

### 2-{6-[5-(2-chloro-pyridin-4-yl)-thiazol-2-ylamino]-3', 4',5',6'-tetrahydro-2'H-[2,4']bipyridinyl-1'-yl}-ethanol

A mixture of [5-(2-chloro-pyridin-4-yl)-thiazol-2-yl]-(1', 2',3',4',5',6'-hexahydro-[2,4']bipyridinyl-6-yl)-amine dihydrochloride (0.15 g, 0.33 mmol), sodium bicarbonate (0.13 g, 1.5 mmol), 2-bromo-ethanol (0.25 g, 2 mmol) and anhydrous DMF (5 ml) was stirred at 70°C for 24 h. (TLC, LCMS control). The reaction mixture was diluted with water (10 ml), formed precipitated was filtered off and dried. The product was purified by column chromatography on silica gel to afford the title compound (0.08 g, 58%).

### Example 47

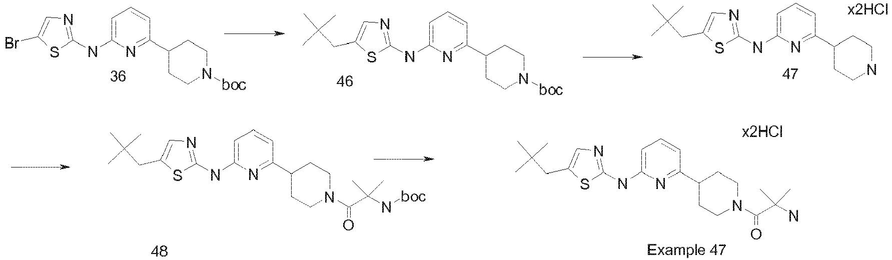

### 6-[5-(2,2-Dimethyl-propyl)-thiazol-2-ylamino]-3',4',5',6'-tetrahydro-2'H-[2,4']bipyridinyl-1'-carboxylic acid tert-butyl ester (46)

A suspension of zinc (1.7 g, 26 mmol), 1,2-dibromoethane (0.19 g, 1 mmol) in anhydrous THF (2 ml) was heated to 65°C for 1 min, cooled to 25°C, and chloro-trimethyl silane (0.1 ml, 0.8 mmol) was added. After 15 min at 25°C, a solution of 1-iodo-2, 2-dimethylpropane (4.95 g, 25 mmol) in anhydrous THF (10 ml) was slowly added at 25°C. The reaction mixture was stirred for 2 h at room temperature.

To a solution of 6-(5-bromo-thiazol-2-ylamino)-3',4',5',6'-tetrahydro-2'H-[2,4']bipyridinyl-1'-carboxylic acid tert-butyl ester (0.22 g, 0.5 mmol), 2 % mol tris(dibenzylideneacetone)dipalladium(0)-chloroform complex (0.01 g), 1-methylimidazole (0.006 ml) butyldi-adamantylphosphine (0.014 g, 0.04 mmol) was added under argon atmosphere. The reaction mixture was stirred for 10 min at room temperature. After a solution of 2,2-dimethylpropanezinc iodide (3 ml) in THF prepared above was added under argon atmosphere. The reaction mixture was stirred for 2 h at 80°C. After cooling to room temperature, water was added (10 ml), followed by extraction with dichloromethane (2x15 ml). The combined organic phases were dried over sodium sulfate; the solvent was removed in *vacuo.* The residue was purified by column chromatography on silica gel to afford the title product (0.15 g, 70%).

### [5-(2,2-Dimethyl-propyl)-thiazol-2-yl]-(1',2',3',4',5',6'-hexahydro-[2,4']bipyridinyl-6-yl)-amine dihydrochloride (47)

To a solution of 6-[5-(2,2-dimethyl-propyl)-thiazol-2-ylamino]-3',4',5',6'-tetrahydro-2'H-[2,4']bipyridinyl-1'-carboxylic acid tert-butyl ester (0.15 g, 0.3 mmol) in methanol (6 ml) 18% HCl in dioxane (5 ml) was added. The reaction mixture was stirred for 2 h at room temperature (TLC control). The solvent was evaporated *in vacuo* to provide the title product (0.12 g, 96%).

### (2-{6-[5-(2,2-Dimethyl-propyl)-thiazol-2-ylamino]-3',4',5',6'-tetrahydro-2'H-[2,4']bipyridinyl-1'-yl}-1,1-dimethyl-2-oxo-ethyl)-carbamic acid tert-butyl ester (48)

A mixture of [5-(2,2-dimethyl-propyl)-thiazol-2-yl]-(1',2',3',4',5',6'-hexahydro-[2,4']bipyridinyl-6-yl)-amine dihydrochloride (0.12 g, 0.3 mmol), 2-tert-butoxycarbonylamino-2-methyl-propionic acid (0.064 g, 0.32 mmol), TBTU (0.105 g, 0.33 mmol) and triethylamine (0.14 ml, 1 mmol) in anhydrous acetonitrile (10 ml) was stirred for 12 h at room temperature. The mixture was diluted with aqueous potassium carbonate solution (10 ml), and the product was extracted with dichloromethane (2 x 10 ml). The organic layers were washed with brine, water and dried over sodium sulfate. The solvent was evaporated *in vacuo.* The residue was purified by column chromatography on silica gel to provide the title product (0.12 g, 77%).

### 2-amino-1-{6-[5-(2,2-dimethyl-propyl)-thiazol-2-ylamino]-3',4',5',6'-tetrahydro-2'H-[2,4']bipyridinyl-1'-yl}-2-methyl-propan-1-one dihydrochloride (Example 47)

Compound was prepared according to the procedure as described for intermediate **23a** (step d) from (2-{6-[5-(2,2-dimethyl-propyl)-thiazol-2-ylamino]-3',4',5',6'-tetrahydro-2'H-[2,4']bipyridinyl-1'-yl}-1,1-dimethyl-2-oxo-ethyl)-carbamic acid tert-butyl ester (0.12 g, 0.23 mmol). Yield 0.12 g (89%).

### Example 48

### 2-{2'-[5-((E)-Styryl)-thiazol-2-ylamino]-3,4,5,6-tetrahydro-2H-[4,4']bipyridinyl-1-yl}-ethanol

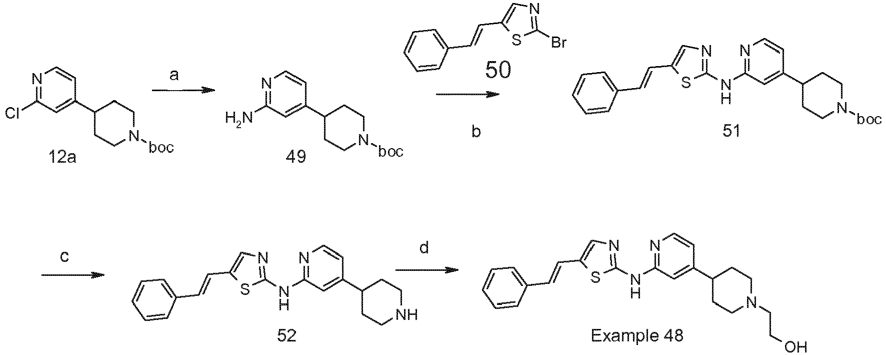

### 2'-Amino-3,4,5,6-tetrahydro-2H-[4,4']bipyridinyl-1-carbocyclic acid tert-butyl ester (49)

To the solution 2'-Chloro-3,4,5,6-tetrahydro-2H-[4,4']bipyridinyl-1-carbocyclic acid tert-butyl ester (3.0 g, 10.1 mmol), Pd₂dba₃ (0.05 g, 0.05 mmol), biphenyl-2-yl-dicyclohexyl-phosphane (0.05 g, 0.14 mmol) in anhydrous THF (5 ml) was added 12 ml LiHMDS solution in THF (1M solution) in the inert atmosphere. Reaction mixture was refluxed for 18 h. Resulting mixture was poured into water (100 ml). Aqueous suspension was extracted with ethyl acetate (2x100 ml). Organic layer was separated, dried over sodium sulphate. Sodium sulphate was filtered off and filtrate was concentrated in vacuo. Resulting residue was purified on silica gel. Yield: 2.2 g (78%).

### 2'-[5-((E)-Styryl)-thiazole-2-ylamino]-3,4,5,6-tetrahydro-2H-[4,4']bipyridinyl-1-carboxylic acid tert-butyl ester (51)

To the solution 2'-Amino-3,4,5,6-tetrahydro-2H-[4,4']bipyridinyl-1-carbocyclic acid tert-butyl ester (0.84 g, 3 mmol) in anhydrous dioxane (9 ml) was added sodium hydride (0.8 g, 20 mmol) in the inert atmosphere. Reaction mixture was stirred at room temperature for 0.5 h. After that 2-bromo-5-((E)-styryl)-thiazole **50** (0.27 g, 1.05 mmol) was added to the reaction mixture and reaction mixture was heated at 130°C for 1 h under microwave irradiation in the inert atmosphere. Resulting mixture was gradually poured into water (70 ml). Aqueous suspension was extracted with dichloromethane (2x100 ml). Organic layer was separated, dried over sodium sulphate. Sodium sulphate was filtered off and filtrate was concentrated in vacuo. Resulting residue was purified on silica gel. Crude product was recrystallized from ethyl acetate. Yield 0.22 g (16%).

2-Bromo-5-((E)-styryl)-thiazole **50** was prepared as described in WO 2007/56023 A2, 2007 Page 34-35

### (1',2',3',4',5',6'-Hexahydro-[4,4']bipyridinyl-2-yl)-[5-((E)-styryl)-thiazole-2-yl]-amine (52)

To the solution of 2'-[5-((E)-Styryl)-thizole-2-ylamino]-3,4,5,6-tetrahydro-2H-[4,4']bipyridinyl-1-carboxylic acid tert-butyl ester (0.20 g, 0.43 mmol) in anhydrous dichloromethane (2 ml) was added trifluoro-acetic acid (0.5 g, 4.4 mmol) at room temperature. Reaction mixture was stirred for 5 h. After completion of the reaction solution of sodium hydroxide (0.175 g, 5 mmol) in water (5 ml) was added to the reaction mixture. The formed solid was collected by filtration, dried and recrystallized from ethyl acetate to provide the title product (0.12 g, 75%).

### 2-{2'-[5-((E)-Styryl)-thiazole-2ylamino]-3,4,5,6-tetrahydro-2H-[4,4']bipyridinyl-1-yl}-ethanol (Example 48)

A title compound was obtained in the same manner as **Example 3** starting from compound **43** Yield is 67%.

Table 5 lists the structures and IUPAC names of the compounds synthesized in accordance with Examples 1 to 48.

**Table 5: Exemplary compounds of the present invention**

| **Example No.** | **Compound Structure** | **Compound Name** |
|---|---|---|
| **001** | 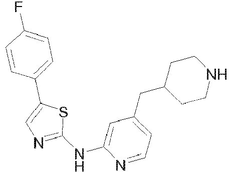 | [5-(4-Fluoro-phenyl)-thiazol-2-yl]-(4-piperidin-4-ylmethyl-pyridin-2-yl)-amine |
| **002** | 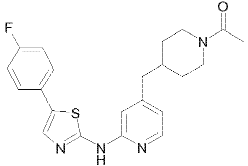 | 1-(4-{2-[5-(4-Fluoro-phenyl)-thiazol-2-ylamino]-pyridin-4-ylmethyl}-piperidin-1-yl)-ethanone |
| **003** | 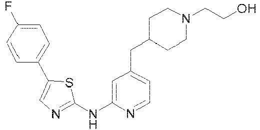 | 2-(4-{2-[5-(4-Fluoro-phenyl)-thiazol-2-ylamino]-pyridin-4-ylmethyl}-piperidin-1-yl)-ethanol |
| **004** | 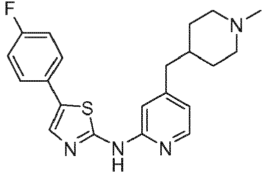 | [5-(4-Fluoro-phenyl)-thiazol-2-yl]-[4-(1-methyl-piperidin-4-ylmethyl)-pyridin-2-yl]-amine |
| **005** | 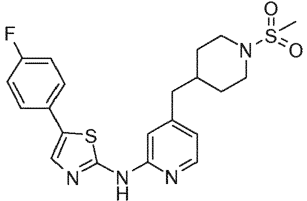 | [5-(4-Fluoro-phenyl)-thiazol-2-yl]-[4-(1-methanesulfonyl-piperidin-4-ylmethyl)-pyridin-2-yl]-amine |
| **006** | 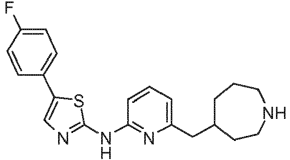 | (6-Azepan-4-ylmethyl-pyridin-2-yl)-[5-(4-fluoro-phenyl)-thiazol-2-yl]-amine |
| **007** | 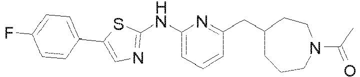 | 1-(4-{6-[5-(4-Fluoro-phenyl)-thiazol-2-ylamino]-pyridin-2-ylmethyl}-azepan-1-yl)-ethanone |
| **008** | 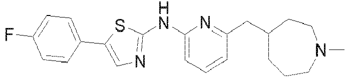 | [5-(4-Fluoro-phenyl)-thiazol-2-yl]-[6-(1-methyl-azepan-4-ylmethyl)-pyridin-2-yl]-amine |
| **009** | 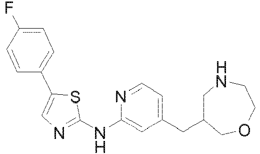 | [5-(4-Fluoro-phenyl)-thiazol-2-yl]-(4-[1,4]oxazepan-6-ylmethyl-pyridin-2-yl)-amine |
| **010** | 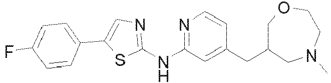 | [5-(4-Fluoro-phenyl)-thiazol-2-yl]-[4-(4-methyl-[1,4]oxazepan-6-ylmethyl)-pyridin-2-yl]-amine |
| **011** | 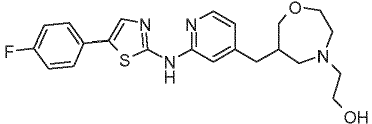 | 2-(6-{2-[5-(4-Fluoro-phenyl)-thiazol-2-ylamino]-pyridin-4-ylmethyl}-[1,4]oxazepan-4-yl)-ethanol |
| **012** | 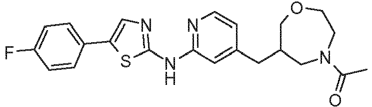 | 1-(6-{2-[5-(4-Fluoro-phenyl)-thiazol-2-ylamino]-pyridin-4-ylmethyl}-[1,4]oxazepan-4-yl)-ethanone |
| **013** | 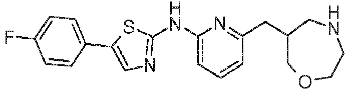 | [5-(4-Fluoro-phenyl)-thiazol-2-yl]-(6-[1,4]oxazepan-6-ylmethyl-pyridin-2-yl)-amine |
| **014** | 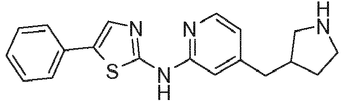 | (5-Phenyl-thiazol-2-yl)-(4-pyrrolidin-3-ylmethyl-pyridin-2-yl)-amine |
| **015** | 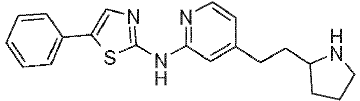 | (5-Phenyl-thiazol-2-yl)-[4-(2-pyrrolidin-2-yl-ethyl)-pyridin-2-yl]-amine |
| **016** | 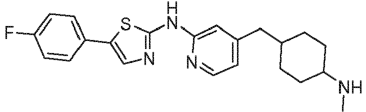 | [5-(4-Fluoro-phenyl)-thiazol-2-yl]-[4-(4-methylamino-cyclohexylmethyl)-pyridin-2-yl]-amine |
| **017** | 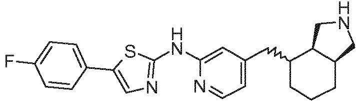 | [5-(4-Fluoro-phenyl)-thiazol-2-yl]-[4-(octahydro-isoindol-4-ylmethyl)-pyridin-2-yl]-amine |
| **018** | 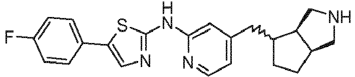 | [5-(4-Fluoro-phenyl)-thiazol-2-yl]-[4-(octahydro-cyclopenta[c]pyrr ol-4-ylmethyl)-pyridin-2-yl]-amine |
| **019** | 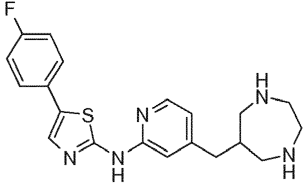 | (4-[1,4]Diazepan-6-ylmethyl-pyridin-2-yl)-[5-(4-fluoro-phenyl)-thiazol-2-yl]-amine |
| **020** | 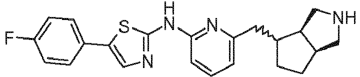 | [5-(4-Fluoro-phenyl)-thiazol-2-yl]-[6-(octahydro-cyclopenta[c]pyrr ol-4-ylmethyl)-pyridin-2-yl]-amine |
| **021** | 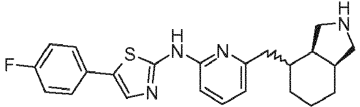 | [5-(4-Fluoro-phenyl)-thiazol-2-yl]-[6-(octahydro-isoindol-4-ylmethyl)-pyridin-2-yl]-amine |
| **022** | 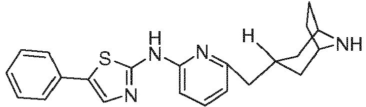 | [6-(8-Aza-bicy-clo[3.2.1]oct-3-ylmethyl)-pyridin-2-yl]-(5-phenyl-thiazol-2-yl)-amine |
| **023** | 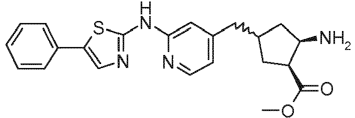 | 2-Amino-4-[2-(5-phenyl-thiazol-2-ylamino)-pyridin-4-ylmethyl]-cyclopentanecar-boxylic acid methyl ester |
| **024** | 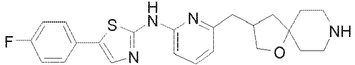 | [5-(4-Fluoro-phenyl)-thiazol-2-yl]-[6-(1-oxa-8-aza-spiro[4.5]dec-3-ylmethyl)-pyridin-2-yl]-amine |
| **025** | 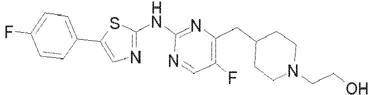 | 2-(4-{5-Fluoro-2-[5-(4-fluoro-phenyl)-thiazol-2-ylamino]-pyrimidin-4-ylmethyl}-piperidin-1-yl)-ethanol |
| **026** | 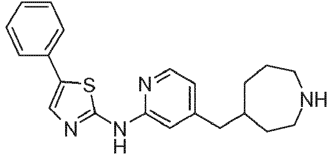 | (4-Azepan-4-ylmethyl-pyridin-2-yl)-(5-phenyl-thiazol-2-yl)-amine |
| **027** | 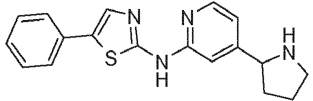 | (5-Phenyl-thiazol-2-yl)-(4-pyrrolidin-2-yl-pyridin-2-yl)-amine |
| **028** | 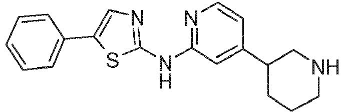 | (1,2,3,4,5,6-Hexahydro-[3,4']bipyridinyl -2'-yl)-(5-phenyl-thiazol-2-yl)-amine |
| **029** | 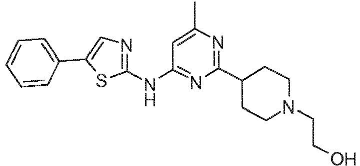 | 2-{4-[4-Methyl-6-(5-phenyl-thiazol-2-ylamino)-pyrimidin-2-yl]-piperidin-1-yl}-ethanol |
| **030** | 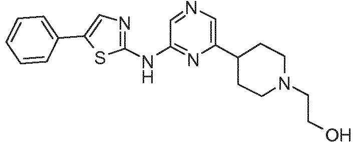 | 2-{4-[6-(5-Phenyl-thiazol-2-ylamino)-pyrazin-2-yl]-piperidin-1-yl}-ethanol |
| **031** | 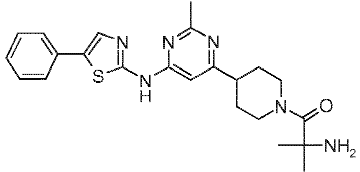 | 2-Amino-2-methyl-1-{4-[2-methyl-6-(5-phenyl-thiazol-2-ylamino)-pyrimidin-4-yl]-piperidin-1-yl}-propan-1-one |
| **032** | 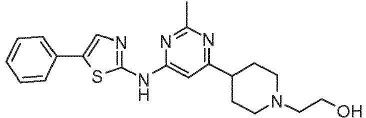 | 2-{4-[2-Methyl-6-(5-phenyl-thiazol-2-ylamino)-pyrimidin-4-yl]-piperidin-1-yl}-ethanol |
| **033** | 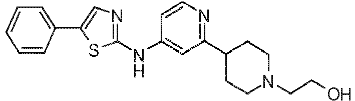 | 2-[4-(5-Phenyl-thiazol-2-ylamino)-3',4',5',6'-tetrahydro-2'H-[2,4']bipyridinyl -1'-yl]-ethanol |
| **034** | 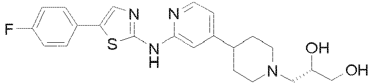 | 3-{2'-[5-(4-Fluoro-phenyl)-thiazol-2-ylamino]-3,4,5,6-tetrahydro-2H-[4,4']bipyridinyl -1-yl}-propane-1,2-diol |
| **035** | 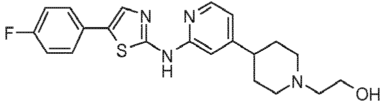 | 2-{2'-[5-4-Fluoro-phenyl)-thiazol-2-ylamino]-3,4,5,6-tetrahydro-2H-[4,4']bipyridinyl -1-yl}-ethanol |
| **036** | 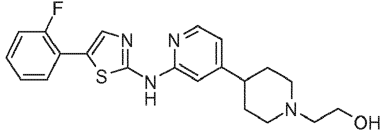 | 2-{2'-[5-(2-Fluoro-phenyl)-thiazol-2-ylamino]-3,4,5,6-tetrahydro-2H-[4,4']bipyridinyl -1-yl}-ethanol |
| **037** | 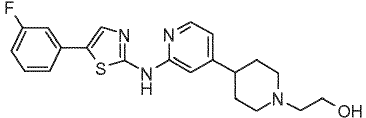 | 2-{2'-[5-(3-Fluoro-phenyl)-thiazol-2-ylamino]-3,4,5,6-tetrahydro-2H-[4,4']bipyridinyl -1-yl}-ethanol |
| **038** | 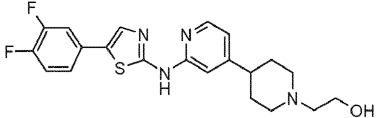 | 2-{2'-[5-(3,4-Difluoro-phenyl)-thiazol-2-ylamino]-3,4,5,6-tetrahydro-2H-[4,4']bipyridinyl -1-yl}-ethanol |
| **039** | 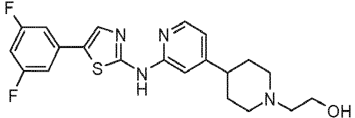 | 2-{2'-[5-(3,5-Difluoro-phenyl)-thiazol-2-ylamino]-3,4,5,6-tetrahydro-2H-[4,4']bipyridinyl -1-yl}-ethanol |
| **040** | 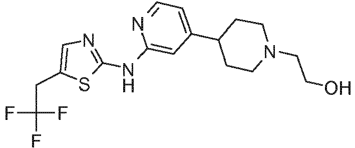 | 2-{2'-[5-(2,2,2-Trifluoro-ethyl)-thiazol-2-ylamino]-3,4,5,6-tetrahydro-2H-[4,4']bipyridinyl -1-yl}-ethanol |
| **041** | 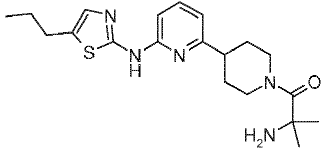 | 2-Amino-2-methyl-1-[6-(5-propyl-thiazol-2-ylamino)-3',4',5',6'-tetrahydro-2'H-[2,4']bipyridinyl -1'-yl]-propan-1-one |
| **042** | 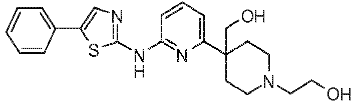 | 2-[4'-Hydroxymethyl-6-(5-phenyl-thiazol-2-ylamino)-3',4',5',6'-tetrahydro-2'H-[2,4']bipyridinyl -1'-yl]-ethanol |
| **043** | 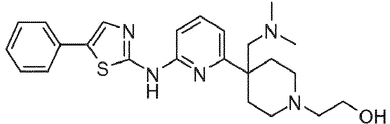 | 2-[4'-Dimethylaminomethyl-6- (5-phenyl-thiazol-2-ylamino)-3',4',5',6'-tetrahydro-2'H-[2,4']bipyridinyl -1'-yl]-ethanol |
| **044** | 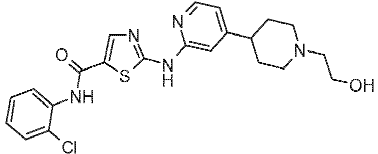 | 2-[1'-{2-Hydroxy-ethyl)-1',2',3',4',5',6' -hexahydro-[4,4']bipyridinyl -2-ylamino]-thiazole-5-carboxylic acid (2-chlorophenyl)-amide |
| **045** | 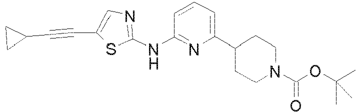 | 6-(5-Cyclopro-pylethynyl-thiazol-2-ylamino)-3',4',5',6'-tetrahydro-2'H-[2,4']bipyridinyl-1'-carboxylic acid tert-butyl ester |
| **046** | 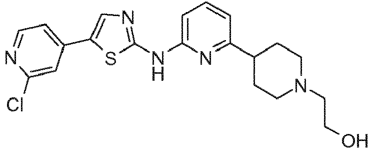 | 2-{6-[5-(2-Chloro-pyridin-4-yl)-thiazol-2-ylamino]-3',4',5',6'-tetrahydro-2'H-[2,4']bipyridinyl -1'-yl}-ethanol |
| **047** | 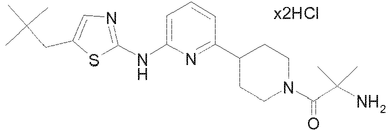 | 2-Amino-1-{6-[5-(2,2-dimethylpropyl)-thiazol-2-ylamino]-3',4',5',6'-tetrahydro-2'H-[2,4']bipyridinyl -1'-yl}-2-methyl-propan-1-one |
| **048** | 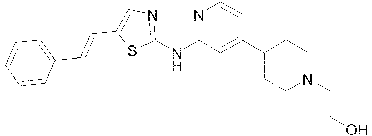 | 2-{2'-[5-((E)-Styryl)-thiazol-2-ylamino]-3,4,5,6-tetrahydro-2H-[4,4']bipyridinyl -1-yl}-ethanol |

Table 6 lists certain physicochemical data of the compounds synthesized in accordance with Examples 1 to 48.

**Table 6. Analytical data (Molecular mass [M+1] and HPLC retention time [min]).**

| **EXAMPLE** | **Calculated MW** | **[M+1]⁺** | **Retention time, min** |
|---|---|---|---|
| 001 | 368.48 | 369.27 | 1.12 |
| 002 | 410.51 | 411.29 | 1.12 |
| 003 | 412.53 | 413.33 | 1.01 |
| 004 | 382.51 | 383.24 | 1.45 |
| 005 | 446.57 | 447.22 | 1.88 |
| 006 | 382.51 | 383.21 | 1.47 |
| 007 | 424.54 | 425.16 | 1.82 |
| 008 | 396.53 | 397.07 | 1.50 |
| 009 | 384.48 | 385.20 | 1.15 |
| 010 | 398.51 | 399.21 | 1.56 |
| 011 | 428.53 | 429.79 | 0.95 |
| 012 | 426.52 | 427.35 | 1.09 |
| 013 | 384.48 | 385.14 | 1.58 |
| 014 | 336.46 | 337.26 | 1.45 |
| 015 | 350.49 | 351.27 | 1.45 |
| 016 | 396.53 | 397.21 | 1.76 |
| 017 | 408.54 | 409.30 | 1.78 |
| 018 | 394.52 | 395.29 | 1.75 |
| 019 | 383.49 | 384.20 | 1.07 |
| 020 | 394.52 | 395.24 | 0.94 |
| 021 | 390.55 | 391.20 | 1.53 |
| 022 | 376.53 | 377.18 | 1.47 |
| 023 | 408.53 | 409.18 | 1.15 |
| 024 | 424.54 | 425.27 | 0.96 |
| 025 | 431.51 | 432.22 | 1.44 |
| 026 | 364.52 | 365.24 | 1.37 |
| 027 | 322.43 | 323.28 | 1.35 |
| 028 | 336.46 | 337.32 | 1.35 |
| 029 | 395.53 | 396.18 | 1.53 |
| 030 | 381.50 | 382.12 | 1.64 |
| 031 | 436.58 | 437.18 | 1.68 |
| 032 | 395.53 | 396.11 | 1.71 |
| 033 | 380.52 | 381.29 | 1.08 |
| 034 | 428.53 | 429.41 | 1.25 |
| 035 | 398.51 | 399.28 | 1.39 |
| 036 | 398.51 | 399.3 | 1.39 |
| 037 | 398.51 | 399.25 | 1.40 |
| 038 | 416.50 | 417.25 | 0.94 |
| 039 | 416.50 | 417.29 | 0.96 |
| 040 | 386.44 | 387.12 | 1.44 |
| 041 | 387.55 | 388.15 | 1.55 |
| 042 | 410.54 | 411.11 | 1.64 |
| 043 | 437.61 | 438.23 | 1.04 |
| 044 | 457.99 | 458.22, 460.20 | 1.36 |
| 045 | 424.57 | 425.34 | 1.76 |
| 046 | 415.95 | 414.22, 416.26 | 1.53 |
| 047 | 415.61 | 416.08 | 1.44 |
| 048 | 406.55 | 407.28 | 1.59 |

### E: Biological Activity Assays

### 1. Kinase Inhibition Assay.

To determine the interaction of our compounds with tyrosine kinases: PDGFR-α, cKIT, PDGFR-β, VEGFR-2 we used enzyme-linked immunosorbent assay (ELISA).

*Determination plates preparation:* The plates are processed by neutravidine for 2 h at 30°C. After washing thrice with TBS/0.1% Tween-20 and blocking with 1% BSA/TBS for 1 h at room temperature (RT) 96-well microtiter plates were washed thrice with TBS/0.1% Tween-20 again.

Reaction: After addition test compounds (in DMSO, DMSO final concentration - 5%) to reaction buffer (20 mM HEPES, pH 7.5, 15 mM MgCl₂, 3 mM MnCl₂, 2 mM DTT, 0.2 mM Na₃VO₄, 0.005% Triton X-100; PDGFR-α,: 0.05 µg/ml, 0.5 µg/ml Poly(G,T)-biotin; PDGFR-β: 0.05 µg/ml, 1 µg/ml Poly(G,T)-biotin; c-KIT: 0.03 µg/ml, 0.15 µg/ml Poly(G,T)-biotin; VEGFR-2: 0.07 µg/ml, 0.05 µg/ml Poly(G,T)-biotin) and preincubation of reaction for 10 min at RT and intensive agitation, ATP solution (PDGFR-α, - 1 µM, PDGFR-β - 0.5 µM, c-KIT - 60 µM, VEGFR-2: - 1.5 µM ATP in reaction buffer per well) was added to initiate reaction. Following incubation of mixture for 1 h at 30°C and intensive agitation the reaction was stopped by stop solution (20 mM HEPES, pH 7.5, 50 mM EDTA).

*Kinase activity determination:* The reaction mixture was transferred to determination plate. After mix incubation for 1 h at RT and intensive agitation the plates were washed thrice with TBS/0.1% Tween-20. Following mouse monoclonal 0.05 µg/ml Anti-p-Tyr (PY20) HRP antibodies (Santa Cruz Biotechnology) in PBS/BSA were added for 1 h at RT and intensive agitation. After washing, antibody binding was detected by tetramethylbenzidine (TMB) color development (Sigma). Measurement of A₄₅₀ nm was carried out after addition of H₂SO₄

Specific compounds of the present invention were tested in the assay described above and were found to have IC₅₀ values as detailed in Table 7 against tested substrates.

**Table 7. Data of biochemical inhibition of RTK for selected examples, IC₅₀ [nM]**

| **EXAMPLE No.** | **IC₅₀ [nM]** | | |
|---|---|---|---|
| | **cKIT** | **PDGFR-α** | **VEGFR-2** |
| 001 | <10 | <10 | >10 |
| 002 | <10 | <10 | >10 |
| 003 | >10 | <10 | >10 |
| 005 | >10 | <10 | >10 |
| 006 | >10 | >10 | >100 |
| 007 | >10 | >10 | >100 |
| 008 | >10 | >10 | >1000 |
| 009 | <10 | <10 | >10 |
| 011 | >10 | <10 | >10 |
| 012 | >10 | <10 | >10 |
| 013 | >10 | >100 | >1000 |
| 014 | >10 | <10 | >10 |
| 015 | >100 | <10 | >10 |
| 016 | <10 | <10 | >10 |
| 017 | <10 | <10 | >10 |
| 018 | <10 | <10 | >10 |
| 019 | <10 | <10 | >10 |
| 020 | <10 | <10 | >100 |
| 021 | >10 | >10 | >100 |
| 022 | >10 | >10 | >100 |
| 023 | <10 | <10 | >10 |
| 024 | >10 | <10 | >10 |
| 027 | >10 | <10 | >10 |
| 028 | >100 | <10 | 32.0 |
| 029 | <10 | >10 | >100 |
| 030 | >100 | >10 | >100 |
| 031 | >10 | <10 | >10 |
| 032 | >10 | <10 | >10 |
| 033 | >100 | >10 | >100 |
| 034 | <10 | <10 | >10 |
| 035 | <10 | <10 | <10 |
| 036 | <10 | <10 | >10 |
| 037 | <10 | <10 | >10 |
| 038 | >10 | <10 | >10 |
| 039 | >10 | <10 | >10 |
| 040 | >100 | >100 | >100 |
| 041 | >100 | no data | no data |
| 042 | >100 | >100 | >10000 |
| 043 | >10000 | >1000 | >10000 |
| 044 | >10 | <10 | >100 |
| 045 | >10000 | >10000 | >10 |
| 046 | >10 | <10 | >100 |
| 047 | >1000 | >100 | >100 |
| 048 | >1000 | >10 | >100 |

### 2. Cellular Assays.

**Cell culture.** The human cancer cell lines A172, A375, A2780 and U251 were purchased from the American Type Culture Collection. All cells were routinely maintained in DMEM containing 10% fetal bovine serum (Omega Scientific), at 37°C in 5% C02.

**Proliferation assay** Inhibition of cell A172, A375, A2780 and U251 cell proliferation was assessed by a MTT assay (MTT = **Thiazolyl Blue Tetrazolium Bromide)** . Cells were inoculated into 96-well microtiter plates containing 0.5% fetal calf serum and incubated at 37°C, 5% CO2, 95% air, and 100% relative humidity for 24 h prior to addition of testing compounds. Following compounds addition, the plates were incubated for an additional 72 h before processing to measure the cell population. Each compound was tested at eight different concentrations in triplicate, as was a control sample. Growth inhibition of 50% (IC₅₀) is calculated by analysis of the data in Prizm3.4 software.

Table 8 lists IC₅₀ values for inhibition of growth of several cancer cell lines, including the glioblastoma cell lines A172 and U251, measured in a MTT cytotoxicity assay.

**Table 8. Data of cytotoxicity assay (MTT) for several cancer cell lines, IC₅₀ [µM]**

| **EXAMPLE** | **IC₅₀ [µM]** | | | |
|---|---|---|---|---|
| | **A172** | **A375** | **A2780** | **U251** |
| 001 | >10 | >1 | >1 | >1 |
| 002 | >1 | >1 | >1 | >10 |
| 003 | ≤1 | >1 | >1 | >10 |
| 004 | >1 | >1 | >1 | >10 |
| 005 | >1 | >1 | >1 | >10 |
| 006 | >1 | ≤1 | ≤1 | >1 |
| 007 | >1 | >1 | >1 | >1 |
| 008 | >10 | >1 | >1 | >1 |
| 009 | >10 | >1 | >1 | >10 |
| 010 | >1 | >1 | >1 | >10 |
| 011 | >1 | >1 | >1 | >10 |
| 012 | >1 | >1 | >1 | >10 |
| 013 | >1 | ≤1 | <1 | >1 |
| 015 | >1 | >1 | >1 | >10 |
| 016 | >1 | no data | >1 | no data |
| 017 | >1 | no data | <1 | no data |
| 018 | >1 | no data | >1 | no data |
| 019 | >10 | >1 | >1 | >10 |
| 020 | ≤1 | no data | <1 | no data |
| 021 | >1 | >1 | >1 | >1 |
| 022 | >1 | ≤1 | ≤1 | >1 |
| 023 | >1 | no data | >1 | no data |
| 024 | ≤1 | no data | ≤1 | no data |
| 026 | >1 | >1 | ≤1 | >10 |
| 028 | >1 | >10 | >1 | >10 |
| 029 | ≤1 | no data | ≤1 | >1 |
| 031 | >1 | no data | >1 | >10 |
| 032 | >1 | no data | ≤1 | >10 |
| 033 | >10 | >10 | >10 | >10 |
| 034 | >1 | no data | >10 | no data |
| 035 | >1 | >1 | >1 | >1 |
| 036 | >10 | >1 | >1 | >1 |
| 037 | >10 | >10 | >1 | >1 |
| 038 | ≤1 | >1 | >10 | >1 |
| 039 | >1 | >1 | >10 | >10 |
| 040 | ≤1 | >1 | >10 | >10 |
| 041 | no data | >1 | ≤1 | no data |
| 044 | no data | >1 | no data | no data |
| 047 | no data | >1 | ≤1 | no data |

### 3. In vivo pharmacokinetic (PK) studies

### PK studies (determination brain -to-plasma ratio)

Male SHK mice (22-25 g) were dosed with 5 mg/kg of related compounds as a single injection via oral gavage (formulated in 20% water solution of HPβCD). Blood samples and whole brains were collected from three mice at each of eight collection time points of 0.05, 0.25, 0.5, 1, 2, 4, 8 and 24 h post dose. Plasma was collected from blood samples, and whole brain samples were homogenized in three volumes of water. Both tissues were analyzed for concentrations of related compounds by liquid chromatography-mass spectrometry (LC-MS/MS).

**Table 9. brain-to-plasma concentration ratios in SHK mice after PO dosing 5mg/kg for several selected examples**

| **EXAMPLE COMPOUND** | **brain-to-plasma concentration ratio** |
|---|---|
| 003 | 3.66 |
| 009 | 10.32 |
| 011 | 12.74 |
| 013 | 0.59 |
| 017 | 0.28 |
| 019 | 0.96 |
| 032 | 0.81 |
| 034 | 1.79 |
| 035 | 18.5 |
| 040 | 1.38 |
| 044 | 0.19 |
| 023 | 0.78 |

### F: Selection and development of drug candidates

In order to select the most appropriate compound to enter further experiments and to assess its suitability for use in a therapeutic composition for the treatment of disorders and diseases, such as cancers, additional data are collected. Such data can include the *in vitro* inhibition of the target molecule, such as a kinase, as measured by IC₅₀, or inhibition of proliferation across a panel of tumor cell lines, and tumor growth inhibition or reduction data and survival data from *in vivo* animal models. Furthermore, such experiments may also include the elucidation and/or determination of the mechanism of action of the subject compound, the target or target profile of the subject compound, and other characteristics of the subject compound, such as the binding affinity of the compound to the target(s) or the binding site of the compound on the target(s) and pharmacokinetic properties. Such experiments may also include molecular modelling of the drug-target interaction and the identification of metabolites formed after administration.

The compound that shows the most appropriate results for IC₅₀ for target inhibition, inhibition of cell proliferation, spectrum across various tumor cell lines, inhibition of tumour growth or tumour reduction data and/or animal-survival data, and/or other features, including ADME, pharmacokinetic and pharmacodynamic properties, may be chosen to enter further experiments. Such experiments may include, for example, therapeutic profiling and toxicology in animals, phase I clinical trials in humans and other clinical trails.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

Those skilled in the art will recognize, or be able to ascertain using no more than routine experimentation, many equivalents to the specific embodiments of the invention described herein. Such equivalents are intended to be encompassed by the following claims. Those skilled in the art will also recognize that all combinations of embodiments, combination of aspects or features of the claims described herein are within the scope of the invention.

**Table 10: Substitution pattern at R¹ and R³ for exemplary compounds:**

| **Example 035:** |
|---|
| Substituent R¹: 4-Fluorophenyl |
| R¹ = substituted aryl |
| Substituent -F = -[(R^{Sm})ₙ] with n = 1: R^{S1} = -F |
| Substituent R³: -CH₂-CH₂-OH |
| R³ = substituted alkyl |
| Substituent -OH = -[(R^{Sm})ₙ] with n = 1: R^{S1} = -OH |

| **Example 034:** |
|---|
| Substituent R¹: 4-Fluorophenyl: see *above* |
| Substituent R³: -CH₂-CH(OH)-CH₂(OH) |
| R³ = substituted alkyl |
| Each substituent -OH = -[(R^{Sm})ₙ] with n = 1: R^{S1} = -OH |

| **Example 041:** |
|---|
| Substituent R¹: -n-propyl |
| Substituent R³: -C(=O)-C(CH₃)₂-NH₂ |
| R³ = -C(=O)-R^{a} |
| R^{a} = substituted alkyl |
| Substituent -NH₂ = -[R^{Sm})ₙ] R^{Sm})ₙ] with n = 1: R^{S1} = -NH₂ |

### Equivalents

Those skilled in the art will recognize, or be able to ascertain using no more than routine experimentation, many equivalents to the specific embodiments of the invention described herein. Such equivalents are intended to be encompassed by the following claims.

All of the above-cited references and publications are hereby incorporated by reference.

## Claims

1. A compound having a structure represented by a general formula (I) 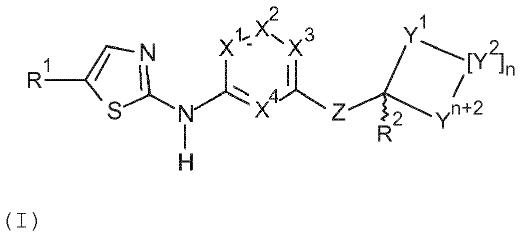 or any tautomeric form thereof, wherein
R¹ is selected from -hydrogen, -halogen, unsubstituted or substituted -alkyl, unsubstituted or substituted -alkynyl, unsubstituted or substituted -aryl, unsubstituted or substituted -heteroaryl, -C(=O)OR⁴, -C(=O)N(R⁵)₂, and -CN; wherein
R⁴ is selected from unsubstituted or substituted alkyl; and each R⁵ is independently selected from unsubstituted or substituted alkyl, or alternatively, the two substituents R⁵, together with the N atom they are attached to, form a heterocycle, which may additionally contain a further heteroatom selected form N, O, or S;
X¹, X², X³, and X⁴ are each independently selected from C-R⁶ and N, provided that either one, two or three of X¹, X², X³, and X⁴ is/are N; wherein
R⁶ is selected from hydrogen, halogen, unsubstituted or substituted -C₁₋₄-alkyl, and CN;
Z is selected from an ordinary bond and unsubstituted or substituted C₁₋₄-alkylene;
R² is selected from hydrogen, halogen, unsubstituted or substituted C₁₋₄-alkyl, -C(=O)OR^{4'}, and -C(=O)N(R⁵)₂;
wherein R^{4'} is selected from hydrogen and unsubstituted or substituted -alkyl; and
Y¹, each of Y² and Yⁿ⁺² are each independently selected from C(R⁷)₂, N(R³) and O, wherein n is selected from 1, 2, 3, 4, and 5, provided that Y¹-[Y²]ₙ-Yⁿ⁺² comprises: (n+2) C(R⁷)₂ residues;
(n+1) C(R⁷)₂ residues and one N(R³) residue;
(n+1) C(R⁷)₂ residues and one O residue;
n C(R⁷)₂ residues, one N(R³) residue and one O residue;
n C(R⁷)₂ residues and two N(R³) residues; or
n C(R⁷)₂ residues and two O residues, provided that the two O residues are separated by at least one C(R⁷)₂ residue; wherein:
each R⁷ is independently selected from hydrogen, F, and unsubstituted or substituted -C₁₋₄-alkyl; and
each R³ is independently selected from hydrogen and substituted or unsubstituted: alkyl, alkenyl, alkynyl, cycloalkyl, cycloalkenyl, (heterocycloalkyl connected via C atom),
(heterocycloalkenyl connected via C atom), aryl, heteroaryl, -C(=O)-R⁸, -C(=O)-aryl, -C(=O)-heteroaryl, - S(=O)₂R⁸, -S(=O)₂N(R⁵)₂, -C(=O)OR^{4'}, and -C(=O)N(R⁵)₂; wherein
R⁸ is selected from substituted or unsubstituted: alkyl, alkenyl, alkynyl, cycloalkyl, cycloalkenyl, heterocycloalkyl, heterocycloalkenyl, aryl, and heteroaryl; and/or two substituents R⁷ and/or R³, together with the ring atom (atoms) they are attached to, form a cycle, which may additionally contain a further heteroatom selected form N, O, or S;
provided that the following compound is excluded:

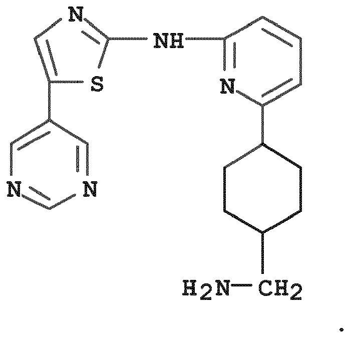

2. The compound of claim 1, wherein R¹ is selected from hydrogen, halogen, unsubstituted or substituted C₁₋₄-alkyl, unsubstituted or substituted -C₂₋₄-alkynyl, unsubstituted or substituted aryl, unsubstituted or substituted -heteroaryl, -C(=O)O-C₁₋₄-alkyl, -C(=O)N(C₁₋₄-alkyl)₂, and -CN.

3. The compounds of claim 1, wherein Z is selected from an ordinary bond and -CH₂-.

4. The compound of claim 1, wherein the compound has a structure represented by formula (Ia) 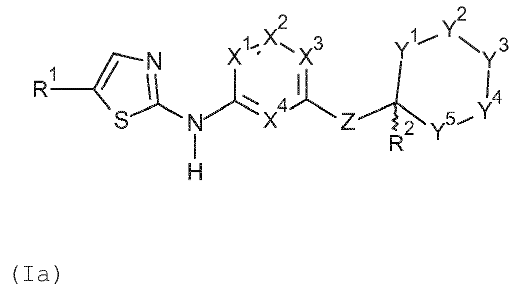 or tautomeric or stereoisomeric form thereof, wherein Y³ and Y⁴ correspond to Y² as defined above, wherein Y⁵ corresponds to Yⁿ⁺² as defined above.

5. The compound of claim 4, wherein the compound has a structure represented by formula (Ib) 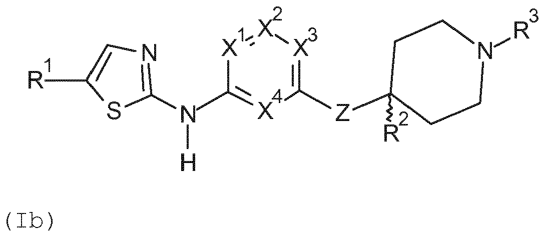 or any tautomeric or stereoisomeric form thereof.

6. The compound of claim 5, wherein the compound has a structure represented by formula (Ic) 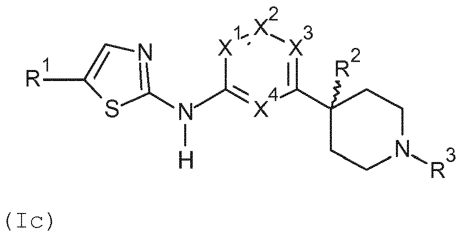 or any tautomeric or stereoisomeric form thereof.

7. The compound of claim 1, wherein the compound is selected from:
[5-((4-Fluoro-phenyl)-thiazol-2-yl]-(4-piperidin-4-ylmethyl-pyridin-2-yl)-amine;
1-((4-{2-[5-(4-Fluoro-phenyl)-thiazol-2-ylamino]-pyridin-4-ylmethyl}-piperidin-1-yl)-ethanone;
2-((4-{2-[5-(4-Fluoro-phenyl)-thiazol-2-ylamino]-pyridin-4-ylmethyl}-piperidin-1-yl)-ethanol;
[5-((4-Fluoro-phenyl)-thiazol-2-yl]-[4-(1-methyl-piperidin-4-ylmethyl)-pyridin-2-yl]-amine;
[5-((4-Fluoro-phenyl)-thiazol-2-yl]-[4-(1-methanesulfonyl-piperidin-4-ylmethyl)-pyridin-2-yl]-amine;
(6-Azepan-4-ylmethyl-pyridin-2-yl)-[5-(4-fluoro-phenyl)-thiazol-2-yl]-amine;
1-((4-{6-[5-(4-Fluoro-phenyl)-thiazol-2-ylamino]-pyridin-2-ylmethyl}-azepan-1-yl)-ethanone;
[5-((4-Fluoro-phenyl)-thiazol-2-yl]-[6-(1-methyl-azepan-4-ylmethyl)-pyridin-2-yl]-amine;
[5-((4-Fluoro-phenyl)-thiazol-2-yl]-(4-[1,4]oxazepan-6-ylmethyl-pyridin-2-yl)-amine;
[5-((4-Fluoro-phenyl)-thiazol-2-yl]-[4-(4-methyl-[1,4]oxazepan-6-ylmethyl)-pyridin-2-yl]-amine;
2-((6-{2-[5-(4-Fluoro-phenyl)-thiazol-2-ylamino]-pyridin-4-ylmethyl}-[1,4]oxazepan-4-yl)-ethanol;
1-((6-{2-[5-(4-Fluoro-phenyl)-thiazol-2-ylamino]-pyridin-4-ylmethyl}-[1,4]oxazepan-4-yl)-ethanone;
[5-((4-Fluoro-phenyl)-thiazol-2-yl]-(6-[1,4]oxazepan-6-ylmethyl-pyridin-2-yl)-amine;
(5-Phenyl-thiazol-2-yl)-(4-pyrrolidin-3-ylmethyl-pyridin-2-yl)-amine;
(5-Phenyl-thiazol-2-yl)-[4-(2-pyrrolidin-2-yl-ethyl)-pyridin-2-yl]-amine;
[5-((4-Fluoro-phenyl)-thiazol-2-yl]-[4-(4-methylamino-cyclohexylmethyl)-pyridin-2-yl]-amine;
[5-((4-Fluoro-phenyl)-thiazol-2-yl]-[4-(octahydro-isoindol-4-ylmethyl)-pyridin-2-yl]-amine;
[5-((4-Fluoro-phenyl)-thiazol-2-yl]-[4-(octahydro-cyclopenta[c]pyrrol-4-ylmethyl)-pyridin-2-yl]-amine;
(4-[1,4]Diazepan-6-ylmethyl-pyridin-2-yl)-[5-(4-fluoro-phenyl)-thiazol-2-yl]-amine;
[5-((4-Fluoro-phenyl)-thiazol-2-yl]-[6-(octahydro-cyclopenta[c]pyrrol-4-ylmethyl)-pyridin-2-yl]-amine;
[5-((4-Fluoro-phenyl)-thiazol-2-yl]-[6-(octahydro-isoindol-4-ylmethyl)-pyridin-2-yl]-amine;
[6-((8-Aza-bicyclo[3.2.1]oct-3-ylmethyl)-pyridin-2-yl]-(5-phenyl-thiazol-2-yl)-amine;
2-Amino-4-[2-(5-phenyl-thiazol-2-ylamino)-pyridin-4-ylmethyl]-cyclopentanecarboxylic acid methyl ester;
[5-((4-Fluoro-phenyl)-thiazol-2-yl]-[6-(1-oxa-8-aza-spiro[4.5]dec-3-ylmethyl)-pyridin-2-yl]-amine;
2-((4-{5-Fluoro-2-[5-(4-fluoro-phenyl)-thiazol-2-ylamino]-pyrimidin-4-ylmethyl}-piperidin-1-yl)-ethanol;
(4-Azepan-4-ylmethyl-pyridin-2-yl)-(5-phenyl-thiazol-2-yl)-amine;
(5-Phenyl-thiazol-2-yl)-(4-pyrrolidin-2-yl-pyridin-2-yl)-amine;
(1,2,3,4,5,6-Hexahydro-[3,4']bipyridinyl-2'-yl)-(5-phenyl-thiazol-2-yl)-amine;
2-{4-[4-Methyl-6-(5-phenyl-thiazol-2-ylamino)-pyrimidin-2-yl]-piperidin-1-yl}-ethanol;
2-{4-[6-(5-Phenyl-thiazol-2-ylamino)-pyrazin-2-yl]-piperidin-1-yl}-ethanol;
2-Amino-2-methyl-1-{4-[2-methyl-6-(5-phenyl-thiazol-2-ylamino)-pyrimidin-4-yl]-piperidin-1-yl}-propan-1-one;
2-{4-[2-Methyl-6-(5-phenyl-thiazol-2-ylamino)-pyrimidin-4-yl]-piperidin-1-yl}-ethanol;
2-[4-(5-Phenyl-thiazol-2-ylamino)-3',4',5',6'-tetrahydro-2'H-[2,4']bipyridinyl-1'-yl]-ethanol;
3-{2'-[5-(4-Fluoro-phenyl)-thiazol-2-ylamino]-3,4,5,6-tetrahydro-2H-[4,4']bipyridinyl-1-yl}-propane-1,2-diol;
2-{2'-[5-(4-Fluoro-phenyl)-thiazol-2-ylamino]-3,4,5,6-tetrahydro-2H-[4,4']bipyridinyl-1-yl}-ethanol;
2-{2'-[5-(2-Fluoro-phenyl)-thiazol-2-ylamino]-3,4,5,6-tetrahydro-2H-[4,4']bipyridinyl-1-yl}-ethanol;
2-{2'-[5-(3-Fluoro-phenyl)-thiazol-2-ylamino]-3,4,5,6-tetrahydro-2H-[4,4']bipyridinyl-1-yl}-ethanol;
2-{2'-[5-(3,4-Difluoro-phenyl)-thiazol-2-ylamino]-3,4,5,6-tetrahydro-2H-[4,4']bipyridinyl-1-yl}-ethanol;
2-{2'-[5-(3,5-Difluoro-phenyl)-thiazol-2-ylamino]-3,4,5,6-tetrahydro-2H-[4,4']bipyridinyl-1-yl}-ethanol;
2-{2'-[5-(2,2,2-Trifluoro-ethyl)-thiazol-2-ylamino]-3,4,5,6-tetrahydro-2H-[4,4']bipyridinyl-1-yl}-ethanol;
2-Amino-2-methyl-1-[6-(5-propyl-thiazol-2-ylamino)-3',4',5',6'-tetrahydro-2'H-[2,4']bipyridinyl-1'-yl]-propan-1-one;
2-[4'-Hydroxymethyl-6-(5-phenyl-thiazol-2-ylamino)-3',4',5',6'-tetrahydro-2'H-[2,4']bipyridinyl-1'-yl]-ethanol;
2-[4'-Dimethylaminomethyl-6-(5-phenyl-thiazol-2-ylamino)-3',4',5',6'-tetrahydro-2'H-[2,4']bipyridinyl-1'-yl]-ethanol;
2-[1'-(2-Hydroxy-ethyl)-1',2',3',4',5',6'-hexahydro-[4,4']bipyridinyl-2-ylamino]-thiazole-5-carboxylic acid (2-chloro-phenyl)-amide;
6-((5-Cyclopropylethynyl-thiazol-2-ylamino)-3',4',5',6'-tetrahydro-2'H-[2,4']bipyridinyl-1'-carboxylic acid tert-butyl ester;
2-{6-[5-(2-Chloro-pyridin-4-yl)-thiazol-2-ylamino]-3',4',5',6'-tetrahydro-2'H-[2,4']bipyridinyl-1'-yl}-ethanol;
2-Amino-1-{6-[5-(2,2-dimethyl-propyl)-thiazol-2-ylamino]-3',4',5',6'-tetrahydro-2'H-[2,4']bipyridinyl-1'-yl}-2-methyl-propan-1-one; and
2-{2'-[5-((E)-Styryl)-thiazol-2-ylamino]-3,4,5,6-tetrahydro-2H-[4,4']bipyridinyl-1-yl}-ethanol.

8. Salts of compounds of any one of claims 1 to 7 with a corresponding acid or base.

9. The salt of claim 8, wherein the salt is a pharmaceutically acceptable salt.

10. A pharmaceutical composition, comprising the compound of any one of claims 1 to 7, or the salt of claim 9, and a pharmaceutically acceptable diluent, excipient or carrier.

11. The pharmaceutical composition of claim 10, comprising a therapeutically effective amount of said compound or salt.

12. The pharmaceutical composition of claim 10, for use in the treatment of an individual in need thereof.

13. The pharmaceutical composition of claim 11, for use in the treatment of an individual in need thereof.

14. The pharmaceutical composition of claim 12, wherein said individual is a human.

15. The pharmaceutical composition of claim 13, wherein said individual is a human.

16. A method for treating a disease or disorder in an individual, comprising administering a therapeutically effective amount of the compound of any one of claims 1 to 7, the salt of claim 9, or the pharmaceutical composition of any one of claims 11 to 15.

17. A method for treating a disease or disorder in an individual comprising administering a therapeutically effective amount of a pharmaceutical composition of claim 10.

18. The method of claim 16, wherein said individual is a mammal selected from: domestic mammal, cat, dog, horse, sheep, cow, rodents, and human, particularly wherein said mammal is a human.

19. The method of claim 17, wherein said individual is a mammal selected from: domestic mammal, cat, dog, horse, sheep, cow, rodents, and human, particularly wherein said mammal is a human.
